# EUROPEAN PATENT SPECIFICATION

(11) **EP 2 275 574 B1**
(45) Date of publication and mention of the grant of the patent: **03.06.2015**
(21) Application number: 10175317.6
(22) Date of filing: 14.08.1998
(51) Int. Cl.: C12Q 1/68, C07K 14/47

(54) **Coding sequence haplotypes of the human BRCA2 gene**
Menschliche BRCA2-Gen-kodierende Sequenzhaplotypen
Codage d'haplotypes de séquence du gène BRCA2 humain

(30) Priority: 15.08.1997 US 55784 P; 07.11.1997 US 64926 P; 12.11.1997 US 65367 P; 01.05.1998 US 71715; 22.05.1998 US 84471
(43) Date of publication of application: 19.01.2011
(62) Divisional of application: 98945756.9
(73) Proprietor: Gene Logic Inc., Gaithersburg, MD 20879 (US)
(72) Inventor: Murphy, Patricia D., Slingerlands, NY 12159-639 (US); White, Marga B., Frederick, MD 21704 (US); Rabin, Mark B., Rockville, MD 20850 (US); Olson, Sheri J., Falls Chuch, VA 22042 (US); Yoshikawa, Matthew, Palo Alto, CA 94304 (US); Jackson, Geoffrey M., Beltsville, MD 20705 (US); Eskandari, Tara, Rockville, MD 20852 (US); Schryer, Brenda, Oxnard, CA 93035 (US); Park, Michael, Rockville, MD 20850 (US)
(74) Representative: Vossius & Partner Patentanwälte Rechtsanwälte mbB

(56) References cited:
- WO-A1-97/19110
- WO-A1-97/22689
- WOOSTER R ET AL: "IDENTIFICATION OF THE BREAST CANCER SUSCEPTIBILITY GENE BRCA2", NATURE: INTERNATIONAL WEEKLY JOURNAL OF SCIENCE, NATURE PUBLISHING GROUP, UNITED KINGDOM, vol. 378, 1 December 1995 (1995-12-01), pages 789-792, XP002029512, ISSN: 0028-0836, DOI: 10.1038/378789A0

### SEQUENCE LISTING

<110> Gene Logic Inc.
<120> CODING SEQUENCE HAPLOTYPES OF THE HUMAN BRCA2 GENE
<130> R2817 EP/1 s3
<150> 60/055,784
   <151> 1997-08-15
<150> 60/064,926
   <151> 1997-11-07
<150> 60/065,367
   <151> 1997-11-12
<150> 09/071,715
   <151> 1998-05-01
<150> 09/084,471
   <151> 1998-05-22
<150> PCT/US98/16905
   <151> 1998-08-14
<150> EP 98 945 756.9
   <151> 1998-08-14
<160> 110
<170> PatentIn version 3.5
<210> 1
   <211> 50
   <212> DNA
   <213> Homo sapiens
<220>
   <221> source
   <223> Exon 5
<400> 1
   tcctgttgtt ctacaatgta cacatgtaac accacaaaga gataagtcag 50
<210> 2
   <211> 182
   <212> DNA
   <213> Homo sapiens
<220>
   <221> source
   <223> Exon 15
<400> 2
   atttaattac aagtcttcag aatgccagag atatacagga tatgcgaatt aagaagaaac 60
   aaaggcaacg cgtctttcca cagccaggca gtctgtatct tgcaaaaaca tccactctgc 120
   ctcgaatctc tctgaaagca gcagtaggag gccaagttcc ctctgcgtgt tctcataaac 180
   ag 182
<210> 3
   <211> 188
   <212> DNA
   <213> Homo sapiens
<220>
   <221> source
   <223> Exon 16
<400> 3
   ctgtatacgt atggcgtttc taaacattgc ataaaaatta acagcaaaaa tgcagagtct 60
   tttcagtttc acactgaaga ttattttggt aaggaaagtt tatggactgg aaaaggaata 120
   cagttggctg atggtggatg gctcataccc tccaatgatg gaaaggctgg aaaagaagaa 180
   ttttatag 188
<210> 4
   <211> 10485
   <212> DNA
   <213> Homo sapiens
<220>
   <221> CDS
   <222> (229)..(10482)
   <223> BRCA2 (OMI1)
<400> 4
   ggtggcgcga gcttctgaaa ctaggcggca gaggcggagc cgctgtggca ctgctgcgcc        60
   tctgctgcgc ctcgggtgtc ttttgcggcg gtgggtcgcc gccgggagaa gcgtgagggg       120
   acagatttgt gaccggcgcg gtttttgtca gcttactccg gccaaaaaag aactgcacct       180
   ctggagcgga cttatttacc aagcattgga ggaatatcgt aggtaaaa atg cct att        237
   gga tcc aaa gag agg cca aca ttt ttt gaa att ttt aag aca cgc tgc         285
   aac aaa gca gat tta gga cca ata agt ctt aat tgg ttt gaa gaa ctt         333
   tct tca gaa gct cca ccc tat aat tct gaa cct gca gaa gaa tct gaa         381
   cat aaa aac aac aat tac gaa cca aac cta ttt aaa act cca caa agg         429
   aaa cca tct tat aat cag ctg gct tca act cca ata ata ttc aaa gag         477
   caa ggg ctg act ctg ccg ctg tac caa tct cct gta aaa gaa tta gat         525
   aaa ttc aaa tta gac tta gga agg aat gtt ccc aat agt aga cat aaa         573
   agt ctt cgc aca gtg aaa act aaa atg gat caa gca gat gat gtt tcc      621
   tgt cca ctt cta aat tct tgt ctt agt gaa agt cct gtt gtt cta caa      669
   tgt aca cat gta aca cca caa aga gat aag tca gtg gta tgt ggg agt      717
   ttg ttt cat aca cca aag ttt gtg aag ggt cgt cag aca cca aaa cat      765
   att tct gaa agt cta gga gct gag gtg gat cct gat atg tct tgg tca      813
   agt tct tta gct aca cca ccc acc ctt agt tct act gtg ctc ata gtc      861
   aga aat gaa gaa gca tct gaa act gta ttt cct cat gat act act gct      909
   aat gtg aaa agc tat ttt tcc aat cat gat gaa agt ctg aag aaa aat      957
   gat aga ttt atc gct tct gtg aca gac agt gaa aac aca aat caa aga     1005
   gaa gct gca agt cat gga ttt gga aaa aca tca ggg aat tca ttt aaa     1053
   gta aat agc tgc aaa gac cac att gga aag tca atg cca aat gtc cta     1101
   gaa gat gaa gta tat gaa aca gtt gta gat acc tct gaa gaa gat agt     1149
   ttt tca tta tgt ttt tct aaa tgt aga aca aaa aat cta caa aaa gta     1197
   aga act agc aag act agg aaa aaa att ttc cat gaa gca aac gct gat     1245
   gaa tgt gaa aaa tct aaa aac caa gtg aaa gaa aaa tac tca ttt gta     1293
   tct gaa gtg gaa cca aat gat act gat cca tta gat tca aat gta gca     1341
   cat cag aag ccc ttt gag agt gga agt gac aaa atc tcc aag gaa gtt     1389
   gta ccg tct ttg gcc tgt gaa tgg tct caa cta acc ctt tca ggt cta     1437
   aat gga gcc cag atg gag aaa ata ccc cta ttg cat att tct tca tgt      1485
   gac caa aat att tca gaa aaa gac cta tta gac aca gag aac aaa aga      1533
   aag aaa gat ttt ctt act tca gag aat tct ttg cca cgt att tct agc      1581
   cta cca aaa tca gag aag cca tta aat gag gaa aca gtg gta aat aag      1629
   aga gat gaa gag cag cat ctt gaa tct cat aca gac tgc att ctt gca      1677
   gta aag cag gca ata tct gga act tct cca gtg gct tct tca ttt cag      1725
   ggt atc aaa aag tct ata ttc aga ata aga gaa tca cct aaa gag act      1773
   ttc aat gca agt ttt tca ggt cat atg act gat cca aac ttt aaa aaa      1821
   gaa act gaa gcc tct gaa agt gga ctg gaa ata cat act gtt tgc tca      1869
   cag aag gag gac tcc tta tgt cca aat tta att gat aat gga agc tgg      1917
   cca gcc acc acc aca cag aat tct gta gct ttg aag aat gca ggt tta      1965
   ata tcc act ttg aaa aag aaa aca aat aag ttt att tat gct ata cat      2013
   gat gaa aca tct tat aaa gga aaa aaa ata ccg aaa gac caa aaa tca      2061
   gaa cta att aac tgt tca gcc cag ttt gaa gca aat gct ttt gaa gca      2109
   cca ctt aca ttt gca aat gct gat tca ggt tta ttg cat tct tct gtg      2157
   aaa aga agc tgt tca cag aat gat tct gaa gaa cca act ttg tcc tta      2205
   act agc tct ttt ggg aca att ctg agg aaa tgt tct aga aat gaa aca      2253
   tgt tct aat aat aca gta atc tct cag gat ctt gat tat aaa gaa gca        2301
   aaa tgt aat aag gaa aaa cta cag tta ttt att acc cca gaa gct gat        2349
   tct ctg tca tgc ctg cag gaa gga cag tgt gaa aat gat cca aaa agc        2397
   aaa aaa gtt tca gat ata aaa gaa gag gtc ttg gct gca gca tgt cac        2445
   cca gta caa cat tca aaa gtg gaa tac agt gat act gac ttt caa tcc        2493
   cag aaa agt ctt tta tat gat cat gaa aat gcc agc act ctt att tta        2541
   act cct act tcc aag gat gtt ctg tca aac cta gtc atg att tct aga        2589
   ggc aaa gaa tca tac aaa atg tca gac aag ctc aaa ggt aac aat tat        2637
   gaa tct gat gtt gaa tta acc aaa aat att ccc atg gaa aag aat caa        2685
   gat gta tgt gct tta aat gaa aat tat aaa aac gtt gag ctg ttg cca        2733
   cct gaa aaa tac atg aga gta gca tca cct tca aga aag gta caa ttc        2781
   aac caa aac aca aat cta aga gta atc caa aaa aat caa gaa gaa act        2829
   act tca att tca aaa ata act gtc aat cca gac tct gaa gaa ctt ttc        2877
   tca gac aat gag aat aat ttt gtc ttc caa gta gct aat gaa agg aat        2925
   aat ctt gct tta gga aat act aag gaa ctt cat gaa aca gac ttg act        2973
   tgt gta aac gaa ccc att ttc aag aac tct acc atg gtt tta tat gga        3021
   gac aca ggt gat aaa caa gca acc caa gtg tca att aaa aaa gat ttg        3069
   gtt tat gtt ctt gca gag gag aac aaa aat agt gta aag cag cat ata        3117
   aaa atg act cta ggt caa gat tta aaa tcg gac atc tcc ttg aat ata        3165
   gat aaa ata cca gaa aaa aat aat gat tac atg aac aaa tgg gca gga        3213
   ctc tta ggt cca att  tca aat cac agt ttt  gga ggt agc ttc aga          3258
   aca gct tca aat aag  gaa atc aag ctc tct  gaa cat aac att aag          3303
   aag agc aaa atg ttc  ttc aaa gat att gaa  gaa caa tat cct act          3348
   agt tta gct tgt gtt  gaa att gta aat acc  ttg gca tta gat aat          3393
   caa aag aaa ctg agc  aag cct cag tca att  aat act gta tct gca          3438
   cat tta cag agt agt  gta gtt gtt tct gat  tgt aaa aat agt cat          3483
   ata acc cct cag atg  tta ttt tcc aag cag  gat ttt aat tca aac          3528
   cat aat tta aca cct  agc caa aag gca gaa  att aca gaa ctt tct          3573
   act ata tta gaa gaa  tca gga agt cag ttt  gaa ttt act cag ttt          3618
   aga aaa cca agc tac  ata ttg cag aag agt  aca ttt gaa gtg cct          3663
   gaa aac cag atg act  atc tta aag acc act  tct gag gaa tgc aga          3708
   gat gct gat ctt cat  gtc ata atg aat gcc  cca tcg att ggt cag          3753
   gta gac agc agc aag  caa ttt gaa ggt aca  gtt gaa att aaa cgg          3798
   aag ttt gct ggc ctg  ttg aaa aat gac tgt  aac aaa agt gct tct          3843
   ggt tat tta aca gat  gaa aat gaa gtg ggg  ttt agg ggc ttt tat       3888
   tct gct cat ggc aca  aaa ctg aat gtt tct  act gaa gct ctg caa       3933
   aaa gct gtg aaa ctg  ttt agt gat att gag  aat att agt gag gaa       3978
   act tct gca gag gta  cat cca ata agt tta  tct tca agt aaa tgt       4023
   cat gat tct gtt gtt  tca atg ttt aag ata  gaa aat cat aat gat       4068
   aaa act gta agt gaa  aaa aat aat aaa tgc  caa ctg ata tta caa       4113
   aat aat att gaa atg  act act ggc act ttt  gtt gaa gaa att act       4158
   gaa aat tac aag aga  aat act gaa aat gaa  gat aac aaa tat act       4203
   gct gcc agt aga aat  tct cat aac tta gaa  ttt gat ggc agt gat       4248
   tca agt aaa aat gat  act gtt tgt att cat  aaa gat gaa acg gac       4293
   ttg cta ttt act gat  cag cac aac ata tgt  ctt aaa tta tct ggc       4338
   cag ttt atg aag gag  gga aac act cag att  aaa gaa gat ttg tca       4383
   gat tta act ttt ttg  gaa gtt gcg aaa gct  caa gaa gca tgt cat       4428
   ggt aat act tca aat  aaa gaa cag tta act  gct act aaa acg gag       4473
   caa aat ata aaa gat  ttt gag act tct gat  aca ttt ttt cag act       4518
   gca agt ggg aaa aat  att agt gtc gcc aaa  gag tca ttt aat aaa       4563
   att gta aat ttc ttt  gat cag aaa cca gaa  gaa ttg cat aac ttt       4608
   tcc tta aat tct gaa  tta cat tct gac ata  aga aag aac aaa atg       4653
   gac att cta agt tat gag gaa aca gac ata gtt aaa cac aaa ata      4698
   ctg aaa gaa agt gtc cca gtt ggt act gga aat caa cta gtg acc      4743
   ttc cag gga caa ccc gaa cgt gat gaa aag atc aaa gaa cct act      4788
   ctg ttg ggt ttt cat aca gct agc ggg aaa aaa gtt aaa att gca      4833
   aag gaa tct ttg gac aaa gtg aaa aac ctt ttt gat gaa aaa gag      4878
   caa ggt act agt gaa atc acc agt ttt agc cat caa tgg gca aag      4923
   acc cta aag tac aga gag gcc tgt aaa gac ctt gaa tta gca tgt      4968
   gag acc att gag atc aca gct gcc cca aag tgt aaa gaa atg cag      5013
   aat tct ctc aat aat gat aaa aac ctt gtt tct att gag act gtg      5058
   gtg cca cct aag ctc tta agt gat aat tta tgt aga caa act gaa      5103
   aat ctc aaa aca tca aaa agt atc ttt ttg aaa gtt aaa gta cat      5148
   gaa aat gta gaa aaa gaa aca gca aaa agt cct gca act tgt tac      5193
   aca aat cag tcc cct tat tca gtc att gaa aat tca gcc tta gct      5238
   ttt tac aca agt tgt agt aga aaa act tct gtg agt cag act tca      5283
   tta ctt gaa gca aaa aaa tgg ctt aga gaa gga ata ttt gat ggt      5328
   caa cca gaa aga ata aat act gca gat tat gta gga aat tat ttg      5373
   tat gaa aat aat tca aac agt act ata gct gaa aat gac aaa aat      5418
   cat ctc tcc gaa aaa  caa gat act tat tta  agt aac agt agc atg      5463
   tct aac agc tat tcc  tac cat tct gat gag  gta tat aat gat tca      5508
   gga tat ctc tca aaa  aat aaa ctt gat tct  ggt att gag cca gta      5553
   ttg aag aat gtt gaa  gat caa aaa aac act  agt ttt tcc aaa gta      5598
   ata tcc aat gta aaa  gat gca aat gca tac  cca caa act gta aat      5643
   gaa gat att tgc gtt  gag gaa ctt gtg act  agc tct tca ccc tgc      5688
   aaa aat aaa aat gca  gcc att aaa ttg tcc  ata tct aat agt aat      5733
   aat ttt gag gta ggg  cca cct gca ttt agg  ata gcc agt ggt aaa      5778
   atc gtt tgt gtt tca  cat gaa aca att aaa  aaa gtg aaa gac ata      5823
   ttt aca gac agt ttc  agt aaa gta att aag  gaa aac aac gag aat      5868
   aaa tca aaa att tgc  caa acg aaa att atg  gca ggt tgt tac gag      5913
   gca ttg gat gat tca  gag gat att ctt cat  aac tct cta gat aat      5958
   gat gaa tgt agc acg  cat tca cat aag gtt  ttt gct gac att cag      6003
   agt gaa gaa att tta  caa cat aac caa aat  atg tct gga ttg gag      6048
   aaa gtt tct aaa ata  tca cct tgt gat gtt  agt ttg gaa act tca      6093
   gat ata tgt aaa tgt  agt ata ggg aag ctt  cat aag tca gtc tca      6138
   tct gca aat act tgt  ggg att ttt agc aca  gca agt gga aaa tct      6183
   gtc cag gta tca gat  gct tca tta caa aac  gca aga caa gtg ttt      6228
   tct gaa ata gaa gat  agt acc aag caa gtc  ttt tcc aaa gta ttg      6273
   ttt aaa agt aac gaa  cat tca gac cag ctc  aca aga gaa gaa aat      6318
   act gct ata cgt act  cca gaa cat tta ata  tcc caa aaa ggc ttt      6363
   tca tat aat gtg gta  aat tca tct gct ttc  tct gga ttt agt aca      6408
   gca agt gga aag caa  gtt tcc att tta gaa  agt tcc tta cac aaa      6453
   gtt aag gga gtg tta  gag gaa ttt gat tta  atc aga act gag cat      6498
   agt ctt cac tat tca  cct acg tct aga caa  aat gta tca aaa ata      6543
   ctt cct cgt gtt gat  aag aga aac cca gag  cac tgt gta aac tca      6588
   gaa atg gaa aaa acc  tgc agt aaa gaa ttt  aaa tta tca aat aac      6633
   tta aat gtt gaa ggt  ggt tct tca gaa aat  aat cac tct att aaa      6678
   gtt tct cca tat ctc  tct caa ttt caa caa  gac aaa caa cag ttg      6723
   gta tta gga acc aaa  gtc tca ctt gtt gag  aac att cat gtt ttg      6768
   gga aaa gaa cag gct  tca cct aaa aac gta  aaa atg gaa att ggt      6813
   aaa act gaa act ttt  tct gat gtt cct gtg  aaa aca aat ata gaa      6858
   gtt tgt tct act tac  tcc aaa gat tca gaa  aac tac ttt gaa aca      6903
   gaa gca gta gaa att  gct aaa gct ttt atg  gaa gat gat gaa ctg      6948
   aca gat tct aaa ctg  cca agt cat gcc aca  cat tct ctt ttt aca      6993
   tgt ccc gaa aat gag  gaa atg gtt ttg tca  aat tca aga att gga      7038
   aaa aga aga gga gag  ccc ctt atc tta gtg  gga gaa ccc tca atc      7083
   aaa aga aac tta tta  aat gaa ttt gac agg  ata ata gaa aat caa      7128
   gaa aaa tcc tta aag  gct tca aaa agc act  cca gat ggc aca ata      7173
   aaa gat cga aga ttg  ttt atg cat cat gtt  tct tta gag ccg att      7218
   acc tgt gta ccc ttt  cgc aca act aag gaa  cgt caa gag ata cag      7263
   aat cca aat ttt acc  gca cct ggt caa gaa  ttt ctg tct aaa tct      7308
   cat ttg tat gaa cat  ctg act ttg gaa aaa  tct tca agc aat tta      7353
   gca gtt tca gga cat  cca ttt tat caa gtt  tct gct aca aga aat      7398
   gaa aaa atg aga cac  ttg att act aca ggc  aga cca acc aaa gtc      7443
   ttt gtt cca cct ttt  aaa act aaa tca cat  ttt cac aga gtt gaa      7488
   cag tgt gtt agg aat  att aac ttg gag gaa  aac aga caa aag caa      7533
   aac att gat gga cat  ggc tct gat gat agt  aaa aat aag att aat      7578
   gac aat gag att cat  cag ttt aac aaa aac  aac tcc aat caa gca      7623
   gca gct gta act ttc  aca aag tgt gaa gaa  gaa cct tta gat tta      7668
   att aca agt ctt cag  aat gcc aga gat ata  cag gat atg cga att      7713
   aag aag aaa caa agg  caa cgc gtc ttt cca  cag cca ggc agt ctg      7758
   tat ctt gca aaa aca  tcc act ctg cct cga  atc tct ctg aaa gca       7803
   gca gta gga ggc caa  gtt ccc tct gcg tgt  tct cat aaa cag ctg       7848
   tat acg tat ggc gtt  tct aaa cat tgc ata  aaa att aac agc aaa       7893
   aat gca gag tct ttt  cag ttt cac act gaa  gat tat ttt ggt aag       7938
   gaa agt tta tgg act  gga aaa gga ata cag  ttg gct gat ggt gga       7983
   tgg ctc ata ccc tcc  aat gat gga aag gct  gga aaa gaa gaa ttt       8028
   tat agg gct ctg tgt  gac act cca ggt gtg  gat cca aag ctt att       8073
   tct aga att tgg gtt  tat aat cac tat aga  tgg atc ata tgg aaa       8118
   ctg gca gct atg gaa  tgt gcc ttt cct aag  gaa ttt gct aat aga       8163
   tgc cta agc cca gaa  agg gtg ctt ctt caa  cta aaa tac aga tat       8208
   gat acg gaa att gat  aga agc aga aga tcg  gct ata aaa aag ata       8253
   atg gaa agg gat gac  aca gct gca aaa aca  ctt gtt ctc tgt gtt       8298
   tct gac ata att tca  ttg agc gca aat ata  tct gaa act tct agc       8343
   aat aaa act agt agt  gca gat acc caa aaa  gtg gcc att att gaa       8388
   ctt aca gat ggg tgg  tat gct gtt aag gcc  cag tta gat cct ccc       8433
   ctc tta gct gtc tta  aag aat ggc aga ctg  aca gtt ggt cag aag       8478
   att att ctt cat gga  gca gaa ctg gtg ggc  tct cct gat gcc tgt       8523
   aca cct ctt gaa gcc cca gaa tct ctt atg tta aag att tct gct 8568
   aac agt act cgg cct gct cgc tgg tat acc aaa ctt gga ttc ttt 8613
   cct gac cct aga cct ttt cct ctg ccc tta tca tcg ctt ttc agt 8658
   gat gga gga aat gtt ggt tgt gtt gat gta att att caa aga gca 8703
   tac cct ata cag tgg atg gag aag aca tca tct gga tta tac ata 8748
   ttt cgc aat gaa aga gag gaa gaa aag gaa gca gca aaa tat gtg 8793
   gag gcc caa caa aag aga cta gaa gcc tta ttc act aaa att cag 8838
   gag gaa ttt gaa gaa cat gaa gaa aac aca aca aaa cca tat tta 8883
   cca tca cgt gca cta aca aga cag caa gtt cgt gct ttg caa gat 8928
   ggt gca gag ctt tat gaa gca gtg aag aat gca gca gac cca gct 8973
   tac ctt gag ggt tat ttc agt gaa gag cag tta aga gcc ttg aat 9018
   aat cac agg caa atg ttg aat gat aag aaa caa gct cag atc cag 9063
   ttg gaa att agg aag gcc atg gaa tct gct gaa caa aag gaa caa 9108
   ggt tta tca agg gat gtc aca acc gtg tgg aag ttg cgt att gta 9153
   agc tat tca aaa aaa gaa aaa gat tca gtt ata ctg agt att tgg 9198
   cgt cca tca tca gat tta tat tct ctg tta aca gaa gga aag aga 9243
   tac aga att tat cat ctt gca act tca aaa tct aaa agt aaa tct 9288
   gaa aga gct aac ata  cag tta gca gcg aca  aaa aaa act cag tat      9333
   caa caa cta ccg gtt  tca gat gaa att tta  ttt cag att tac cag      9378
   cca cgg gag ccc ctt  cac ttc agc aaa ttt  tta gat cca gac ttt      9423
   cag cca tct tgt tct  gag gtg gac cta ata  gga ttt gtc gtt tct      9468
   gtt gtg aaa aaa aca  gga ctt gcc cct ttc  gtc tat ttg tca gac      9513
   gaa tgt tac aat tta  ctg gca ata aag ttt  tgg ata gac ctt aat      9558
   gag gac att att aag  cct cat atg tta att  gct gca agc aac ctc      9603
   cag tgg cga cca gaa  tcc aaa tca ggc ctt  ctt act tta ttt gct      9648
   gga gat ttt tct gtg  ttt tct gct agt cca  aaa gag ggc cac ttt      9693
   caa gag aca ttc aac  aaa atg aaa aat act  gtt gag aat att gac      9738
   ata ctt tgc aat gaa  gca gaa aac aag ctt  atg cat ata ctg cat      9783
   gca aat gat ccc aag  tgg tcc acc cca act  aaa gac tgt act tca      9828
   ggg ccg tac act gct  caa atc att cct ggt  aca gga aac aag ctt      9873
   ctg atg tct tct cct  aat tgt gag ata tat  tat caa agt cct tta      9918
   tca ctt tgt atg gcc  aaa agg aag tct gtt  tcc aca cct gtc tca      9963
   gcc cag atg act tca  aag tct tgt aaa ggg  gag aaa gag att gat     10008
   gac caa aag aac tgc  aaa aag aga aga gcc  ttg gat ttc ttg agt     10053
   aga ctg cct tta cct  cca cct gtt agt ccc  att tgt aca ttt gtt        10098
   tct ccg gct gca cag  aag gca ttt cag cca  cca agg agt tgt ggc        10143
   acc aaa tac gaa aca  ccc ata aag aaa aaa  gaa ctg aat tct cct        10188
   cag atg act cca ttt  aaa aaa ttc aat gaa  att tct ctt ttg gaa        10233
   agt aat tca ata gct  gac gaa gaa ctt gca  ttg ata aat acc caa        10278
   gct ctt ttg tct ggt  tca aca gga gaa aaa  caa ttt ata tct gtc        10323
   agt gaa tcc act agg  act gct ccc acc agt  tca gaa gat tat ctc        10368
   aga ctg aaa cga cgt  tgt act aca tct ctg  atc aaa gaa cag gag        10413
   agt tcc cag gcc agt  acg gaa gaa tgt gag  aaa aat aag cag gac        10458
   aca att aca act aaa  aaa tat atc taa                                 10485
<210> 5
   <211> 3418
   <212> PRT
   <213> Homo sapiens
<400> 5
<210> 6
   <211> 10485
   <212> DNA
   <213> Homo sapiens
<220>
   <221> CDS
   <222> (229)..(10482)
   <223> BRCA2 (OMI2)
<400> 6
   ggtggcgcga gcttctgaaa ctaggcggca gaggcggagc cgctgtggca ctgctgcgcc      60
   tctgctgcgc ctcgggtgtc ttttgcggcg gtgggtcgcc gccgggagaa gcgtgagggg     120
   acagatttgt gaccggcgcg gtttttgtca gcttactccg gccaaaaaag aactgcacct     180
   ctggagcgga cttatttacc aagcattgga ggaatatcgt aggtaaaa atg cct att      237
   gga tcc aaa gag agg cca aca ttt ttt gaa att ttt aag aca cgc tgc      285
   aac aaa gca gat tta gga cca ata agt ctt aat tgg ttt gaa gaa ctt      333
   tct tca gaa gct cca ccc tat aat tct gaa cct gca gaa gaa tct gaa      381
   cat aaa aac aac aat tac gaa cca aac cta ttt aaa act cca caa agg      429
   aaa cca tct tat aat cag ctg gct tca act cca ata ata ttc aaa gag      477
   caa ggg ctg act ctg ccg ctg tac caa tct cct gta aaa gaa tta gat      525
   aaa ttc aaa tta gac tta gga agg aat gtt ccc aat agt aga cat aaa      573
   agt ctt cgc aca gtg aaa act aaa atg gat caa gca gat gat gtt tcc      621
   tgt cca ctt cta aat tct tgt ctt agt gaa agt cct gtt gtt cta caa      669
   tgt aca cat gta aca cca caa aga gat aag tca gtg gta tgt ggg agt      717
   ttg ttt cat aca cca aag ttt gtg aag ggt cgt cag aca cca aaa cat      765
   att tct gaa agt cta gga gct gag gtg gat cct gat atg tct tgg tca      813
   agt tct tta gct aca cca ccc acc ctt agt tct act gtg ctc ata gtc      861
   aga aat gaa gaa gca tct gaa act gta ttt cct cat gat act act gct      909
   aat gtg aaa agc tat ttt tcc aat cat gat gaa agt ctg aag aaa aat      957
   gat aga ttt atc gct tct gtg aca gac agt gaa aac aca aat caa aga     1005
   gaa gct gca agt cat gga ttt gga aaa aca tca ggg aat tca ttt aaa     1053
   gta aat agc tgc aaa gac cac att gga aag tca atg cca aat gtc cta     1101
   gaa gat gaa gta tat gaa aca gtt gta gat acc tct gaa gaa gat agt      1149
   ttt tca tta tgt ttt tct aaa tgt aga aca aaa aat cta caa aaa gta      1197
   aga act agc aag act agg aaa aaa att ttc cat gaa gca aac gct gat      1245
   gaa tgt gaa aaa tct aaa aac caa gtg aaa gaa aaa tac tca ttt gta      1293
   tct gaa gtg gaa cca aat gat act gat cca tta gat tca aat gta gca      1341
   cat cag aag ccc ttt gag agt gga agt gac aaa atc tcc aag gaa gtt      1389
   gta ccg tct ttg gcc tgt gaa tgg tct caa cta acc ctt tca ggt cta      1437
   aat gga gcc cag atg gag aaa ata ccc cta ttg cat att tct tca tgt      1485
   gac caa aat att tca gaa aaa gac cta tta gac aca gag aac aaa aga      1533
   aag aaa gat ttt ctt act tca gag aat tct ttg cca cgt att tct agc      1581
   cta cca aaa tca gag aag cca tta aat gag gaa aca gtg gta aat aag      1629
   aga gat gaa gag cag cat ctt gaa tct cat aca gac tgc att ctt gca      1677
   gta aag cag gca ata tct gga act tct cca gtg gct tct tca ttt cag      1725
   ggt atc aaa aag tct ata ttc aga ata aga gaa tca cct aaa gag act      1773
   ttc aat gca agt ttt tca ggt cat atg act gat cca aac ttt aaa aaa      1821
   gaa act gaa gcc tct gaa agt gga ctg gaa ata cat act gtt tgc tca      1869
   cag aag gag gac tcc tta tgt cca aat tta att gat aat gga agc tgg      1917
   cca gcc acc acc aca cag aat tct gta gct ttg aag aat gca ggt tta      1965
   ata tcc act ttg aaa aag aaa aca aat aag ttt att tat gct ata cat      2013
   gat gaa aca tct tat aaa gga aaa aaa ata ccg aaa gac caa aaa tca      2061
   gaa cta att aac tgt tca gcc cag ttt gaa gca aat gct ttt gaa gca      2109
   cca ctt aca ttt gca aat gct gat tca ggt tta ttg cat tct tct gtg      2157
   aaa aga agc tgt tca cag aat gat tct gaa gaa cca act ttg tcc tta      2205
   act agc tct ttt ggg aca att ctg agg aaa tgt tct aga aat gaa aca      2253
   tgt tct aat aat aca gta atc tct cag gat ctt gat tat aaa gaa gca      2301
   aaa tgt aat aag gaa aaa cta cag tta ttt att acc cca gaa gct gat      2349
   tct ctg tca tgc ctg cag gaa gga cag tgt gaa aat gat cca aaa agc      2397
   aaa aaa gtt tca gat ata aaa gaa gag gtc ttg gct gca gca tgt cac      2445
   cca gta caa cat tca aaa gtg gaa tac agt gat act gac ttt caa tcc      2493
   cag aaa agt ctt tta tat gat cat gaa aat gcc agc act ctt att tta      2541
   act cct act tcc aag gat gtt ctg tca aac cta gtc atg att tct aga      2589
   ggc aaa gaa tca tac aaa atg tca gac aag ctc aaa ggt aac aat tat      2637
   gaa tct gat gtt gaa tta acc aaa aat att ccc atg gaa aag aat caa      2685
   gat gta tgt gct tta aat gaa aat tat aaa aac gtt gag ctg ttg cca      2733
   cct gaa aaa tac atg aga gta gca tca cct tca aga aag gta caa ttc 2781
   aac caa aac aca aat cta aga gta atc caa aaa aat caa gaa gaa act 2829
   act tca att tca aaa ata act gtc aat cca gac tct gaa gaa ctt ttc 2877
   tca gac aat gag aat aat ttt gtc ttc caa gta gct aat gaa agg aat 2925
   aat ctt gct tta gga aat act aag gaa ctt cat gaa aca gac ttg act 2973
   tgt gta aac gaa ccc att ttc aag aac tct acc atg gtt tta tat gga 3021
   gac aca ggt gat aaa caa gca acc caa gtg tca att aaa aaa gat ttg 3069
   gtt tat gtt ctt gca gag gag aac aaa aat agt gta aag cag cat ata 3117
   aaa atg act cta ggt caa gat tta aaa tcg gac atc tcc ttg aat ata 3165
   gat aaa ata cca gaa aaa aat aat gat tac atg aac aaa tgg gca gga 3213
   ctc tta ggt cca att tca aat cac agt ttt gga ggt agc ttc aga 3258
   aca gct tca aat aag gaa atc aag ctc tct gaa cat aac att aag 3303
   aag agc aaa atg ttc ttc aaa gat att gaa gaa caa tat cct act 3348
   agt tta gct tgt gtt gaa att gta aat acc ttg gca tta gat aat 3393
   caa aag aaa ctg agc aag cct cag tca att aat act gta tct gca 3438
   cat tta cag agt agt gta gtt gtt tct gat tgt aaa aat agt cat 3483
   ata acc cct cag atg tta ttt tcc aag cag gat ttt aat tca aac 3528
   cat aat tta aca cct agc caa aag gca gaa att aca gaa ctt tct 3573
   act ata tta gaa gaa tca gga agt cag ttt gaa ttt act cag ttt 3618
   aga aar cca agc tac ata ttg cag aag agt aca ttt gaa gtg cct 3663
   gaa aac cag atg act atc tta aag acc act tct gag gaa tgc aga 3708
   gat gct gat ctt cat gtc ata atg aat gcc cca tcg att ggt cag 3753
   gta gac agc agc aag caa ttt gaa ggt aca gtt gaa att aaa cgg 3798
   aag ttt gct ggc ctg ttg aaa aat gac tgt aac aaa agt gct tct 3843
   ggt tat tta aca gat gaa aat gaa gtg ggg ttt agg ggc ttt tat 3888
   tct gct cat ggc aca aaa ctg aat gtt tct act gaa gct ctg caa 3933
   aaa gct gtg aaa ctg ttt agt gat att gag aat att agt gag gaa 3978
   act tct gca gag gta cat cca ata agt tta tct tca agt aaa tgt 4023
   cat gat tct gtc gtt tca atg ttt aag ata gaa aat cat aat gat 4068
   aaa act gta agt gaa aaa aat aat aaa tgc caa ctg ata tta caa 4113
   aat aat att gaa atg act act ggc act ttt gtt gaa gaa att act 4158
   gaa aat tac aag aga aat act gaa aat gaa gat aac aaa tat act 4203
   gct gcc agt aga aat tct cat aac tta gaa ttt gat ggc agt gat 4248
   tca agt aaa aat gat act gtt tgt att cat aaa gat gaa acg gac 4293
   ttg cta ttt act gat cag cac aac ata tgt ctt aaa tta tct ggc 4338
   cag ttt atg aag gag  gga aac act cag att  aaa gaa gat ttg tca      4383
   gat tta act ttt ttg  gaa gtt gcg aaa gct  caa gaa gca tgt cat      4428
   ggt aat act tca aat  aaa gaa cag tta act  gct act aaa acg gag      4473
   caa aat ata aaa gat  ttt gag act tct gat  aca ttt ttt cag act      4518
   gca agt ggg aaa aat  att agt gtc gcc aaa  gag tca ttt aat aaa      4563
   att gta aat ttc ttt  gat cag aaa cca gaa  gaa ttg cat aac ttt      4608
   tcc tta aat tct gaa  tta cat tct gac ata  aga aag aac aaa atg      4653
   gac att cta agt tat  gag gaa aca gac ata  gtt aaa cac aaa ata      4698
   ctg aaa gaa agt gtc  cca gtt ggt act gga  aat caa cta gtg acc      4743
   ttc cag gga caa ccc  gaa cgt gat gaa aag  atc aaa gaa cct act      4788
   ctg ttg ggt ttt cat  aca gct agc ggg aaa  aaa gtt aaa att gca      4833
   aag gaa tct ttg gac  aaa gtg aaa aac ctt  ttt gat gaa aaa gag      4878
   caa ggt act agt gaa  atc acc agt ttt agc  cat caa tgg gca aag      4923
   acc cta aag tac aga  gag gcc tgt aaa gac  ctt gaa tta gca tgt      4968
   gag acc att gag atc  aca gct gcc cca aag  tgt aaa gaa atg cag      5013
   aat tct ctc aat aat  gat aaa aac ctt gtt  tct att gag act gtg      5058
   gtg cca cct aag ctc  tta agt gat aat tta  tgt aga caa act gaa      5103
   aat ctc aaa aca tca aaa agt atc ttt ttg aaa gtt aaa gta cat      5148
   gaa aat gta gaa aaa gaa aca gca aaa agt cct gca act tgt tac      5193
   aca aat cag tcc cct tat tca gtc att gaa aat tca gcc tta gct      5238
   ttt tac aca agt tgt agt aga aaa act tct gtg agt cag act tca      5283
   tta ctt gaa gca aaa aaa tgg ctt aga gaa gga ata ttt gat ggt      5328
   caa cca gaa aga ata aat act gca gat tat gta gga aat tat ttg      5373
   tat gaa aat aat tca aac agt act ata gct gaa aat gac aaa aat      5418
   cat ctc tcc gaa aaa caa gat act tat tta agt aac agt agc atg      5463
   tct aac agc tat tcc tac cat tct gat gag gta tat aat gat tca      5508
   gga tat ctc tca aaa aat aaa ctt gat tct ggt att gag cca gta      5553
   ttg aag aat gtt gaa gat caa aaa aac act agt ttt tcc aaa gta      5598
   ata tcc aat gta aaa gat gca aat gca tac cca caa act gta aat      5643
   gaa gat att tgc gtt gag gaa ctt gtg act agc tct tca ccc tgc      5688
   aaa aat aaa aat gca gcc att aaa ttg tcc ata tct aat agt aat      5733
   aat ttt gag gta ggg cca cct gca ttt agg ata gcc agt ggt aaa      5778
   atc gtt tgt gtt tca cat gaa aca att aaa aaa gtg aaa gac ata      5823
   ttt aca gac agt ttc agt aaa gta att aag gaa aac aac gag aat      5868
   aaa tca aaa att tgc  caa acg aaa att atg  gca ggt tgt tac gag       5913
   gca ttg gat gat tca  gag gat att ctt cat  aac tct cta gat aat       5958
   gat gaa tgt agc acg  cat tca cat aag gtt  ttt gct gac att cag       6003
   agt gaa gaa att tta  caa cat aac caa aat  atg tct gga ttg gag       6048
   aaa gtt tct aaa ata  tca cct tgt gat gtt  agt ttg gaa act tca       6093
   gat ata tgt aaa tgt  agt ata ggg aag ctt  cat aag tca gtc tca       6138
   tct gca aat act tgt  ggg att ttt agc aca  gca agt gga aaa tct       6183
   gtc cag gta tca gat  gct tca tta caa aac  gca aga caa gtg ttt       6228
   tct gaa ata gaa gat  agt acc aag caa gtc  ttt tcc aaa gta ttg       6273
   ttt aaa agt aac gaa  cat tca gac cag ctc  aca aga gaa gaa aat       6318
   act gct ata cgt act  cca gaa cat tta ata  tcc caa aaa ggc ttt       6363
   tca tat aat gtg gta  aat tca tct gct ttc  tct gga ttt agt aca       6408
   gca agt gga aag caa  gtt tcc att tta gaa  agt tcc tta cac aaa       6453
   gtt aag gga gtg tta  gag gaa ttt gat tta  atc aga act gag cat       6498
   agt ctt cac tat tca  cct acg tct aga caa  aat gta tca aaa ata       6543
   ctt cct cgt gtt gat  aag aga aac cca gag  cac tgt gta aac tca       6588
   gaa atg gaa aaa acc  tgc agt aaa gaa ttt  aaa tta tca aat aac       6633
   tta aat gtt gaa ggt  ggt tct tca gaa aat  aat cac tct att aaa       6678
   gtt tct cca tat ctc  tct caa ttt caa caa  gac aaa caa cag ttg       6723
   gta tta gga acc aaa  gtc tca ctt gtt gag  aac att cat gtt ttg       6768
   gga aaa gaa cag gct  tca cct aaa aac gta  aaa atg gaa att ggt       6813
   aaa act gaa act ttt  tct gat gtt cct gtg  aaa aca aat ata gaa       6858
   gtt tgt tct act tac  tcc aaa gat tca gaa  aac tac ttt gaa aca       6903
   gaa gca gta gaa att  gct aaa gct ttt atg  gaa gat gat gaa ctg       6948
   aca gat tct aaa ctg  cca agt cat gcc aca  cat tct ctt ttt aca       6993
   tgt ccc gaa aat gag  gaa atg gtt ttg tca  aat tca aga att gga       7038
   aaa aga aga gga gag  ccc ctt atc tta gtg  gga gaa ccc tca atc       7083
   aaa aga aac tta tta  aat gaa ttt gac agg  ata ata gaa aat caa       7128
   gaa aaa tcc tta aag  gct tca aaa agc act  cca gat ggc aca ata       7173
   aaa gat cga aga ttg  ttt atg cat cat gtt  tct tta gag ccg att       7218
   acc tgt gta ccc ttt  cgc aca act aag gaa  cgt caa gag ata cag       7263
   aat cca aat ttt acc  gca cct ggt caa gaa  ttt ctg tct aaa tct       7308
   cat ttg tat gaa cat  ctg act ttg gaa aaa  tct tca agc aat tta       7353
   gca gtt tca gga cat  cca ttt tat caa gtt  tct gct aca aga aat       7398
   gaa aaa atg aga cac  ttg att act aca ggc  aga cca acc aaa gtc       7443
   ttt gtt cca cct ttt  aaa act aaa tca cat  ttt cac aga gtt gaa       7488
   cag tgt gtt agg aat  att aac ttg gag gaa  aac aga caa aag caa       7533
   aac att gat gga cat  ggc tct gat gat agt  aaa aat aag att aat       7578
   gac aat gag att cat  cag ttt aac aaa aac  aac tcc aat caa gca       7623
   gca gct gta act ttc  aca aag tgt gaa gaa  gaa cct tta gat tta       7668
   att aca agt ctt cag  aat gcc aga gat ata  cag gat atg cga att       7713
   aag aag aaa caa agg  caa cgc gtc ttt cca  cag cca ggc agt ctg       7758
   tat ctt gca aaa aca  tcc act ctg cct cga  atc tct ctg aaa gca       7803
   gca gta gga ggc caa  gtt ccc tct gcg tgt  tct cat aaa cag ctg       7848
   tat acg tat ggc gtt  tct aaa cat tgc ata  aaa att aac agc aaa       7893
   aat gca gag tct ttt  cag ttt cac act gaa  gat tat ttt ggt aag       7938
   gaa agt tta tgg act  gga aaa gga ata cag  ttg gct gat ggt gga       7983
   tgg ctc ata ccc tcc  aat gat gga aag gct  gga aaa gaa gaa ttt       8028
   tat agg gct ctg tgt  gac act cca ggt gtg  gat cca aag ctt att       8073
   tct aga att tgg gtt  tat aat cac tat aga  tgg atc ata tgg aaa       8118
   ctg gca gct atg gaa  tgt gcc ttt cct aag  gaa ttt gct aat aga       8163
   tgc cta agc cca gaa  agg gtg ctt ctt caa  cta aaa tac aga tat       8208
   gat acg gaa att gat aga agc aga aga tcg gct ata aaa aag ata 8253
   atg gaa agg gat gac aca gct gca aaa aca ctt gtt ctc tgt gtt 8298
   tct gac ata att tca ttg agc gca aat ata tct gaa act tct agc 8343
   aat aaa act agt agt gca gat acc caa aaa gtg gcc att att gaa 8388
   ctt aca gat ggg tgg tat gct gtt aag gcc cag tta gat cct ccc 8433
   ctc tta gct gtc tta aag aat ggc aga ctg aca gtt ggt cag aag 8478
   att att ctt cat gga gca gaa ctg gtg ggc tct cct gat gcc tgt 8523
   aca cct ctt gaa gcc cca gaa tct ctt atg tta aag att tct gct 8568
   aac agt act cgg cct gct cgc tgg tat acc aaa ctt gga ttc ttt 8613
   cct gac cct aga cct ttt cct ctg ccc tta tca tcg ctt ttc agt 8658
   gat gga gga aat gtt ggt tgt gtt gat gta att att caa aga gca 8703
   tac cct ata cag tgg atg gag aag aca tca tct gga tta tac ata 8748
   ttt cgc aat gaa aga gag gaa gaa aag gaa gca gca aaa tat gtg 8793
   gag gcc caa caa aag aga cta gaa gcc tta ttc act aaa att cag 8838
   gag gaa ttt gaa gaa cat gaa gaa aac aca aca aaa cca tat tta 8883
   cca tca cgt gca cta aca aga cag caa gtt cgt gct ttg caa gat 8928
   ggt gca gag ctt tat gaa gca gtg aag aat gca gca gac cca gct 8973
   tac ctt gag ggt tat  ttc agt gaa gag cag  tta aga gcc ttg aat     9018
   aat cac agg caa atg  ttg aat gat aag aaa  caa gct cag atc cag     9063
   ttg gaa att agg aag  gcc atg gaa tct gct  gaa caa aag gaa caa     9108
   ggt tta tca agg gat  gtc aca acc gtg tgg  aag ttg cgt att gta     9153
   agc tat tca aaa aaa  gaa aaa gat tca gtt  ata ctg agt att tgg     9198
   cgt cca tca tca gat  tta tat tct ctg tta  aca gaa gga aag aga     9243
   tac aga att tat cat  ctt gca act tca aaa  tct aaa agt aaa tct     9288
   gaa aga gct aac ata  cag tta gca gcg aca  aaa aaa act cag tat     9333
   caa caa cta ccg gtt  tca gat gaa att tta  ttt cag att tac cag     9378
   cca cgg gag ccc ctt  cac ttc agc aaa ttt  tta gat cca gac ttt     9423
   cag cca tct tgt tct  gag gtg gac cta ata  gga ttt gtc gtt tct     9468
   gtt gtg aaa aaa aca  gga ctt gcc cct ttc  gtc tat ttg tca gac     9513
   gaa tgt tac aat tta  ctg gca ata aag ttt  tgg ata gac ctt aat     9558
   gag gac att att aag  cct cat atg tta att  gct gca agc aac ctc     9603
   cag tgg cga cca gaa  tcc aaa tca ggc ctt  ctt act tta ttt gct     9648
   gga gat ttt tct gtg  ttt tct gct agt cca  aaa gag ggc cac ttt     9693
   caa gag aca ttc aac  aaa atg aaa aat act  gtt gag aat att gac     9738
   ata ctt tgc aat gaa gca gaa aac aag ctt atg cat ata ctg cat      9783
   gca aat gat ccc aag tgg tcc acc cca act aaa gac tgt act tca      9828
   ggg ccg tac act gct caa atc att cct ggt aca gga aac aag ctt      9873
   ctg atg tct tct cct aat tgt gag ata tat tat caa agt cct tta      9918
   tca ctt tgt atg gcc aaa agg aag tct gtt tcc aca cct gtc tca      9963
   gcc cag atg act tca aag tct tgt aaa ggg gag aaa gag att gat      10008
   gac caa aag aac tgc aaa aag aga aga gcc ttg gat ttc ttg agt      10053
   aga ctg cct tta cct cca cct gtt agt ccc att tgt aca ttt gtt      10098
   tct ccg gct gca cag aag gca ttt cag cca cca agg agt tgt ggc      10143
   acc aaa tac gaa aca ccc ata aag aaa aaa gaa ctg aat tct cct      10188
   cag atg act cca ttt aaa aaa ttc aat gaa att tct ctt ttg gaa      10233
   agt aat tca ata gct gac gaa gaa ctt gca ttg ata aat acc caa      10278
   gct ctt ttg tct ggt tca aca gga gaa aaa caa ttt ata tct gtc      10323
   agt gaa tcc act agg act gct ccc acc agt tca gaa gat tat ctc      10368
   aga ctg aaa cga cgt tgt act aca tct ctg atc aaa gaa cag gag      10413
   agt tcc cag gcc agt acg gaa gaa tgt gag aaa aat aag cag gac      10458
   aca att aca act aaa aaa tat atc taa                              10485
<210> 7
   <211> 3418
   <212> PRT
   <213> Homo sapiens
<400> 7
<210> 8
   <211> 10485
   <212> DNA
   <213> Homo sapiens
<220>
   <221> CDS
   <222> (229)..(10482)
   <223> BRCA2 (OMI3)
<400> 8
   ggtggcgcga gcttctgaaa ctaggcggca gaggcggagc cgctgtggca ctgctgcgcc        60
   tctgctgcgc ctcgggtgtc ttttgcggcg gtgggtcgcc gccgggagaa gcgtgagggg       120
   acagatttgt gaccggcgcg gtttttgtca gcttactccg gccaaaaaag aactgcacct       180
   ctggagcgga cttatttacc aagcattgga ggaatatcgt aggtaaaa atg cct att        237
                                                        Met Pro Ile
                                                        1
   gga tcc aaa gag agg cca aca ttt ttt gaa att ttt aag aca cgc tgc         285
   Gly Ser Lys Glu Arg Pro Thr Phe Phe Glu Ile Phe Lys Thr Arg Cys
       5                   10                  15
   aac aaa gca gat tta gga cca ata agt ctt aat tgg ttt gaa gaa ctt         333
   Asn Lys Ala Asp Leu Gly Pro Ile Ser Leu Asn Trp Phe Glu Glu Leu
   20                  25                  30                  35
   tct tca gaa gct cca ccc tat aat tct gaa cct gca gaa gaa tct gaa         381
   Ser Ser Glu Ala Pro Pro Tyr Asn Ser Glu Pro Ala Glu Glu Ser Glu
                   40                  45                  50
   cat aaa aac aac aat tac gaa cca aac cta ttt aaa act cca caa agg         429
   His Lys Asn Asn Asn Tyr Glu Pro Asn Leu Phe Lys Thr Pro Gln Arg
               55                  60                  65
   aaa cca tct tat aat cag ctg gct tca act cca ata ata ttc aaa gag         477
   Lys Pro Ser Tyr Asn Gln Leu Ala Ser Thr Pro Ile Ile Phe Lys Glu
           70                  75                  80
   caa ggg ctg act ctg ccg ctg tac caa tct cct gta aaa gaa tta gat         525
   Gln Gly Leu Thr Leu Pro Leu Tyr Gln Ser Pro Val Lys Glu Leu Asp
       85                  90                  95
   aaa ttc aaa tta gac tta gga agg aat gtt ccc aat agt aga cat aaa         573
   Lys Phe Lys Leu Asp Leu Gly Arg Asn Val Pro Asn Ser Arg His Lys
   100                 105                 110                 115
   agt ctt cgc aca gtg aaa act aaa atg gat caa gca gat gat gtt tcc         621
   Ser Leu Arg Thr Val Lys Thr Lys Met Asp Gln Ala Asp Asp Val Ser
                   120                 125                 130
   tgt cca ctt cta aat tct tgt ctt agt gaa agt cct gtt gtt cta caa         669
   Cys Pro Leu Leu Asn Ser Cys Leu Ser Glu Ser Pro Val Val Leu Gln
               135                 140                 145
   tgt aca cat gta aca cca caa aga gat aag tca gtg gta tgt ggg agt         717
   Cys Thr His Val Thr Pro Gln Arg Asp Lys Ser Val Val Cys Gly Ser
           150                 155                 160
   ttg ttt cat aca cca aag ttt gtg aag ggt cgt cag aca cca aaa cat         765
   Leu Phe His Thr Pro Lys Phe Val Lys Gly Arg Gln Thr Pro Lys His
   165 170 175
   att tct gaa agt cta gga gct gag gtg gat cct gat atg tct tgg tca 813
   Ile Ser Glu Ser Leu Gly Ala Glu Val Asp Pro Asp Met Ser Trp Ser
   180 185 190 195
   agt tct tta gct aca cca ccc acc ctt agt tct act gtg ctc ata gtc 861
   Ser Ser Leu Ala Thr Pro Pro Thr Leu Ser Ser Thr Val Leu Ile Val
   200 205 210
   aga aat gaa gaa gca tct gaa act gta ttt cct cat gat act act gct 909
   Arg Asn Glu Glu Ala Ser Glu Thr Val Phe Pro His Asp Thr Thr Ala
   215 220 225
   aat gtg aaa agc tat ttt tcc aat cat gat gaa agt ctg aag aaa aat 957
   Asn Val Lys Ser Tyr Phe Ser Asn His Asp Glu Ser Leu Lys Lys Asn
   230 235 240
   gat aga ttt atc gct tct gtg aca gac agt gaa aac aca aat caa aga 1005
   Asp Arg Phe Ile Ala Ser Val Thr Asp Ser Glu Asn Thr Asn Gln Arg
   245 250 255
   gaa gct gca agt cat gga ttt gga aaa aca tca ggg aat tca ttt aaa 1053
   Glu Ala Ala Ser His Gly Phe Gly Lys Thr Ser Gly Asn Ser Phe Lys
   260 265 270 275
   gta aat agc tgc aaa gac cac att gga aag tca atg cca cat gtc cta 1101
   Val Asn Ser Cys Lys Asp His Ile Gly Lys Ser Met Pro His Val Leu
   280 285 290
   gaa gat gaa gta tat gaa aca gtt gta gat acc tct gaa gaa gat agt 1149
   Glu Asp Glu Val Tyr Glu Thr Val Val Asp Thr Ser Glu Glu Asp Ser
   295 300 305
   ttt tca tta tgt ttt tct aaa tgt aga aca aaa aat cta caa aaa gta 1197
   Phe Ser Leu Cys Phe Ser Lys Cys Arg Thr Lys Asn Leu Gln Lys Val
   310 315 320
   aga act agc aag act agg aaa aaa att ttc cat gaa gca aac gct gat 1245
   Arg Thr Ser Lys Thr Arg Lys Lys Ile Phe His Glu Ala Asn Ala Asp
   325 330 335
   gaa tgt gaa aaa tct aaa aac caa gtg aaa gaa aaa tac tca ttt gta 1293
   Glu Cys Glu Lys Ser Lys Asn Gln Val Lys Glu Lys Tyr Ser Phe Val
   340 345 350 355
   tct gaa gtg gaa cca aat gat act gat cca tta gat tca aat gta gca 1341
   Ser Glu Val Glu Pro Asn Asp Thr Asp Pro Leu Asp Ser Asn Val Ala
   360 365 370
   aat cag aag ccc ttt gag agt gga agt gac aaa atc tcc aag gaa gtt 1389
   Asn Gln Lys Pro Phe Glu Ser Gly Ser Asp Lys Ile Ser Lys Glu Val
   375 380 385
   gta ccg tct ttg gcc tgt gaa tgg tct caa cta acc ctt tca ggt cta 1437
   Val Pro Ser Leu Ala Cys Glu Trp Ser Gln Leu Thr Leu Ser Gly Leu
   390 395 400
   aat gga gcc cag atg gag aaa ata ccc cta ttg cat att tct tca tgt 1485
   Asn Gly Ala Gln Met Glu Lys Ile Pro Leu Leu His Ile Ser Ser Cys
   405 410 415
   gac caa aat att tca gaa aaa gac cta tta gac aca gag aac aaa aga 1533
   Asp Gln Asn Ile Ser Glu Lys Asp Leu Leu Asp Thr Glu Asn Lys Arg
   420 425 430 435
   aag aaa gat ttt ctt act tca gag aat tct ttg cca cgt att tct agc 1581
   Lys Lys Asp Phe Leu Thr Ser Glu Asn Ser Leu Pro Arg Ile Ser Ser
                       440                     445                     450
   cta cca aaa tca gag aag cca tta aat gag gaa aca gtg gta aat aag        1629
   Leu Pro Lys Ser Glu Lys Pro Leu Asn Glu Glu Thr Val Val Asn Lys
               455                 460                     465
   aga gat gaa gag cag cat ctt gaa tct cat aca gac tgc att ctt gca        1677
   Arg Asp Glu Glu Gln His Leu Glu Ser His Thr Asp Cys Ile Leu Ala
           470                 475                 480
   gta aag cag gca ata tct gga act tct cca gtg gct tct tca ttt cag        1725
   Val Lys Gln Ala Ile Ser Gly Thr Ser Pro Val Ala Ser Ser Phe Gln
       485                 490                 495
   ggt atc aaa aag tct ata ttc aga ata aga gaa tca cct aaa gag act        1773
   Gly Ile Lys Lys Ser Ile Phe Arg Ile Arg Glu Ser Pro Lys Glu Thr
   500                 505                 510                     515
   ttc aat gca agt ttt tca ggt cat atg act gat cca aac ttt aaa aaa        1821
   Phe Asn Ala Ser Phe Ser Gly His Met Thr Asp Pro Asn Phe Lys Lys
                   520                 525                 530
   gaa act gaa gcc tct gaa agt gga ctg gaa ata cat act gtt tgc tca        1869
   Glu Thr Glu Ala Ser Glu Ser Gly Leu Glu Ile His Thr Val Cys Ser
               535                 540                 545
   cag aag gag gac tcc tta tgt cca aat tta att gat aat gga agc tgg        1917
   Gln Lys Glu Asp Ser Leu Cys Pro Asn Leu Ile Asp Asn Gly Ser Trp
           550                 555                 560
   cca gcc acc acc aca cag aat tct gta gct ttg aag aat gca ggt tta        1965
   Pro Ala Thr Thr Thr Gln Asn Ser Val Ala Leu Lys Asn Ala Gly Leu
       565                 570                 575
   ata tcc act ttg aaa aag aaa aca aat aag ttt att tat gct ata cat        2013
   Ile Ser Thr Leu Lys Lys Lys Thr Asn Lys Phe Ile Tyr Ala Ile His
   580                 585                 590                     595
   gat gaa aca tct tat aaa gga aaa aaa ata ccg aaa gac caa aaa tca        2061
   Asp Glu Thr Ser Tyr Lys Gly Lys Lys Ile Pro Lys Asp Gln Lys Ser
                   600                 605                 610
   gaa cta att aac tgt tca gcc cag ttt gaa gca aat gct ttt gaa gca        2109
   Glu Leu Ile Asn Cys Ser Ala Gln Phe Glu Ala Asn Ala Phe Glu Ala
               615                 620                 625
   cca ctt aca ttt gca aat gct gat tca ggt tta ttg cat tct tct gtg        2157
   Pro Leu Thr Phe Ala Asn Ala Asp Ser Gly Leu Leu His Ser Ser Val
           630                 635                 640
   aaa aga agc tgt tca cag aat gat tct gaa gaa cca act ttg tcc tta        2205
   Lys Arg Ser Cys Ser Gln Asn Asp Ser Glu Glu Pro Thr Leu Ser Leu
       645                 650                 655
   act agc tct ttt ggg aca att ctg agg aaa tgt tct aga aat gaa aca        2253
   Thr Ser Ser Phe Gly Thr Ile Leu Arg Lys Cys Ser Arg Asn Glu Thr
   660                 665                 670                     675
   tgt tct aat aat aca gta atc tct cag gat ctt gat tat aaa gaa gca        2301
   Cys Ser Asn Asn Thr Val Ile Ser Gln Asp Leu Asp Tyr Lys Glu Ala
                   680                 685                 690
   aaa tgt aat aag gaa aaa cta cag tta ttt att acc cca gaa gct gat        2349
   Lys Cys Asn Lys Glu Lys Leu Gln Leu Phe Ile Thr Pro Glu Ala Asp
               695                 700                 705
   tct ctg tca tgc ctg cag gaa gga cag tgt gaa aat gat cca aaa agc        2397
   Ser Leu Ser Cys Leu Gln Glu Gly Gln Cys Glu Asn Asp Pro Lys Ser
           710                 715                 720
   aaa aaa gtt tca gat ata aaa gaa gag gtc ttg gct gca gca tgt cac        2445
   Lys Lys Val Ser Asp Ile Lys Glu Glu Val Leu Ala Ala Ala Cys His
       725                 730                 735
   cca gta caa cac tca aaa gtg gaa tac agt gat act gac ttt caa tcc        2493
   Pro Val Gln His Ser Lys Val Glu Tyr Ser Asp Thr Asp Phe Gln Ser
   740                 745                 750                 755
   cag aaa agt ctt tta tat gat cat gaa aat gcc agc act ctt att tta        2541
   Gln Lys Ser Leu Leu Tyr Asp His Glu Asn Ala Ser Thr Leu Ile Leu
                   760                 765                 770
   act cct act tcc aag gat gtt ctg tca aac cta gtc atg att tct aga        2589
   Thr Pro Thr Ser Lys Asp Val Leu Ser Asn Leu Val Met Ile Ser Arg
               775                 780                 785
   ggc aaa gaa tca tac aaa atg tca gac aag ctc aaa ggt aac aat tat        2637
   Gly Lys Glu Ser Tyr Lys Met Ser Asp Lys Leu Lys Gly Asn Asn Tyr
           790                 795                 800
   gaa tct gat gtt gaa tta acc aaa aat att ccc atg gaa aag aat caa        2685
   Glu Ser Asp Val Glu Leu Thr Lys Asn Ile Pro Met Glu Lys Asn Gln
       805                 810                 815
   gat gta tgt gct tta aat gaa aat tat aaa aac gtt gag ctg ttg cca        2733
   Asp Val Cys Ala Leu Asn Glu Asn Tyr Lys Asn Val Glu Leu Leu Pro
   820                 825                 830                 835
   cct gaa aaa tac atg aga gta gca tca cct tca aga aag gta caa ttc        2781
   Pro Glu Lys Tyr Met Arg Val Ala Ser Pro Ser Arg Lys Val Gln Phe
                   840                 845                 850
   aac caa aac aca aat cta aga gta atc caa aaa aat caa gaa gaa act        2829
   Asn Gln Asn Thr Asn Leu Arg Val Ile Gln Lys Asn Gln Glu Glu Thr
               855                 860                 865
   act tca att tca aaa ata act gtc aat cca gac tct gaa gaa ctt ttc        2877
   Thr Ser Ile Ser Lys Ile Thr Val Asn Pro Asp Ser Glu Glu Leu Phe
           870                 875                 880
   tca gac aat gag aat aat ttt gtc ttc caa gta gct aat gaa agg aat        2925
   Ser Asp Asn Glu Asn Asn Phe Val Phe Gln Val Ala Asn Glu Arg Asn
       885                 890                 895
   aat ctt gct tta gga aat act aag gaa ctt cat gaa aca gac ttg act        2973
   Asn Leu Ala Leu Gly Asn Thr Lys Glu Leu His Glu Thr Asp Leu Thr
   900                 905                 910                 915
   tgt gta aac gaa ccc att ttc aag aac tct acc atg gtt tta tat gga        3021
   Cys Val Asn Glu Pro Ile Phe Lys Asn Ser Thr Met Val Leu Tyr Gly
                   920                 925                 930
   gac aca ggt gat aaa caa gca acc caa gtg tca att aaa aaa gat ttg        3069
   Asp Thr Gly Asp Lys Gln Ala Thr Gln Val Ser Ile Lys Lys Asp Leu
               935                 940                 945
   gtt tat gtt ctt gca gag gag aac aaa aat agt gta aag cag cat ata        3117
   Val Tyr Val Leu Ala Glu Glu Asn Lys Asn Ser Val Lys Gln His Ile
           950                 955                 960
   aaa atg act cta ggt caa gat tta aaa tcg gac atc tcc ttg aat ata        3165
   Lys Met Thr Leu Gly Gln Asp Leu Lys Ser Asp Ile Ser Leu Asn Ile
       965                 970                 975
   gat aaa ata cca gaa aaa aat aat gat tac atg gac aaa tgg gca gga        3213
   Asp Lys Ile Pro Glu Lys Asn Asn Asp Tyr Met Asp Lys Trp Ala Gly
   980                 985                 990                 995
   ctc tta ggt cca att  tca aat cac agt ttt  gga ggt agc ttc aga       3258
   Leu Leu Gly Pro Ile  Ser Asn His Ser Phe  Gly Gly Ser Phe Arg
                   1000                 1005                 1010
   aca gct tca aat aag  gaa atc aag ctc tct  gaa cat aac att aag       3303
   Thr Ala Ser Asn Lys  Glu Ile Lys Leu Ser  Glu His Asn Ile Lys
                   1015                 1020                 1025
   aag agc aaa atg ttc  ttc aaa gat att gaa  gaa caa tat cct act       3348
   Lys Ser Lys Met Phe  Phe Lys Asp Ile Glu  Glu Gln Tyr Pro Thr
                   1030                 1035                 1040
   agt tta gct tgt gtt  gaa att gta aat acc  ttg gca tta gat aat       3393
   Ser Leu Ala Cys Val  Glu Ile Val Asn Thr  Leu Ala Leu Asp Asn
                   1045                 1050                 1055
   caa aag aaa ctg agc  aag cct cag tca att  aat act gta tct gca       3438
   Gln Lys Lys Leu Ser  Lys Pro Gln Ser Ile  Asn Thr Val Ser Ala
                   1060                 1065                 1070
   cat tta cag agt agt  gta gtt gtt tct gat  tgt aaa aat agt cat       3483
   His Leu Gln Ser Ser  Val Val Val Ser Asp  Cys Lys Asn Ser His
                   1075                 1080                 1085
   ata acc cct cag atg  tta ttt tcc aag cag  gat ttt aat tca aac       3528
   Ile Thr Pro Gln Met  Leu Phe Ser Lys Gln  Asp Phe Asn Ser Asn
                   1090                 1095                 1100
   cat aat tta aca cct  agc caa aag gca gaa  att aca gaa ctt tct       3573
   His Asn Leu Thr Pro  Ser Gln Lys Ala Glu  Ile Thr Glu Leu Ser
                   1105                 1110                 1115
   act ata tta gaa gaa  tca gga agt cag ttt  gaa ttt act cag ttt       3618
   Thr Ile Leu Glu Glu  Ser Gly Ser Gln Phe  Glu Phe Thr Gln Phe
                   1120                 1125                 1130
   aga aag cca agc tac  ata ttg cag aag agt  aca ttt gaa gtg cct       3663
   Arg Lys Pro Ser Tyr  Ile Leu Gln Lys Ser  Thr Phe Glu Val Pro
                   1135                 1140                 1145
   gaa aac cag atg act  atc tta aag acc act  tct gag gaa tgc aga       3708
   Glu Asn Gln Met Thr  Ile Leu Lys Thr Thr  Ser Glu Glu Cys Arg
                   1150                 1155                 1160
   gat gct gat ctt cat  gtc ata atg aat gcc  cca tcg att ggt cag       3753
   Asp Ala Asp Leu His  Val Ile Met Asn Ala  Pro Ser Ile Gly Gln
                   1165                 1170                 1175
   gta gac agc agc aag  caa ttt gaa ggt aca  gtt gaa att aaa cgg       3798
   Val Asp Ser Ser Lys  Gln Phe Glu Gly Thr  Val Glu Ile Lys Arg
                   1180                 1185                 1190
   aag ttt gct ggc ctg  ttg aaa aat gac tgt  aac aaa agt gct tct       3843
   Lys Phe Ala Gly Leu  Leu Lys Asn Asp Cys  Asn Lys Ser Ala Ser
                   1195                 1200                 1205
   ggt tat tta aca gat  gaa aat gaa gtg ggg  ttt agg ggc ttt tat       3888
   Gly Tyr Leu Thr Asp  Glu Asn Glu Val Gly  Phe Arg Gly Phe Tyr
                   1210                 1215                 1220
   tct gct cat ggc aca  aaa ctg aat gtt tct  act gaa gct ctg caa       3933
   Ser Ala His Gly Thr  Lys Leu Asn Val Ser  Thr Glu Ala Leu Gln
                   1225                 1230                 1235
   aaa gct gtg aaa ctg  ttt agt gat att gag  aat att agt gag gaa       3978
   Lys Ala Val Lys Leu  Phe Ser Asp Ile Glu  Asn Ile Ser Glu Glu
                   1240                 1245                 1250
   act tct gca gag gta  cat cca ata agt tta  tct tca agt aaa tgt       4023
   Thr Ser Ala Glu Val  His Pro Ile Ser Leu  Ser Ser Ser Lys Cys
                   1255                 1260                 1265
   cat gat tct gtt gtt  tca atg ttt aag ata  gaa aat cat aat gat       4068
   His Asp Ser Val Val  Ser Met Phe Lys Ile  Glu Asn His Asn Asp
                   1270                 1275                 1280
   aaa act gta agt gaa  aaa aat aat aaa tgc  caa ctg ata tta caa       4113
   Lys Thr Val Ser Glu  Lys Asn Asn Lys Cys  Gln Leu Ile Leu Gln
                   1285                 1290                 1295
   aat aat att gaa atg  act act ggc act ttt  gtt gaa gaa att act       4158
   Asn Asn Ile Glu Met  Thr Thr Gly Thr Phe  Val Glu Glu Ile Thr
                   1300                 1305                 1310
   gaa aat tac aag aga  aat act gaa aat gaa  gat aac aaa tat act       4203
   Glu Asn Tyr Lys Arg  Asn Thr Glu Asn Glu  Asp Asn Lys Tyr Thr
                   1315                 1320                 1325
   gct gcc agt aga aat  tct cat aac tta gaa  ttt gat ggc agt gat       4248
   Ala Ala Ser Arg Asn  Ser His Asn Leu Glu  Phe Asp Gly Ser Asp
                   1330                 1335                 1340
   tca agt aaa aat gat  act gtt tgt att cat  aaa gat gaa acg gac       4293
   Ser Ser Lys Asn Asp  Thr Val Cys Ile His  Lys Asp Glu Thr Asp
                   1345                 1350                 1355
   ttg cta ttt act gat  cag cac aac ata tgt  ctt aaa tta tct ggc       4338
   Leu Leu Phe Thr Asp  Gln His Asn Ile Cys  Leu Lys Leu Ser Gly
                   1360                 1365                 1370
   cag ttt atg aag gag  gga aac act cag att  aaa gaa gat ttg tca       4383
   Gln Phe Met Lys Glu  Gly Asn Thr Gln Ile  Lys Glu Asp Leu Ser
                   1375                 1380                 1385
   gat tta act ttt ttg  gaa gtt gcg aaa gct  caa gaa gca tgt cat       4428
   Asp Leu Thr Phe Leu  Glu Val Ala Lys Ala  Gln Glu Ala Cys His
                   1390                 1395                 1400
   ggt aat act tca aat  aaa gaa cag tta act  gct act aaa acg gag       4473
   Gly Asn Thr Ser Asn  Lys Glu Gln Leu Thr  Ala Thr Lys Thr Glu
                   1405                 1410                 1415
   caa aat ata aaa gat  ttt gag act tct gat  aca ttt ttt cag act       4518
   Gln Asn Ile Lys Asp  Phe Glu Thr Ser Asp  Thr Phe Phe Gln Thr
                   1420                 1425                 1430
   gca agt ggg aaa aat  att agt gtc gcc aaa  gag tca ttt aat aaa       4563
   Ala Ser Gly Lys Asn  Ile Ser Val Ala Lys  Glu Ser Phe Asn Lys
                   1435                 1440                 1445
   att gta aat ttc ttt  gat cag aaa cca gaa  gaa ttg cat aac ttt       4608
   Ile Val Asn Phe Phe  Asp Gln Lys Pro Glu  Glu Leu His Asn Phe
                   1450                 1455                 1460
   tcc tta aat tct gaa  tta cat tct gac ata  aga aag aac aaa atg       4653
   Ser Leu Asn Ser Glu  Leu His Ser Asp Ile  Arg Lys Asn Lys Met
                   1465                 1470                 1475
   gac att cta agt tat  gag gaa aca gac ata  gtt aaa cac aaa ata       4698
   Asp Ile Leu Ser Tyr  Glu Glu Thr Asp Ile  Val Lys His Lys Ile
                   1480                 1485                 1490
   ctg aaa gaa agt gtc  cca gtt ggt act gga  aat caa cta gtg acc       4743
   Leu Lys Glu Ser Val  Pro Val Gly Thr Gly  Asn Gln Leu Val Thr
                   1495                 1500                 1505
   ttc cag gga caa ccc  gaa cgt gat gaa aag  atc aaa gaa cct act       4788
   Phe Gln Gly Gln Pro  Glu Arg Asp Glu Lys  Ile Lys Glu Pro Thr
                       1510                    1515                    1520
   ctg ttg ggt ttt cat aca gct agc ggg aaa aaa gtt aaa att gca        4833
   Leu Leu Gly Phe His Thr Ala Ser Gly Lys Lys Val Lys Ile Ala
                       1525                    1530                    1535
   aag gaa tct ttg gac aaa gtg aaa aac ctt ttt gat gaa aaa gag        4878
   Lys Glu Ser Leu Asp Lys Val Lys Asn Leu Phe Asp Glu Lys Glu
                       1540                    1545                    1550
   caa ggt act agt gaa atc acc agt ttt agc cat caa tgg gca aag        4923
   Gln Gly Thr Ser Glu Ile Thr Ser Phe Ser His Gln Trp Ala Lys
                       1555                    1560                    1565
   acc cta aag tac aga gag gcc tgt aaa gac ctt gaa tta gca tgt        4968
   Thr Leu Lys Tyr Arg Glu Ala Cys Lys Asp Leu Glu Leu Ala Cys
                       1570                    1575                    1580
   gag acc att gag atc aca gct gcc cca aag tgt aaa gaa atg cag        5013
   Glu Thr Ile Glu Ile Thr Ala Ala Pro Lys Cys Lys Glu Met Gln
                       1585                    1590                    1595
   aat tct ctc aat aat gat aaa aac ctt gtt tct att gag act gtg        5058
   Asn Ser Leu Asn Asn Asp Lys Asn Leu Val Ser Ile Glu Thr Val
                       1600                    1605                    1610
   gtg cca cct aag ctc tta agt gat aat tta tgt aga caa act gaa        5103
   Val Pro Pro Lys Leu Leu Ser Asp Asn Leu Cys Arg Gln Thr Glu
                       1615                    1620                    1625
   aat ctc aaa aca tca aaa agt atc ttt ttg aaa gtt aaa gta cat        5148
   Asn Leu Lys Thr Ser Lys Ser Ile Phe Leu Lys Val Lys Val His
                       1630                    1635                    1640
   gaa aat gta gaa aaa gaa aca gca aaa agt cct gca act tgt tac        5193
   Glu Asn Val Glu Lys Glu Thr Ala Lys Ser Pro Ala Thr Cys Tyr
                       1645                    1650                    1655
   aca aat cag tcc cct tat tca gtc att gaa aat tca gcc tta gct        5238
   Thr Asn Gln Ser Pro Tyr Ser Val Ile Glu Asn Ser Ala Leu Ala
                       1660                    1665                    1670
   ttt tac aca agt tgt agt aga aaa act tct gtg agt cag act tca        5283
   Phe Tyr Thr Ser Cys Ser Arg Lys Thr Ser Val Ser Gln Thr Ser
                       1675                    1680                    1685
   tta ctt gaa gca aaa aaa tgg ctt aga gaa gga ata ttt gat ggt        5328
   Leu Leu Glu Ala Lys Lys Trp Leu Arg Glu Gly Ile Phe Asp Gly
                       1690                    1695                    1700
   caa cca gaa aga ata aat act gca gat tat gta gga aat tat ttg        5373
   Gln Pro Glu Arg Ile Asn Thr Ala Asp Tyr Val Gly Asn Tyr Leu
                       1705                    1710                    1715
   tat gaa aat aat tca aac agt act ata gct gaa aat gac aaa aat        5418
   Tyr Glu Asn Asn Ser Asn Ser Thr Ile Ala Glu Asn Asp Lys Asn
                       1720                    1725                    1730
   cat ctc tcc gaa aaa caa gat act tat tta agt aac agt agc atg        5463
   His Leu Ser Glu Lys Gln Asp Thr Tyr Leu Ser Asn Ser Ser Met
                       1735                    1740                    1745
   tct aac agc tat tcc tac cat tct gat gag gta tat aat gat tca        5508
   Ser Asn Ser Tyr Ser Tyr His Ser Asp Glu Val Tyr Asn Asp Ser
                       1750                    1755                    1760
   gga tat ctc tca aaa aat aaa ctt gat tct ggt att gag cca gta        5553
   Gly Tyr Leu Ser Lys Asn Lys Leu Asp Ser Gly Ile Glu Pro Val
                       1765                    1770                    1775
   ttg aag aat gtt gaa  gat caa aaa aac act  agt ttt tcc aaa gta         5598
   Leu Lys Asn Val Glu  Asp Gln Lys Asn Thr  Ser Phe Ser Lys Val
                   1780                 1785                 1790
   ata tcc aat gta aaa  gat gca aat gca tac  cca caa act gta aat         5643
   Ile Ser Asn Val Lys  Asp Ala Asn Ala Tyr  Pro Gln Thr Val Asn
                   1795                 1800                 1805
   gaa gat att tgc gtt  gag gaa ctt gtg act  agc tct tca ccc tgc         5688
   Glu Asp Ile Cys Val  Glu Glu Leu Val Thr  Ser Ser Ser Pro Cys
                   1810                 1815                 1820
   aaa aat aaa aat gca  gcc att aaa ttg tcc  ata tct aat agt aat         5733
   Lys Asn Lys Asn Ala  Ala Ile Lys Leu Ser  Ile Ser Asn Ser Asn
                   1825                 1830                 1835
   aat ttt gag gta ggg  cca cct gca ttt agg  ata gcc agt ggt aaa         5778
   Asn Phe Glu Val Gly  Pro Pro Ala Phe Arg  Ile Ala Ser Gly Lys
                   1840                 1845                 1850
   atc gtt tgt gtt tca  cat gaa aca att aaa  aaa gtg aaa gac ata         5823
   Ile Val Cys Val Ser  His Glu Thr Ile Lys  Lys Val Lys Asp Ile
                   1855                 1860                 1865
   ttt aca gac agt ttc  agt aaa gta att aag  gaa aac aac gag aat         5868
   Phe Thr Asp Ser Phe  Ser Lys Val Ile Lys  Glu Asn Asn Glu Asn
                   1870                 1875                 1880
   aaa tca aaa att tgc  caa acg aaa att atg  gca ggt tgt tac gag         5913
   Lys Ser Lys Ile Cys  Gln Thr Lys Ile Met  Ala Gly Cys Tyr Glu
                   1885                 1890                 1895
   gca ttg gat gat tca  gag gat att ctt cat  aac tct cta gat aat         5958
   Ala Leu Asp Asp Ser  Glu Asp Ile Leu His  Asn Ser Leu Asp Asn
                   1900                 1905                 1910
   gat gaa tgt agc acg  cat tca cat aag gtt  ttt gct gac att cag         6003
   Asp Glu Cys Ser Thr  His Ser His Lys Val  Phe Ala Asp Ile Gln
                   1915                 1920                 1925
   agt gaa gaa att tta  caa cat aac caa aat  atg tct gga ttg gag         6048
   Ser Glu Glu Ile Leu  Gln His Asn Gln Asn  Met Ser Gly Leu Glu
                   1930                 1935                 1940
   aaa gtt tct aaa ata  tca cct tgt gat gtt  agt ttg gaa act tca         6093
   Lys Val Ser Lys Ile  Ser Pro Cys Asp Val  Ser Leu Glu Thr Ser
                   1945                 1950                 1955
   gat ata tgt aaa tgt  agt ata ggg aag ctt  cat aag tca gtc tca         6138
   Asp Ile Cys Lys Cys  Ser Ile Gly Lys Leu  His Lys Ser Val Ser
                   1960                 1965                 1970
   tct gca aat act tgt  ggg att ttt agc aca  gca agt gga aaa tct         6183
   Ser Ala Asn Thr Cys  Gly Ile Phe Ser Thr  Ala Ser Gly Lys Ser
                   1975                 1980                 1985
   gtc cag gta tca gat  gct tca tta caa aac  gca aga caa gtg ttt         6228
   Val Gln Val Ser Asp  Ala Ser Leu Gln Asn  Ala Arg Gln Val Phe
                   1990                 1995                 2000
   tct gaa ata gaa gat  agt acc aag caa gtc  ttt tcc aaa gta ttg         6273
   Ser Glu Ile Glu Asp  Ser Thr Lys Gln Val  Phe Ser Lys Val Leu
                   2005                 2010                 2015
   ttt aaa agt aac gaa  cat tca gac cag ctc  aca aga gaa gaa aat         6318
   Phe Lys Ser Asn Glu  His Ser Asp Gln Leu  Thr Arg Glu Glu Asn
                   2020                 2025                 2030
   act gct ata cgt act  cca gaa cat tta ata  tcc caa aaa ggc ttt        6363
   Thr Ala Ile Arg Thr  Pro Glu His Leu Ile  Ser Gln Lys Gly Phe
                   2035                 2040                 2045
   tca tat aat gtg gta  aat tca tct gct ttc  tct gga ttt agt aca        6408
   Ser Tyr Asn Val Val  Asn Ser Ser Ala Phe  Ser Gly Phe Ser Thr
                   2050                 2055                 2060
   gca agt gga aag caa  gtt tcc att tta gaa  agt tcc tta cac aaa        6453
   Ala Ser Gly Lys Gln  Val Ser Ile Leu Glu  Ser Ser Leu His Lys
                   2065                 2070                 2075
   gtt aag gga gtg tta  gag gaa ttt gat tta  atc aga act gag cat        6498
   Val Lys Gly Val Leu  Glu Glu Phe Asp Leu  Ile Arg Thr Glu His
                   2080                 2085                 2090
   agt ctt cac tat tca  cct acg tct aga caa  aat gta tca aaa ata        6543
   Ser Leu His Tyr Ser  Pro Thr Ser Arg Gln  Asn Val Ser Lys Ile
                   2095                 2100                 2105
   ctt cct cgt gtt gat  aag aga aac cca gag  cac tgt gta aac tca        6588
   Leu Pro Arg Val Asp  Lys Arg Asn Pro Glu  His Cys Val Asn Ser
                   2110                 2115                 2120
   gaa atg gaa aaa acc  tgc agt aaa gaa ttt  aaa tta tca aat aac        6633
   Glu Met Glu Lys Thr  Cys Ser Lys Glu Phe  Lys Leu Ser Asn Asn
                   2125                 2130                 2135
   tta aat gtt gaa ggt  ggt tct tca gaa aat  aat cac tct att aaa        6678
   Leu Asn Val Glu Gly  Gly Ser Ser Glu Asn  Asn His Ser Ile Lys
                   2140                 2145                 2150
   gtt tct cca tat ctc  tct caa ttt caa caa  gac aaa caa cag ttg        6723
   Val Ser Pro Tyr Leu  Ser Gln Phe Gln Gln  Asp Lys Gln Gln Leu
                   2155                 2160                 2165
   gta tta gga acc aaa  gtc tca ctt gtt gag  aac att cat gtt ttg        6768
   Val Leu Gly Thr Lys  Val Ser Leu Val Glu  Asn Ile His Val Leu
                   2170                 2175                 2180
   gga aaa gaa cag gct  tca cct aaa aac gta  aaa atg gaa att ggt        6813
   Gly Lys Glu Gln Ala  Ser Pro Lys Asn Val  Lys Met Glu Ile Gly
                   2185                 2190                 2195
   aaa act gaa act ttt  tct gat gtt cct gtg  aaa aca aat ata gaa        6858
   Lys Thr Glu Thr Phe  Ser Asp Val Pro Val  Lys Thr Asn Ile Glu
                   2200                 2205                 2210
   gtt tgt tct act tac  tcc aaa gat tca gaa  aac tac ttt gaa aca        6903
   Val Cys Ser Thr Tyr  Ser Lys Asp Ser Glu  Asn Tyr Phe Glu Thr
                   2215                 2220                 2225
   gaa gca gta gaa att  gct aaa gct ttt atg  gaa gat gat gaa ctg        6948
   Glu Ala Val Glu Ile  Ala Lys Ala Phe Met  Glu Asp Asp Glu Leu
                   2230                 2235                 2240
   aca gat tct aaa ctg  cca agt cat gcc aca  cat tct ctt ttt aca        6993
   Thr Asp Ser Lys Leu  Pro Ser His Ala Thr  His Ser Leu Phe Thr
                   2245                 2250                 2255
   tgt ccc gaa aat gag  gaa atg gtt ttg tca  aat tca aga att gga        7038
   Cys Pro Glu Asn Glu  Glu Met Val Leu Ser  Asn Ser Arg Ile Gly
                   2260                 2265                 2270
   aaa aga aga gga gag  ccc ctt atc tta gtg  gga gaa ccc tca atc        7083
   Lys Arg Arg Gly Glu  Pro Leu Ile Leu Val  Gly Glu Pro Ser Ile
                   2275                 2280                 2285
   aaa aga aac tta tta  aat gaa ttt gac agg  ata ata gaa aat caa        7128
   Lys Arg Asn Leu Leu  Asn Glu Phe Asp Arg  Ile Ile Glu Asn Gln
                   2290                 2295                 2300
   gaa aaa tcc tta aag  gct tca aaa agc act  cca gat ggc aca ata      7173
   Glu Lys Ser Leu Lys  Ala Ser Lys Ser Thr  Pro Asp Gly Thr Ile
                   2305                 2310                 2315
   aaa gat cga aga ttg  ttt atg cat cat gtt  tct tta gag ccg att      7218
   Lys Asp Arg Arg Leu  Phe Met His His Val  Ser Leu Glu Pro Ile
                   2320                 2325                 2330
   acc tgt gta ccc ttt  cgc aca act aag gaa  cgt caa gag ata cag      7263
   Thr Cys Val Pro Phe  Arg Thr Thr Lys Glu  Arg Gln Glu Ile Gln
                   2335                 2340                 2345
   aat cca aat ttt acc  gca cct ggt caa gaa  ttt ctg tct aaa tct      7308
   Asn Pro Asn Phe Thr  Ala Pro Gly Gln Glu  Phe Leu Ser Lys Ser
                   2350                 2355                 2360
   cat ttg tat gaa cat  ctg act ttg gaa aaa  tct tca agc aat tta      7353
   His Leu Tyr Glu His  Leu Thr Leu Glu Lys  Ser Ser Ser Asn Leu
                   2365                 2370                 2375
   gca gtt tca gga cat  cca ttt tat caa gtt  tct gct aca aga aat      7398
   Ala Val Ser Gly His  Pro Phe Tyr Gln Val  Ser Ala Thr Arg Asn
                   2380                 2385                 2390
   gaa aaa atg aga cac  ttg att act aca ggc  aga cca acc aaa gtc      7443
   Glu Lys Met Arg His  Leu Ile Thr Thr Gly  Arg Pro Thr Lys Val
                   2395                 2400                 2405
   ttt gtt cca cct ttt  aaa act aaa tcg cat  ttt cac aga gtt gaa      7488
   Phe Val Pro Pro Phe  Lys Thr Lys Ser His  Phe His Arg Val Glu
                   2410                 2415                 2420
   cag tgt gtt agg aat  att aac ttg gag gaa  aac aga caa aag caa      7533
   Gln Cys Val Arg Asn  Ile Asn Leu Glu Glu  Asn Arg Gln Lys Gln
                   2425                 2430                 2435
   aac att gat gga cat  ggc tct gat gat agt  aaa aat aag att aat      7578
   Asn Ile Asp Gly His  Gly Ser Asp Asp Ser  Lys Asn Lys Ile Asn
                   2440                 2445                 2450
   gac aat gag att cat  cag ttt aac aaa aac  aac tcc aat caa gca      7623
   Asp Asn Glu Ile His  Gln Phe Asn Lys Asn  Asn Ser Asn Gln Ala
                   2455                 2460                 2465
   gca gct gta act ttc  aca aag tgt gaa gaa  gaa cct tta gat tta      7668
   Ala Ala Val Thr Phe  Thr Lys Cys Glu Glu  Glu Pro Leu Asp Leu
                   2470                 2475                 2480
   att aca agt ctt cag  aat gcc aga gat ata  cag gat atg cga att      7713
   Ile Thr Ser Leu Gln  Asn Ala Arg Asp Ile  Gln Asp Met Arg Ile
                   2485                 2490                 2495
   aag aag aaa caa agg  caa cgc gtc ttt cca  cag cca ggc agt ctg      7758
   Lys Lys Lys Gln Arg  Gln Arg Val Phe Pro  Gln Pro Gly Ser Leu
                   2500                 2505                 2510
   tat ctt gca aaa aca  tcc act ctg cct cga  atc tct ctg aaa gca      7803
   Tyr Leu Ala Lys Thr  Ser Thr Leu Pro Arg  Ile Ser Leu Lys Ala
                   2515                 2520                 2525
   gca gta gga ggc caa  gtt ccc tct gcg tgt  tct cat aaa cag ctg      7848
   Ala Val Gly Gly Gln  Val Pro Ser Ala Cys  Ser His Lys Gln Leu
                   2530                 2535                 2540
   tat acg tat ggc gtt  tct aaa cat tgc ata  aaa att aac agc aaa      7893
   Tyr Thr Tyr Gly Val  Ser Lys His Cys Ile  Lys Ile Asn Ser Lys
                       2545                    2550                    2555
   aat gca gag tct ttt  cag ttt cac act gaa  gat tat ttt ggt aag      7938
   Asn Ala Glu Ser Phe  Gln Phe His Thr Glu  Asp Tyr Phe Gly Lys
                   2560                 2565                 2570
   gaa agt tta tgg act  gga aaa gga ata cag  ttg gct gat ggt gga      7983
   Glu Ser Leu Trp Thr  Gly Lys Gly Ile Gln  Leu Ala Asp Gly Gly
                   2575                 2580                 2585
   tgg ctc ata ccc tcc  aat gat gga aag gct  gga aaa gaa gaa ttt      8028
   Trp Leu Ile Pro Ser  Asn Asp Gly Lys Ala  Gly Lys Glu Glu Phe
                   2590                 2595                 2600
   tat agg gct ctg tgt  gac act cca ggt gtg  gat cca aag ctt att      8073
   Tyr Arg Ala Leu Cys  Asp Thr Pro Gly Val  Asp Pro Lys Leu Ile
                   2605                 2610                 2615
   tct aga att tgg gtt  tat aat cac tat aga  tgg atc ata tgg aaa      8118
   Ser Arg Ile Trp Val  Tyr Asn His Tyr Arg  Trp Ile Ile Trp Lys
                   2620                 2625                 2630
   ctg gca gct atg gaa  tgt gcc ttt cct aag  gaa ttt gct aat aga      8163
   Leu Ala Ala Met Glu  Cys Ala Phe Pro Lys  Glu Phe Ala Asn Arg
                   2635                 2640                 2645
   tgc cta agc cca gaa  agg gtg ctt ctt caa  cta aaa tac aga tat      8208
   Cys Leu Ser Pro Glu  Arg Val Leu Leu Gln  Leu Lys Tyr Arg Tyr
                   2650                 2655                 2660
   gat acg gaa att gat  aga agc aga aga tcg  gct ata aaa aag ata      8253
   Asp Thr Glu Ile Asp  Arg Ser Arg Arg Ser  Ala Ile Lys Lys Ile
                   2665                 2670                 2675
   atg gaa agg gat gac  aca gct gca aaa aca  ctt gtt ctc tgt gtt      8298
   Met Glu Arg Asp Asp  Thr Ala Ala Lys Thr  Leu Val Leu Cys Val
                   2680                 2685                 2690
   tct gac ata att tca  ttg agc gca aat ata  tct gaa act tct agc      8343
   Ser Asp Ile Ile Ser  Leu Ser Ala Asn Ile  Ser Glu Thr Ser Ser
                   2695                 2700                 2705
   aat aaa act agt agt  gca gat acc caa aaa  gtg gcc att att gaa      8388
   Asn Lys Thr Ser Ser  Ala Asp Thr Gln Lys  Val Ala Ile Ile Glu
                   2710                 2715                 2720
   ctt aca gat ggg tgg  tat gct gtt aag gcc  cag tta gat cct ccc      8433
   Leu Thr Asp Gly Trp  Tyr Ala Val Lys Ala  Gln Leu Asp Pro Pro
                   2725                 2730                 2735
   ctc tta gct gtc tta  aag aat ggc aga ctg  aca gtt ggt cag aag      8478
   Leu Leu Ala Val Leu  Lys Asn Gly Arg Leu  Thr Val Gly Gln Lys
                   2740                 2745                 2750
   att att ctt cat gga  gca gaa ctg gtg ggc  tct cct gat gcc tgt      8523
   Ile Ile Leu His Gly  Ala Glu Leu Val Gly  Ser Pro Asp Ala Cys
                   2755                 2760                 2765
   aca cct ctt gaa gcc  cca gaa tct ctt atg  tta aag att tct gct      8568
   Thr Pro Leu Glu Ala  Pro Glu Ser Leu Met  Leu Lys Ile Ser Ala
                   2770                 2775                 2780
   aac agt act cgg cct  gct cgc tgg tat acc  aaa ctt gga ttc ttt      8613
   Asn Ser Thr Arg Pro  Ala Arg Trp Tyr Thr  Lys Leu Gly Phe Phe
                   2785                 2790                 2795
   cct gac cct aga cct  ttt cct ctg ccc tta  tca tcg ctt ttc agt      8658
   Pro Asp Pro Arg Pro  Phe Pro Leu Pro Leu  Ser Ser Leu Phe Ser
                   2800                 2805                 2810
   gat gga gga aat gtt  ggt tgt gtt gat gta  att att caa aga gca        8703
   Asp Gly Gly Asn Val  Gly Cys Val Asp Val  Ile Ile Gln Arg Ala
                   2815                 2820                 2825
   tac cct ata cag tgg  atg gag aag aca tca  tct gga tta tac ata        8748
   Tyr Pro Ile Gln Trp  Met Glu Lys Thr Ser  Ser Gly Leu Tyr Ile
                   2830                 2835                 2840
   ttt cgc aat gaa aga  gag gaa gaa aag gaa  gca gca aaa tat gtg        8793
   Phe Arg Asn Glu Arg  Glu Glu Glu Lys Glu  Ala Ala Lys Tyr Val
                   2845                 2850                 2855
   gag gcc caa caa aag  aga cta gaa gcc tta  ttc act aaa att cag        8838
   Glu Ala Gln Gln Lys  Arg Leu Glu Ala Leu  Phe Thr Lys Ile Gln
                   2860                 2865                 2870
   gag gaa ttt gaa gaa  cat gaa gaa aac aca  aca aaa cca tat tta        8883
   Glu Glu Phe Glu Glu  His Glu Glu Asn Thr  Thr Lys Pro Tyr Leu
                   2875                 2880                 2885
   cca tca cgt gca cta  aca aga cag caa gtt  cgt gct ttg caa gat        8928
   Pro Ser Arg Ala Leu  Thr Arg Gln Gln Val  Arg Ala Leu Gln Asp
                   2890                 2895                 2900
   ggt gca gag ctt tat  gaa gca gtg aag aat  gca gca gac cca gct        8973
   Gly Ala Glu Leu Tyr  Glu Ala Val Lys Asn  Ala Ala Asp Pro Ala
                   2905                 2910                 2915
   tac ctt gag ggt tat  ttc agt gaa gag cag  tta aga gcc ttg aat        9018
   Tyr Leu Glu Gly Tyr  Phe Ser Glu Glu Gln  Leu Arg Ala Leu Asn
                   2920                 2925                 2930
   aat cac agg caa atg  ttg aat gat aag aaa  caa gct cag atc cag        9063
   Asn His Arg Gln Met  Leu Asn Asp Lys Lys  Gln Ala Gln Ile Gln
                   2935                 2940                 2945
   ttg gaa att agg aag  gcc atg gaa tct gct  gaa caa aag gaa caa        9108
   Leu Glu Ile Arg Lys  Ala Met Glu Ser Ala  Glu Gln Lys Glu Gln
                   2950                 2955                 2960
   ggt tta tca agg gat  gtc aca acc gtg tgg  aag ttg cgt att gta        9153
   Gly Leu Ser Arg Asp  Val Thr Thr Val Trp  Lys Leu Arg Ile Val
                   2965                 2970                 2975
   agc tat tca aaa aaa  gaa aaa gat tca gtt  ata ctg agt att tgg        9198
   Ser Tyr Ser Lys Lys  Glu Lys Asp Ser Val  Ile Leu Ser Ile Trp
                   2980                 2985                 2990
   cgt cca tca tca gat  tta tat tct ctg tta  aca gaa gga aag aga        9243
   Arg Pro Ser Ser Asp  Leu Tyr Ser Leu Leu  Thr Glu Gly Lys Arg
                   2995                 3000                 3005
   tac aga att tat cat  ctt gca act tca aaa  tct aaa agt aaa tct        9288
   Tyr Arg Ile Tyr His  Leu Ala Thr Ser Lys  Ser Lys Ser Lys Ser
                   3010                 3015                 3020
   gaa aga gct aac ata  cag tta gca gcg aca  aaa aaa act cag tat        9333
   Glu Arg Ala Asn Ile  Gln Leu Ala Ala Thr  Lys Lys Thr Gln Tyr
                   3025                 3030                 3035
   caa caa cta ccg gtt  tca gat gaa att tta  ttt cag att tac cag        9378
   Gln Gln Leu Pro Val  Ser Asp Glu Ile Leu  Phe Gln Ile Tyr Gln
                   3040                 3045                 3050
   cca cgg gag ccc ctt  cac ttc agc aaa ttt  tta gat cca gac ttt        9423
   Pro Arg Glu Pro Leu  His Phe Ser Lys Phe  Leu Asp Pro Asp Phe
                   3055                 3060                 3065
   cag cca tct tgt tct  gag gtg gac cta ata  gga ttt gtc gtt tct         9468
   Gln Pro Ser Cys Ser  Glu Val Asp Leu Ile  Gly Phe Val Val Ser
                   3070                 3075                 3080
   gtt gtg aaa aaa aca  gga ctt gcc cct ttc  gtc tat ttg tca gac         9513
   Val Val Lys Lys Thr  Gly Leu Ala Pro Phe  Val Tyr Leu Ser Asp
                   3085                 3090                 3095
   gaa tgt tac aat tta  ctg gca ata aag ttt  tgg ata gac ctt aat         9558
   Glu Cys Tyr Asn Leu  Leu Ala Ile Lys Phe  Trp Ile Asp Leu Asn
                   3100                 3105                 3110
   gag gac att att aag  cct cat atg tta att  gct gca agc aac ctc         9603
   Glu Asp Ile Ile Lys  Pro His Met Leu Ile  Ala Ala Ser Asn Leu
                   3115                 3120                 3125
   cag tgg cga cca gaa  tcc aaa tca ggc ctt  ctt act tta ttt gct         9648
   Gln Trp Arg Pro Glu  Ser Lys Ser Gly Leu  Leu Thr Leu Phe Ala
                   3130                 3135                 3140
   gga gat ttt tct gtg  ttt tct gct agt cca  aaa gag ggc cac ttt         9693
   Gly Asp Phe Ser Val  Phe Ser Ala Ser Pro  Lys Glu Gly His Phe
                   3145                 3150                 3155
   caa gag aca ttc aac  aaa atg aaa aat act  gtt gag aat att gac         9738
   Gln Glu Thr Phe Asn  Lys Met Lys Asn Thr  Val Glu Asn Ile Asp
                   3160                 3165                 3170
   ata ctt tgc aat gaa  gca gaa aac aag ctt  atg cat ata ctg cat         9783
   Ile Leu Cys Asn Glu  Ala Glu Asn Lys Leu  Met His Ile Leu His
                   3175                 3180                 3185
   gca aat gat ccc aag  tgg tcc acc cca act  aaa gac tgt act tca         9828
   Ala Asn Asp Pro Lys  Trp Ser Thr Pro Thr  Lys Asp Cys Thr Ser
                   3190                 3195                 3200
   ggg ccg tac act gct  caa atc att cct ggt  aca gga aac aag ctt         9873
   Gly Pro Tyr Thr Ala  Gln Ile Ile Pro Gly  Thr Gly Asn Lys Leu
                   3205                 3210                 3215
   ctg atg tct tct cct  aat tgt gag ata tat  tat caa agt cct tta         9918
   Leu Met Ser Ser Pro  Asn Cys Glu Ile Tyr  Tyr Gln Ser Pro Leu
                   3220                 3225                 3230
   tca ctt tgt atg gcc  aaa agg aag tct gtt  tcc aca cct gtc tca         9963
   Ser Leu Cys Met Ala  Lys Arg Lys Ser Val  Ser Thr Pro Val Ser
                   3235                 3240                 3245
   gcc cag atg act tca  aag tct tgt aaa ggg  gag aaa gag att gat        10008
   Ala Gln Met Thr Ser  Lys Ser Cys Lys Gly  Glu Lys Glu Ile Asp
                   3250                 3255                 3260
   gac caa aag aac tgc  aaa aag aga aga gcc  ttg gat ttc ttg agt        10053
   Asp Gln Lys Asn Cys  Lys Lys Arg Arg Ala  Leu Asp Phe Leu Ser
                   3265                 3270                 3275
   aga ctg cct tta cct  cca cct gtt agt ccc  att tgt aca ttt gtt        10098
   Arg Leu Pro Leu Pro  Pro Pro Val Ser Pro  Ile Cys Thr Phe Val
                   3280                 3285                 3290
   tct ccg gct gca cag  aag gca ttt cag cca  cca agg agt tgt ggc        10143
   Ser Pro Ala Ala Gln  Lys Ala Phe Gln Pro  Pro Arg Ser Cys Gly
                   3295                 3300                 3305
   acc aaa tac gaa aca  ccc ata aag aaa aaa  gaa ctg aat tct cct        10188
   Thr Lys Tyr Glu Thr  Pro Ile Lys Lys Lys  Glu Leu Asn Ser Pro
                   3310                 3315                 3320
   cag atg act cca ttt  aaa aaa ttc aat gaa  att tct ctt ttg gaa        10233
   Gln Met Thr Pro Phe  Lys Lys Phe Asn Glu  Ile Ser Leu Leu Glu
                   3325                 3330                 3335
   agt aat tca ata gct  gac gaa gaa ctt gca  ttg ata aat acc caa     10278
   Ser Asn Ser Ile Ala  Asp Glu Glu Leu Ala  Leu Ile Asn Thr Gln
                   3340                 3345                 3350
   gct ctt ttg tct ggt  tca aca gga gaa aaa  caa ttt ata tct gtc     10323
   Ala Leu Leu Ser Gly  Ser Thr Gly Glu Lys  Gln Phe Ile Ser Val
                   3355                 3360                 3365
   agt gaa tcc act agg  act gct ccc acc agt  tca gaa gat tat ctc     10368
   Ser Glu Ser Thr Arg  Thr Ala Pro Thr Ser  Ser Glu Asp Tyr Leu
                   3370                 3375                 3380
   aga ctg aaa cga cgt  tgt act aca tct ctg  atc aaa gaa cag gag     10413
   Arg Leu Lys Arg Arg  Cys Thr Thr Ser Leu  Ile Lys Glu Gln Glu
                   3385                 3390                 3395
   agt tcc cag gcc agt  acg gaa gaa tgt gag  aaa aat aag cag gac     10458
   Ser Ser Gln Ala Ser  Thr Glu Glu Cys Glu  Lys Asn Lys Gln Asp
                   3400                 3405                 3410
   aca att aca act aaa  aaa tat atc taa                              10485
   Thr Ile Thr Thr Lys  Lys Tyr Ile
                   3415
<210> 9
   <211> 3418
   <212> PRT
   <213> Homo sapiens
<400> 9
   Met Pro Ile Gly Ser Lys Glu Arg Pro Thr Phe Phe Glu Ile Phe Lys
   1               5                   10                  15
   Thr Arg Cys Asn Lys Ala Asp Leu Gly Pro Ile Ser Leu Asn Trp Phe
               20                  25                  30
   Glu Glu Leu Ser Ser Glu Ala Pro Pro Tyr Asn Ser Glu Pro Ala Glu
           35                  40                  45
   Glu Ser Glu His Lys Asn Asn Asn Tyr Glu Pro Asn Leu Phe Lys Thr
       50                  55                  60
   Pro Gln Arg Lys Pro Ser Tyr Asn Gln Leu Ala Ser Thr Pro Ile Ile
   65                  70                  75                  80
   Phe Lys Glu Gln Gly Leu Thr Leu Pro Leu Tyr Gln Ser Pro Val Lys
                   85                  90                  95
   Glu Leu Asp Lys Phe Lys Leu Asp Leu Gly Arg Asn Val Pro Asn Ser
               100                 105                 110
   Arg His Lys Ser Leu Arg Thr Val Lys Thr Lys Met Asp Gln Ala Asp
           115                 120                 125
   Asp Val Ser Cys Pro Leu Leu Asn Ser Cys Leu Ser Glu Ser Pro Val
       130                 135                 140
   Val Leu Gln Cys Thr His Val Thr Pro Gln Arg Asp Lys Ser Val Val
   145                 150                 155                 160
   Cys Gly Ser Leu Phe His Thr Pro Lys Phe Val Lys Gly Arg Gln Thr
                   165                 170                 175
   Pro Lys His Ile Ser Glu Ser Leu Gly Ala Glu Val Asp Pro Asp Met
               180                 185                 190
   Ser Trp Ser Ser Ser Leu Ala Thr Pro Pro Thr Leu Ser Ser Thr Val
           195                 200                 205
   Leu Ile Val Arg Asn Glu Glu Ala Ser Glu Thr Val Phe Pro His Asp
       210                 215                 220
   Thr Thr Ala Asn Val Lys Ser Tyr Phe Ser Asn His Asp Glu Ser Leu
   225                 230                 235                 240
   Lys Lys Asn Asp Arg Phe Ile Ala Ser Val Thr Asp Ser Glu Asn Thr
                   245                 250                 255
   Asn Gln Arg Glu Ala Ala Ser His Gly Phe Gly Lys Thr Ser Gly Asn
               260                 265                 270
   Ser Phe Lys Val Asn Ser Cys Lys Asp His Ile Gly Lys Ser Met Pro
           275                 280                 285
   His Val Leu Glu Asp Glu Val Tyr Glu Thr Val Val Asp Thr Ser Glu
       290                 295                 300
   Glu Asp Ser Phe Ser Leu Cys Phe Ser Lys Cys Arg Thr Lys Asn Leu
   305                 310                 315                 320
   Gln Lys Val Arg Thr Ser Lys Thr Arg Lys Lys Ile Phe His Glu Ala
                   325                 330                 335
   Asn Ala Asp Glu Cys Glu Lys Ser Lys Asn Gln Val Lys Glu Lys Tyr
               340                 345                 350
   Ser Phe Val Ser Glu Val Glu Pro Asn Asp Thr Asp Pro Leu Asp Ser
           355                 360                 365
   Asn Val Ala Asn Gln Lys Pro Phe Glu Ser Gly Ser Asp Lys Ile Ser
       370                 375                 380
   Lys Glu Val Val Pro Ser Leu Ala Cys Glu Trp Ser Gln Leu Thr Leu
   385                 390                 395                 400
   Ser Gly Leu Asn Gly Ala Gln Met Glu Lys Ile Pro Leu Leu His Ile
                   405                 410                 415
   Ser Ser Cys Asp Gln Asn Ile Ser Glu Lys Asp Leu Leu Asp Thr Glu
   420 425 430
   Asn Lys Arg Lys Lys Asp Phe Leu Thr Ser Glu Asn Ser Leu Pro Arg
   435 440 445
   Ile Ser Ser Leu Pro Lys Ser Glu Lys Pro Leu Asn Glu Glu Thr Val
   450 455 460
   Val Asn Lys Arg Asp Glu Glu Gln His Leu Glu Ser His Thr Asp Cys
   465 470 475 480
   Ile Leu Ala Val Lys Gln Ala Ile Ser Gly Thr Ser Pro Val Ala Ser
   485 490 495
   Ser Phe Gln Gly Ile Lys Lys Ser Ile Phe Arg Ile Arg Glu Ser Pro
   500 505 510
   Lys Glu Thr Phe Asn Ala Ser Phe Ser Gly His Met Thr Asp Pro Asn
   515 520 525
   Phe Lys Lys Glu Thr Glu Ala Ser Glu Ser Gly Leu Glu Ile His Thr
   530 535 540
   Val Cys Ser Gln Lys Glu Asp Ser Leu Cys Pro Asn Leu Ile Asp Asn
   545 550 555 560
   Gly Ser Trp Pro Ala Thr Thr Thr Gln Asn Ser Val Ala Leu Lys Asn
   565 570 575
   Ala Gly Leu Ile Ser Thr Leu Lys Lys Lys Thr Asn Lys Phe Ile Tyr
   580 585 590
   Ala Ile His Asp Glu Thr Ser Tyr Lys Gly Lys Lys Ile Pro Lys Asp
   595 600 605
   Gln Lys Ser Glu Leu Ile Asn Cys Ser Ala Gln Phe Glu Ala Asn Ala
   610 615 620
   Phe Glu Ala Pro Leu Thr Phe Ala Asn Ala Asp Ser Gly Leu Leu His
   625 630 635 640
   Ser Ser Val Lys Arg Ser Cys Ser Gln Asn Asp Ser Glu Glu Pro Thr
   645 650 655
   Leu Ser Leu Thr Ser Ser Phe Gly Thr Ile Leu Arg Lys Cys Ser Arg
   660 665 670
   Asn Glu Thr Cys Ser Asn Asn Thr Val Ile Ser Gln Asp Leu Asp Tyr
   675 680 685
   Lys Glu Ala Lys Cys Asn Lys Glu Lys Leu Gln Leu Phe Ile Thr Pro
       690                 695                 700
   Glu Ala Asp Ser Leu Ser Cys Leu Gln Glu Gly Gln Cys Glu Asn Asp
   705                 710                 715                 720
   Pro Lys Ser Lys Lys Val Ser Asp Ile Lys Glu Glu Val Leu Ala Ala
                   725                 730                 735
   Ala Cys His Pro Val Gln His Ser Lys Val Glu Tyr Ser Asp Thr Asp
               740                 745                 750
   Phe Gln Ser Gln Lys Ser Leu Leu Tyr Asp His Glu Asn Ala Ser Thr
           755                 760                 765
   Leu Ile Leu Thr Pro Thr Ser Lys Asp Val Leu Ser Asn Leu Val Met
       770                 775                 780
   Ile Ser Arg Gly Lys Glu Ser Tyr Lys Met Ser Asp Lys Leu Lys Gly
   785                 790                 795                 800
   Asn Asn Tyr Glu Ser Asp Val Glu Leu Thr Lys Asn Ile Pro Met Glu
                   805                 810                 815
   Lys Asn Gln Asp Val Cys Ala Leu Asn Glu Asn Tyr Lys Asn Val Glu
               820                 825                 830
   Leu Leu Pro Pro Glu Lys Tyr Met Arg Val Ala Ser Pro Ser Arg Lys
           835                 840                 845
   Val Gln Phe Asn Gln Asn Thr Asn Leu Arg Val Ile Gln Lys Asn Gln
       850                 855                 860
   Glu Glu Thr Thr Ser Ile Ser Lys Ile Thr Val Asn Pro Asp Ser Glu
   865                 870                 875                 880
   Glu Leu Phe Ser Asp Asn Glu Asn Asn Phe Val Phe Gln Val Ala Asn
                   885                 890                 895
   Glu Arg Asn Asn Leu Ala Leu Gly Asn Thr Lys Glu Leu His Glu Thr
               900                 905                 910
   Asp Leu Thr Cys Val Asn Glu Pro Ile Phe Lys Asn Ser Thr Met Val
           915                 920                 925
   Leu Tyr Gly Asp Thr Gly Asp Lys Gln Ala Thr Gln Val Ser Ile Lys
       930                 935                 940
   Lys Asp Leu Val Tyr Val Leu Ala Glu Glu Asn Lys Asn Ser Val Lys
   945                 950                 955                 960
   Gln His Ile Lys Met Thr Leu Gly Gln Asp Leu Lys Ser Asp Ile Ser
   965 970 975
   Leu Asn Ile Asp Lys Ile Pro Glu Lys Asn Asn Asp Tyr Met Asp Lys
   980 985 990
   Trp Ala Gly Leu Leu Gly Pro Ile Ser Asn His Ser Phe Gly Gly Ser
   995 1000 1005
   Phe Arg Thr Ala Ser Asn Lys Glu Ile Lys Leu Ser Glu His Asn
   1010 1015 1020
   Ile Lys Lys Ser Lys Met Phe Phe Lys Asp Ile Glu Glu Gln Tyr
   1025 1030 1035
   Pro Thr Ser Leu Ala Cys Val Glu Ile Val Asn Thr Leu Ala Leu
   1040 1045 1050
   Asp Asn Gln Lys Lys Leu Ser Lys Pro Gln Ser Ile Asn Thr Val
   1055 1060 1065
   Ser Ala His Leu Gln Ser Ser Val Val Val Ser Asp Cys Lys Asn
   1070 1075 1080
   Ser His Ile Thr Pro Gln Met Leu Phe Ser Lys Gln Asp Phe Asn
   1085 1090 1095
   Ser Asn His Asn Leu Thr Pro Ser Gln Lys Ala Glu Ile Thr Glu
   1100 1105 1110
   Leu Ser Thr Ile Leu Glu Glu Ser Gly Ser Gln Phe Glu Phe Thr
   1115 1120 1125
   Gln Phe Arg Lys Pro Ser Tyr Ile Leu Gln Lys Ser Thr Phe Glu
   1130 1135 1140
   Val Pro Glu Asn Gln Met Thr Ile Leu Lys Thr Thr Ser Glu Glu
   1145 1150 1155
   Cys Arg Asp Ala Asp Leu His Val Ile Met Asn Ala Pro Ser Ile
   1160 1165 1170
   Gly Gln Val Asp Ser Ser Lys Gln Phe Glu Gly Thr Val Glu Ile
   1175 1180 1185
   Lys Arg Lys Phe Ala Gly Leu Leu Lys Asn Asp Cys Asn Lys Ser
   1190 1195 1200
   Ala Ser Gly Tyr Leu Thr Asp Glu Asn Glu Val Gly Phe Arg Gly
   1205 1210 1215
   Phe Tyr Ser Ala His Gly Thr Lys Leu Asn Val Ser Thr Glu Ala
   1220 1225 1230
   Leu Gln Lys Ala Val Lys Leu Phe Ser Asp Ile Glu Asn Ile Ser
       1235                1240                1245
   Glu Glu Thr Ser Ala Glu Val His Pro Ile Ser Leu Ser Ser Ser
       1250                1255                1260
   Lys Cys His Asp Ser Val Val Ser Met Phe Lys Ile Glu Asn His
       1265                1270                1275
   Asn Asp Lys Thr Val Ser Glu Lys Asn Asn Lys Cys Gln Leu Ile
       1280                1285                1290
   Leu Gln Asn Asn Ile Glu Met Thr Thr Gly Thr Phe Val Glu Glu
       1295                1300                1305
   Ile Thr Glu Asn Tyr Lys Arg Asn Thr Glu Asn Glu Asp Asn Lys
       1310                1315                1320
   Tyr Thr Ala Ala Ser Arg Asn Ser His Asn Leu Glu Phe Asp Gly
       1325                1330                1335
   Ser Asp Ser Ser Lys Asn Asp Thr Val Cys Ile His Lys Asp Glu
       1340                1345                1350
   Thr Asp Leu Leu Phe Thr Asp Gln His Asn Ile Cys Leu Lys Leu
       1355                1360                1365
   Ser Gly Gln Phe Met Lys Glu Gly Asn Thr Gln Ile Lys Glu Asp
       1370                1375                1380
   Leu Ser Asp Leu Thr Phe Leu Glu Val Ala Lys Ala Gln Glu Ala
       1385                1390                1395
   Cys His Gly Asn Thr Ser Asn Lys Glu Gln Leu Thr Ala Thr Lys
       1400                1405                1410
   Thr Glu Gln Asn Ile Lys Asp Phe Glu Thr Ser Asp Thr Phe Phe
       1415                1420                1425
   Gln Thr Ala Ser Gly Lys Asn Ile Ser Val Ala Lys Glu Ser Phe
       1430                1435                1440
   Asn Lys Ile Val Asn Phe Phe Asp Gln Lys Pro Glu Glu Leu His
       1445                1450                1455
   Asn Phe Ser Leu Asn Ser Glu Leu His Ser Asp Ile Arg Lys Asn
       1460                1465                1470
   Lys Met Asp Ile Leu Ser Tyr Glu Glu Thr Asp Ile Val Lys His
       1475                1480                1485
   Lys Ile Leu Lys Glu Ser Val Pro Val Gly Thr Gly Asn Gln Leu
   1490 1495 1500
   Val Thr Phe Gln Gly Gln Pro Glu Arg Asp Glu Lys Ile Lys Glu
   1505 1510 1515
   Pro Thr Leu Leu Gly Phe His Thr Ala Ser Gly Lys Lys Val Lys
   1520 1525 1530
   Ile Ala Lys Glu Ser Leu Asp Lys Val Lys Asn Leu Phe Asp Glu
   1535 1540 1545
   Lys Glu Gln Gly Thr Ser Glu Ile Thr Ser Phe Ser His Gln Trp
   1550 1555 1560
   Ala Lys Thr Leu Lys Tyr Arg Glu Ala Cys Lys Asp Leu Glu Leu
   1565 1570 1575
   Ala Cys Glu Thr Ile Glu Ile Thr Ala Ala Pro Lys Cys Lys Glu
   1580 1585 1590
   Met Gln Asn Ser Leu Asn Asn Asp Lys Asn Leu Val Ser Ile Glu
   1595 1600 1605
   Thr Val Val Pro Pro Lys Leu Leu Ser Asp Asn Leu Cys Arg Gln
   1610 1615 1620
   Thr Glu Asn Leu Lys Thr Ser Lys Ser Ile Phe Leu Lys Val Lys
   1625 1630 1635
   Val His Glu Asn Val Glu Lys Glu Thr Ala Lys Ser Pro Ala Thr
   1640 1645 1650
   Cys Tyr Thr Asn Gln Ser Pro Tyr Ser Val Ile Glu Asn Ser Ala
   1655 1660 1665
   Leu Ala Phe Tyr Thr Ser Cys Ser Arg Lys Thr Ser Val Ser Gln
   1670 1675 1680
   Thr Ser Leu Leu Glu Ala Lys Lys Trp Leu Arg Glu Gly Ile Phe
   1685 1690 1695
   Asp Gly Gln Pro Glu Arg Ile Asn Thr Ala Asp Tyr Val Gly Asn
   1700 1705 1710
   Tyr Leu Tyr Glu Asn Asn Ser Asn Ser Thr Ile Ala Glu Asn Asp
   1715 1720 1725
   Lys Asn His Leu Ser Glu Lys Gln Asp Thr Tyr Leu Ser Asn Ser
   1730 1735 1740
   Ser Met Ser Asn Ser Tyr Ser Tyr His Ser Asp Glu Val Tyr Asn
   1745 1750 1755
   Asp Ser Gly Tyr Leu Ser Lys Asn Lys Leu Asp Ser Gly Ile Glu
   1760 1765 1770
   Pro Val Leu Lys Asn Val Glu Asp Gln Lys Asn Thr Ser Phe Ser
   1775 1780 1785
   Lys Val Ile Ser Asn Val Lys Asp Ala Asn Ala Tyr Pro Gln Thr
   1790 1795 1800
   Val Asn Glu Asp Ile Cys Val Glu Glu Leu Val Thr Ser Ser Ser
   1805 1810 1815
   Pro Cys Lys Asn Lys Asn Ala Ala Ile Lys Leu Ser Ile Ser Asn
   1820 1825 1830
   Ser Asn Asn Phe Glu Val Gly Pro Pro Ala Phe Arg Ile Ala Ser
   1835 1840 1845
   Gly Lys Ile Val Cys Val Ser His Glu Thr Ile Lys Lys Val Lys
   1850 1855 1860
   Asp Ile Phe Thr Asp Ser Phe Ser Lys Val Ile Lys Glu Asn Asn
   1865 1870 1875
   Glu Asn Lys Ser Lys Ile Cys Gln Thr Lys Ile Met Ala Gly Cys
   1880 1885 1890
   Tyr Glu Ala Leu Asp Asp Ser Glu Asp Ile Leu His Asn Ser Leu
   1895 1900 1905
   Asp Asn Asp Glu Cys Ser Thr His Ser His Lys Val Phe Ala Asp
   1910 1915 1920
   Ile Gln Ser Glu Glu Ile Leu Gln His Asn Gln Asn Met Ser Gly
   1925 1930 1935
   Leu Glu Lys Val Ser Lys Ile Ser Pro Cys Asp Val Ser Leu Glu
   1940 1945 1950
   Thr Ser Asp Ile Cys Lys Cys Ser Ile Gly Lys Leu His Lys Ser
   1955 1960 1965
   Val Ser Ser Ala Asn Thr Cys Gly Ile Phe Ser Thr Ala Ser Gly
   1970 1975 1980
   Lys Ser Val Gln Val Ser Asp Ala Ser Leu Gln Asn Ala Arg Gln
   1985 1990 1995
   Val Phe Ser Glu Ile Glu Asp Ser Thr Lys Gln Val Phe Ser Lys
   2000 2005 2010
   Val Leu Phe Lys Ser Asn Glu His Ser Asp Gln Leu Thr Arg Glu
   2015 2020 2025
   Glu Asn Thr Ala Ile Arg Thr Pro Glu His Leu Ile Ser Gln Lys
   2030 2035 2040
   Gly Phe Ser Tyr Asn Val Val Asn Ser Ser Ala Phe Ser Gly Phe
   2045 2050 2055
   Ser Thr Ala Ser Gly Lys Gln Val Ser Ile Leu Glu Ser Ser Leu
   2060 2065 2070
   His Lys Val Lys Gly Val Leu Glu Glu Phe Asp Leu Ile Arg Thr
   2075 2080 2085
   Glu His Ser Leu His Tyr Ser Pro Thr Ser Arg Gln Asn Val Ser
   2090 2095 2100
   Lys Ile Leu Pro Arg Val Asp Lys Arg Asn Pro Glu His Cys Val
   2105 2110 2115
   Asn Ser Glu Met Glu Lys Thr Cys Ser Lys Glu Phe Lys Leu Ser
   2120 2125 2130
   Asn Asn Leu Asn Val Glu Gly Gly Ser Ser Glu Asn Asn His Ser
   2135 2140 2145
   Ile Lys Val Ser Pro Tyr Leu Ser Gln Phe Gln Gln Asp Lys Gln
   2150 2155 2160
   Gln Leu Val Leu Gly Thr Lys Val Ser Leu Val Glu Asn Ile His
   2165 2170 2175
   Val Leu Gly Lys Glu Gln Ala Ser Pro Lys Asn Val Lys Met Glu
   2180 2185 2190
   Ile Gly Lys Thr Glu Thr Phe Ser Asp Val Pro Val Lys Thr Asn
   2195 2200 2205
   Ile Glu Val Cys Ser Thr Tyr Ser Lys Asp Ser Glu Asn Tyr Phe
   2210 2215 2220
   Glu Thr Glu Ala Val Glu Ile Ala Lys Ala Phe Met Glu Asp Asp
   2225 2230 2235
   Glu Leu Thr Asp Ser Lys Leu Pro Ser His Ala Thr His Ser Leu
   2240 2245 2250
   Phe Thr Cys Pro Glu Asn Glu Glu Met Val Leu Ser Asn Ser Arg
   2255 2260 2265
   Ile Gly Lys Arg Arg Gly Glu Pro Leu Ile Leu Val Gly Glu Pro
       2270                2275                2280
   Ser Ile Lys Arg Asn Leu Leu Asn Glu Phe Asp Arg Ile Ile Glu
       2285                2290                2295
   Asn Gln Glu Lys Ser Leu Lys Ala Ser Lys Ser Thr Pro Asp Gly
       2300                2305                2310
   Thr Ile Lys Asp Arg Arg Leu Phe Met His His Val Ser Leu Glu
       2315                2320                2325
   Pro Ile Thr Cys Val Pro Phe Arg Thr Thr Lys Glu Arg Gln Glu
       2330                2335                2340
   Ile Gln Asn Pro Asn Phe Thr Ala Pro Gly Gln Glu Phe Leu Ser
       2345                2350                2355
   Lys Ser His Leu Tyr Glu His Leu Thr Leu Glu Lys Ser Ser Ser
       2360                2365                2370
   Asn Leu Ala Val Ser Gly His Pro Phe Tyr Gln Val Ser Ala Thr
       2375                2380                2385
   Arg Asn Glu Lys Met Arg His Leu Ile Thr Thr Gly Arg Pro Thr
       2390                2395                2400
   Lys Val Phe Val Pro Pro Phe Lys Thr Lys Ser His Phe His Arg
       2405                2410                2415
   Val Glu Gln Cys Val Arg Asn Ile Asn Leu Glu Glu Asn Arg Gln
       2420                2425                2430
   Lys Gln Asn Ile Asp Gly His Gly Ser Asp Asp Ser Lys Asn Lys
       2435                2440                2445
   Ile Asn Asp Asn Glu Ile His Gln Phe Asn Lys Asn Asn Ser Asn
       2450                2455                2460
   Gln Ala Ala Ala Val Thr Phe Thr Lys Cys Glu Glu Glu Pro Leu
       2465                2470                2475
   Asp Leu Ile Thr Ser Leu Gln Asn Ala Arg Asp Ile Gln Asp Met
       2480                2485                2490
   Arg Ile Lys Lys Lys Gln Arg Gln Arg Val Phe Pro Gln Pro Gly
       2495                2500                2505
   Ser Leu Tyr Leu Ala Lys Thr Ser Thr Leu Pro Arg Ile Ser Leu
       2510                2515                2520
   Lys Ala Ala Val Gly Gly Gln Val Pro Ser Ala Cys Ser His Lys
   2525 2530 2535
   Gln Leu Tyr Thr Tyr Gly Val Ser Lys His Cys Ile Lys Ile Asn
   2540 2545 2550
   Ser Lys Asn Ala Glu Ser Phe Gln Phe His Thr Glu Asp Tyr Phe
   2555 2560 2565
   Gly Lys Glu Ser Leu Trp Thr Gly Lys Gly Ile Gln Leu Ala Asp
   2570 2575 2580
   Gly Gly Trp Leu Ile Pro Ser Asn Asp Gly Lys Ala Gly Lys Glu
   2585 2590 2595
   Glu Phe Tyr Arg Ala Leu Cys Asp Thr Pro Gly Val Asp Pro Lys
   2600 2605 2610
   Leu Ile Ser Arg Ile Trp Val Tyr Asn His Tyr Arg Trp Ile Ile
   2615 2620 2625
   Trp Lys Leu Ala Ala Met Glu Cys Ala Phe Pro Lys Glu Phe Ala
   2630 2635 2640
   Asn Arg Cys Leu Ser Pro Glu Arg Val Leu Leu Gln Leu Lys Tyr
   2645 2650 2655
   Arg Tyr Asp Thr Glu Ile Asp Arg Ser Arg Arg Ser Ala Ile Lys
   2660 2665 2670
   Lys Ile Met Glu Arg Asp Asp Thr Ala Ala Lys Thr Leu Val Leu
   2675 2680 2685
   Cys Val Ser Asp Ile Ile Ser Leu Ser Ala Asn Ile Ser Glu Thr
   2690 2695 2700
   Ser Ser Asn Lys Thr Ser Ser Ala Asp Thr Gln Lys Val Ala Ile
   2705 2710 2715
   Ile Glu Leu Thr Asp Gly Trp Tyr Ala Val Lys Ala Gln Leu Asp
   2720 2725 2730
   Pro Pro Leu Leu Ala Val Leu Lys Asn Gly Arg Leu Thr Val Gly
   2735 2740 2745
   Gln Lys Ile Ile Leu His Gly Ala Glu Leu Val Gly Ser Pro Asp
   2750 2755 2760
   Ala Cys Thr Pro Leu Glu Ala Pro Glu Ser Leu Met Leu Lys Ile
   2765 2770 2775
   Ser Ala Asn Ser Thr Arg Pro Ala Arg Trp Tyr Thr Lys Leu Gly
   2780 2785 2790
   Phe Phe Pro Asp Pro Arg Pro Phe Pro Leu Pro Leu Ser Ser Leu
   2795 2800 2805
   Phe Ser Asp Gly Gly Asn Val Gly Cys Val Asp Val Ile Ile Gln
   2810 2815 2820
   Arg Ala Tyr Pro Ile Gln Trp Met Glu Lys Thr Ser Ser Gly Leu
   2825 2830 2835
   Tyr Ile Phe Arg Asn Glu Arg Glu Glu Glu Lys Glu Ala Ala Lys
   2840 2845 2850
   Tyr Val Glu Ala Gln Gln Lys Arg Leu Glu Ala Leu Phe Thr Lys
   2855 2860 2865
   Ile Gln Glu Glu Phe Glu Glu His Glu Glu Asn Thr Thr Lys Pro
   2870 2875 2880
   Tyr Leu Pro Ser Arg Ala Leu Thr Arg Gln Gln Val Arg Ala Leu
   2885 2890 2895
   Gln Asp Gly Ala Glu Leu Tyr Glu Ala Val Lys Asn Ala Ala Asp
   2900 2905 2910
   Pro Ala Tyr Leu Glu Gly Tyr Phe Ser Glu Glu Gln Leu Arg Ala
   2915 2920 2925
   Leu Asn Asn His Arg Gln Met Leu Asn Asp Lys Lys Gln Ala Gln
   2930 2935 2940
   Ile Gln Leu Glu Ile Arg Lys Ala Met Glu Ser Ala Glu Gln Lys
   2945 2950 2955
   Glu Gln Gly Leu Ser Arg Asp Val Thr Thr Val Trp Lys Leu Arg
   2960 2965 2970
   Ile Val Ser Tyr Ser Lys Lys Glu Lys Asp Ser Val Ile Leu Ser
   2975 2980 2985
   Ile Trp Arg Pro Ser Ser Asp Leu Tyr Ser Leu Leu Thr Glu Gly
   2990 2995 3000
   Lys Arg Tyr Arg Ile Tyr His Leu Ala Thr Ser Lys Ser Lys Ser
   3005 3010 3015
   Lys Ser Glu Arg Ala Asn Ile Gln Leu Ala Ala Thr Lys Lys Thr
   3020 3025 3030
   Gln Tyr Gln Gln Leu Pro Val Ser Asp Glu Ile Leu Phe Gln Ile
           3035                3040                3045
   Tyr Gln Pro Arg Glu Pro Leu His Phe Ser Lys Phe Leu Asp Pro
       3050                3055                3060
   Asp Phe Gln Pro Ser Cys Ser Glu Val Asp Leu Ile Gly Phe Val
       3065                3070                3075
   Val Ser Val Val Lys Lys Thr Gly Leu Ala Pro Phe Val Tyr Leu
       3080                3085                3090
   Ser Asp Glu Cys Tyr Asn Leu Leu Ala Ile Lys Phe Trp Ile Asp
       3095                3100                3105
   Leu Asn Glu Asp Ile Ile Lys Pro His Met Leu Ile Ala Ala Ser
       3110                3115                3120
   Asn Leu Gln Trp Arg Pro Glu Ser Lys Ser Gly Leu Leu Thr Leu
       3125                3130                3135
   Phe Ala Gly Asp Phe Ser Val Phe Ser Ala Ser Pro Lys Glu Gly
       3140                3145                3150
   His Phe Gln Glu Thr Phe Asn Lys Met Lys Asn Thr Val Glu Asn
       3155                3160                3165
   Ile Asp Ile Leu Cys Asn Glu Ala Glu Asn Lys Leu Met His Ile
       3170                3175                3180
   Leu His Ala Asn Asp Pro Lys Trp Ser Thr Pro Thr Lys Asp Cys
       3185                3190                3195
   Thr Ser Gly Pro Tyr Thr Ala Gln Ile Ile Pro Gly Thr Gly Asn
       3200                3205                3210
   Lys Leu Leu Met Ser Ser Pro Asn Cys Glu Ile Tyr Tyr Gln Ser
       3215                3220                3225
   Pro Leu Ser Leu Cys Met Ala Lys Arg Lys Ser Val Ser Thr Pro
       3230                3235                3240
   Val Ser Ala Gln Met Thr Ser Lys Ser Cys Lys Gly Glu Lys Glu
       3245                3250                3255
   Ile Asp Asp Gln Lys Asn Cys Lys Lys Arg Arg Ala Leu Asp Phe
       3260                3265                3270
   Leu Ser Arg Leu Pro Leu Pro Pro Pro Val Ser Pro Ile Cys Thr
       3275                3280                3285
   Phe Val Ser Pro Ala Ala Gln Lys Ala Phe Gln Pro Pro Arg Ser
       3290                3295                3300
   Cys Gly  Thr Lys Tyr Glu Thr  Pro Ile Lys Lys Lys  Glu Leu Asn
       3305                 3310                 3315
   Ser Pro  Gln Met Thr Pro Phe  Lys Lys Phe Asn Glu  Ile Ser Leu
       3320                 3325                 3330
   Leu Glu  Ser Asn Ser Ile Ala  Asp Glu Glu Leu Ala  Leu Ile Asn
       3335                 3340                 3345
   Thr Gln  Ala Leu Leu Ser Gly  Ser Thr Gly Glu Lys  Gln Phe Ile
       3350                 3355                 3360
   Ser Val  Ser Glu Ser Thr Arg  Thr Ala Pro Thr Ser  Ser Glu Asp
       3365                 3370                 3375
   Tyr Leu  Arg Leu Lys Arg Arg  Cys Thr Thr Ser Leu  Ile Lys Glu
       3380                 3385                 3390
   Gln Glu  Ser Ser Gln Ala Ser  Thr Glu Glu Cys Glu  Lys Asn Lys
       3395                 3400                 3405
   Gln Asp  Thr Ile Thr Thr Lys  Lys Tyr Ile
       3410                 3415
<210> 10
   <211> 10485
   <212> DNA
   <213> Homo sapiens
<220>
   <221> CDS
   <222> (229)..(10482)
   <223> BRCA2 (OMI4)
<400> 10
   ggtggcgcga gcttctgaaa ctaggcggca gaggcggagc cgctgtggca ctgctgcgcc       60
   tctgctgcgc ctcgggtgtc ttttgcggcg gtgggtcgcc gccgggagaa gcgtgagggg      120
   acagatttgt gaccggcgcg gtttttgtca gcttactccg gccaaaaaag aactgcacct      180
   ctggagcgga cttatttacc aagcattgga ggaatatcgt aggtaaaa atg cct att       237
   gga tcc aaa gag agg cca aca ttt ttt gaa att ttt aag aca cgc tgc        285
   aac aaa gca gat tta gga cca ata agt ctt aat tgg ttt gaa gaa ctt        333
   tct tca gaa gct cca ccc tat aat tct gaa cct gca gaa gaa tct gaa        381
   cat aaa aac aac aat tac gaa cca aac cta ttt aaa act cca caa agg        429
   aaa cca tct tat aat cag ctg gct tca act cca ata ata ttc aaa gag      477
   caa ggg ctg act ctg ccg ctg tac caa tct cct gta aaa gaa tta gat      525
   aaa ttc aaa tta gac tta gga agg aat gtt ccc aat agt aga cat aaa      573
   agt ctt cgc aca gtg aaa act aaa atg gat caa gca gat gat gtt tcc      621
   tgt cca ctt cta aat tct tgt ctt agt gaa agt cct gtt gtt cta caa      669
   tgt aca cat gta aca cca caa aga gat aag tca gtg gta tgt ggg agt      717
   ttg ttt cat aca cca aag ttt gtg aag ggt cgt cag aca cca aaa cat      765
   att tct gaa agt cta gga gct gag gtg gat cct gat atg tct tgg tca      813
   agt tct tta gct aca cca ccc acc ctt agt tct act gtg ctc ata gtc      861
   aga aat gaa gaa gca tct gaa act gta ttt cct cat gat act act gct      909
   aat gtg aaa agc tat ttt tcc aat cat gat gaa agt ctg aag aaa aat      957
   gat aga ttt atc gct tct gtg aca gac agt gaa aac aca aat caa aga     1005
   gaa gct gca agt cat gga ttt gga aaa aca tca ggg aat tca ttt aaa     1053
   gta aat agc tgc aaa gac cac att gga aag tca atg cca aat gtc cta     1101
   gaa gat gaa gta tat gaa aca gtt gta gat acc tct gaa gaa gat agt     1149
   ttt tca tta tgt ttt tct aaa tgt aga aca aaa aat cta caa aaa gta     1197
   aga act agc aag act agg aaa aaa att ttc cat gaa gca aac gct gat     1245
   gaa tgt gaa aaa tct aaa aac caa gtg aaa gaa aaa tac tca ttt gta      1293
   tct gaa gtg gaa cca aat gat act gat cca tta gat tca aat gta gca      1341
   cat cag aag ccc ttt gag agt gga agt gac aaa atc tcc aag gaa gtt      1389
   gta ccg tct ttg gcc tgt gaa tgg tct caa cta acc ctt tca ggt cta      1437
   aat gga gcc cag atg gag aaa ata ccc cta ttg cat att tct tca tgt      1485
   gac caa aat att tca gaa aaa gac cta tta gac aca gag aac aaa aga      1533
   aag aaa gat ttt ctt act tca gag aat tct ttg cca cgt att tct agc      1581
   cta cca aaa tca gag aag cca tta aat gag gaa aca gtg gta aat aag      1629
   aga gat gaa gag cag cat ctt gaa tct cat aca gac tgc att ctt gca      1677
   gta aag cag gca ata tct gga act tct cca gtg gct tct tca ttt cag      1725
   ggt atc aaa aag tct ata ttc aga ata aga gaa tca cct aaa gag act      1773
   ttc aat gca agt ttt tca ggt cat atg act gat cca aac ttt aaa aaa      1821
   gaa act gaa gcc tct gaa agt gga ctg gaa ata cat act gtt tgc tca      1869
   cag aag gag gac tcc tta tgt cca aat tta att gat aat gga agc tgg      1917
   cca gcc acc acc aca cag aat tct gta gct ttg aag aat gca ggt tta      1965
   ata tcc act ttg aaa aag aaa aca aat aag ttt att tat gct ata cat      2013
   gat gaa aca tct tat aaa gga aaa aaa ata ccg aaa gac caa aaa tca      2061
   gaa cta att aac tgt tca gcc cag ttt gaa gca aat gct ttt gaa gca      2109
   cca ctt aca ttt gca aat gct gat tca ggt tta ttg cat tct tct gtg      2157
   aaa aga agc tgt tca cag aat gat tct gaa gaa cca act ttg tcc tta      2205
   act agc tct ttt ggg aca att ctg agg aaa tgt tct aga aat gaa aca      2253
   tgt tct aat aat aca gta atc tct cag gat ctt gat tat aaa gaa gca      2301
   aaa tgt aat aag gaa aaa cta cag tta ttt att acc cca gaa gct gat      2349
   tct ctg tca tgc ctg cag gaa gga cag tgt gaa aat gat cca aaa agc      2397
   aaa aaa gtt tca gat ata aaa gaa gag gtc ttg gct gca gca tgt cac      2445
   cca gta caa cat tca aaa gtg gaa tac agt gat act gac ttt caa tcc      2493
   cag aaa agt ctt tta tat gat cat gaa aat gcc agc act ctt att tta      2541
   act cct act tcc aag gat gtt ctg tca aac cta gtc atg att tct aga      2589
   ggc aaa gaa tca tac aaa atg tca gac aag ctc aaa ggt aac aat tat      2637
   gaa tct gat gtt gaa tta acc aaa aat att ccc atg gaa aag aat caa      2685
   gat gta tgt gct tta aat gaa aat tat aaa aac gtt gag ctg ttg cca      2733
   cct gaa aaa tac atg aga gta gca tca cct tca aga aag gta caa ttc      2781
   aac caa aac aca aat cta aga gta atc caa aaa aat caa gaa gaa act      2829
   act tca att tca aaa ata act gtc aat cca gac tct gaa gaa ctt ttc      2877
   tca gac aat gag aat aat ttt gtc ttc caa gta gct aat gaa agg aat        2925
   aat ctt gct tta gga aat act aag gaa ctt cat gaa aca gac ttg act        2973
   tgt gta aac gaa ccc att ttc aag aac tct acc atg gtt tta tat gga        3021
   gac aca ggt gat aaa caa gca acc caa gtg tca att aaa aaa gat ttg        3069
   gtt tat gtt ctt gca gag gag aac aaa aat agt gta aag cag cat ata        3117
   aaa atg act cta ggt caa gat tta aaa tcg gac atc tcc ttg aat ata        3165
   gat aaa ata cca gaa aaa aat aat gat tac atg aac aaa tgg gca gga        3213
   ctc tta ggt cca att  tca aat cac agt ttt  gga ggt agc ttc aga          3258
   aca gct tca aat aag  gaa atc aag ctc tct  gaa cat aac att aag          3303
   aag agc aaa atg ttc  ttc aaa gat att gaa  gaa caa tat cct act          3348
   agt tta gct tgt gtt  gaa att gta aat acc  ttg gca tta gat aat          3393
   caa aag aaa ctg agc  aag cct cag tca att  aat act gta tct gca          3438
   cat tta cag agt agt  gta gtt gtt tct gat  tgt aaa aat agt cat          3483
   ata acc cct cag atg  tta ttt tcc aag cag  gat ttt aat tca aac          3528
   cat aat tta aca cct  agc caa aag gca gaa  att aca gaa ctt tct          3573
   act ata tta gaa gaa  tca gga agt cag ttt  gaa ttt act cag ttt          3618
   aga aag cca agc tac  ata ttg cag aag agt  aca ttt gaa gtg cct          3663
   gaa aac cag atg act  atc tta aag acc act  tct gag gaa tgc aga          3708
   gat gct gat ctt cat  gtc ata atg aat gcc  cca tcg att ggt cag        3753
   gta gac agc agc aag  caa ttt gaa ggt aca  gtt gaa att aaa cgg        3798
   aag ttt gct ggc ctg  ttg aaa aat gac tgt  aac aaa agt gct tct        3843
   ggt tat tta aca gat  gaa aat gaa gtg ggg  ttt agg ggc ttt tat        3888
   tct gct cat ggc aca  aaa ctg aat gtt tct  act gaa gct ctg caa        3933
   aaa gct gtg aaa ctg  ttt agt gat att gag  aat att agt gag gaa        3978
   act tct gca gag gta  cat cca ata agt tta  tct tca agt aaa tgt        4023
   cat gat tct gtt gtt  tca atg ttt aag ata  gaa aat cat aat gat        4068
   aaa act gta agt gaa  aaa aat aat aaa tgc  caa ctg ata tta caa        4113
   aat aat att gaa atg  act act ggc act ttt  gtt gaa gaa att act        4158
   gaa aat tac aag aga  aat act gaa aat gaa  gat aac aaa tat act        4203
   gct gcc agt aga aat  tct cat aac tta gaa  ttt gat ggc agt gat        4248
   tca agt aaa aat gat  act gtt tgt att cat  aaa gat gaa acg gac        4293
   ttg cta ttt act gat  cag cac aac ata tgt  ctt aaa tta tct ggc        4338
   cag ttt atg aag gag  gga aac act cag att  aaa gaa gat ttg tca        4383
   gat tta act ttt ttg  gaa gtt gcg aaa gct  caa gaa gca tgt cat        4428
   ggt aat act tca aat  aaa gaa cag tta act  gct act aaa acg gag        4473
   caa aat ata aaa gat  ttt gag act tct gat  aca ttt ttt cag act     4518
   gca agt ggg aaa aat  att agt gtc gcc aaa  gag tca ttt aat aaa     4563
   att gta aat ttc ttt  gat cag aaa cca gaa  gaa ttg cat aac ttt     4608
   tcc tta aat tct gaa  tta cat tct gac ata  aga aag aac aaa atg     4653
   gac att cta agt tat  gag gaa aca gac ata  gtt aaa cac aaa ata     4698
   ctg aaa gaa agt gtc  cca gtt ggt act gga  aat caa cta gtg acc     4743
   ttc cag gga caa ccc  gaa cgt gat gaa aag  atc aaa gaa cct act     4788
   ctg ttg ggt ttt cat  aca gct agc ggg aaa  aaa gtt aaa att gca     4833
   aag gaa tct ttg gac  aaa gtg aaa aac ctt  ttt gat gaa aaa gag     4878
   caa ggt act agt gaa  atc acc agt ttt agc  cat caa tgg gca aag     4923
   acc cta aag tac aga  gag gcc tgt aaa gac  ctt gaa tta gca tgt     4968
   gag acc att gag atc  aca gct gcc cca aag  tgt aaa gaa atg cag     5013
   aat tct ctc aat aat  gat aaa aac ctt gtt  tct att gag act gtg     5058
   gtg cca cct aag ctc  tta agt gat aat tta  tgt aga caa act gaa     5103
   aat ctc aaa aca tca  aaa agt atc ttt ttg  aaa gtt aaa gta cat     5148
   gaa aat gta gaa aaa  gaa aca gca aaa agt  cct gca act tgt tac     5193
   aca aat cag tcc cct  tat tca gtc att gaa  aat tca gcc tta gct     5238
   ttt tac aca agt tgt  agt aga aaa act tct  gtg agt cag act tca      5283
   tta ctt gaa gca aaa  aaa tgg ctt aga gaa  gga ata ttt gat ggt      5328
   caa cca gaa aga ata  aat act gca gat tat  gta gga aat tat ttg      5373
   tat gaa aat aat tca  aac agt act ata gct  gaa aat gac aaa aat      5418
   cat ctc tcc gaa aaa  caa gat act tat tta  agt aac agt agc atg      5463
   tct aac agc tat tcc  tac cat tct gat gag  gta tat aat gat tca      5508
   gga tat ctc tca aaa  aat aaa ctt gat tct  ggt att gag cca gta      5553
   ttg aag aat gtt gaa  gat caa aaa aac act  agt ttt tcc aaa gta      5598
   ata tcc aat gta aaa  gat gca aat gca tac  cca caa act gta aat      5643
   gaa gat att tgc gtt  gag gaa ctt gtg act  agc tct tca ccc tgc      5688
   aaa aat aaa aat gca  gcc att aaa ttg tcc  ata tct aat agt aat      5733
   aat ttt gag gta ggg  cca cct gca ttt agg  ata gcc agt ggt aaa      5778
   atc gtt tgt gtt tca  cat gaa aca att aaa  aaa gtg aaa gac ata      5823
   ttt aca gac agt ttc  agt aaa gta att aag  gaa aac aac gag aat      5868
   aaa tca aaa att tgc  caa acg aaa att atg  gca ggt tgt tac gag      5913
   gca ttg gat gat tca  gag gat att ctt cat  aac tct cta gat aat      5958
   gat gaa tgt agc acg  cat tca cat aag gtt  ttt gct gac att cag      6003
   agt gaa gaa att tta  caa cat aac caa aat  atg tct gga ttg gag     6048
   aaa gtt tct aaa ata  tca cct tgt gat gtt  agt ttg gaa act tca     6093
   gat ata tgt aaa tgt  agt ata ggg aag ctt  cat aag tca gtc tca     6138
   tct gca aat act tgt  ggg att ttt agc aca  gca agt gga aaa tct     6183
   gtc cag gta tca gat  gct tca tta caa aac  gca aga caa gtg ttt     6228
   tct gaa ata gaa gat  agt acc aag caa gtc  ttt tcc aaa gta ttg     6273
   ttt aaa agt aac gaa  cat tca gac cag ctc  aca aga gaa gaa aat     6318
   act gct ata cgt act  cca gaa cat tta ata  tcc caa aaa ggc ttt     6363
   tca tat aat gtg gta  aat tca tct gct ttc  tct gga ttt agt aca     6408
   gca agt gga aag caa  gtt tcc att tta gaa  agt tcc tta cac aaa     6453
   gtt aag gga gtg tta  gag gaa ttt gat tta  atc aga act gag cat     6498
   agt ctt cac tat tca  cct acg tct aga caa  aat gta tca aaa ata     6543
   ctt cct cgt gtt gat  aag aga aac cca gag  cac tgt gta aac tca     6588
   gaa atg gaa aaa acc  tgc agt aaa gaa ttt  aaa tta tca aat aac     6633
   tta aat gtt gaa ggt  ggt tct tca gaa aat  aat cac tct att aaa     6678
   gtt tct cca tat ctc  tct caa ttt caa caa  gac aaa caa cag ttg     6723
   gta tta gga acc aaa  gtc tca ctt gtt gag  aac att cat gtt ttg     6768
   gga aaa gaa cag gct  tca cct aaa aac gta  aaa atg gaa att ggt     6813
   aaa act gaa act ttt  tct gat gtt cct gtg  aaa aca aat ata gaa      6858
   gtt tgt tct act tac  tcc aaa gat tca gaa  aac tac ttt gaa aca      6903
   gaa gca gta gaa att  gct aaa gct ttt atg  gaa gat gat gaa ctg      6948
   aca gat tct aaa ctg  cca agt cat gcc aca  cat tct ctt ttt aca      6993
   tgt ccc gaa aat gag  gaa atg gtt ttg tca  aat tca aga att gga      7038
   aaa aga aga gga gag  ccc ctt atc tta gtg  gga gaa ccc tca atc      7083
   aaa aga aac tta tta  aat gaa ttt gac agg  ata ata gaa aat caa      7128
   gaa aaa tcc tta aag  gct tca aaa agc act  cca gat ggc aca ata      7173
   aaa gat cga aga ttg  ttt atg cat cat gtt  tct tta gag ccg att      7218
   acc tgt gta ccc ttt  cgc aca act aag gaa  cgt caa gag ata cag      7263
   aat cca aat ttt acc  gca cct ggt caa gaa  ttt ctg tct aaa tct      7308
   cat ttg tat gaa cat  ctg act ttg gaa aaa  tct tca agc aat tta      7353
   gca gtt tca gga cat  cca ttt tat caa gtt  tct gct aca aga aat      7398
   gaa aaa atg aga cac  ttg att act aca ggc  aga cca acc aaa gtc      7443
   ttt gtt cca cct ttt  aaa act aaa tcg cat  ttt cac aga gtt gaa      7488
   cag tgt gtt agg aat  att aac ttg gag gaa  aac aga caa aag caa      7533
   aac att gat gga cat  ggc tct gat gat agt  aaa aat aag att aat      7578
   gac aat gag att cat  cag ttt aac aaa aac  aac tcc aat caa gca        7623
   gca gct gta act ttc  aca aag tgt gaa gaa  gaa cct tta gat tta        7668
   att aca agt ctt cag  aat gcc aga gat ata  cag gat atg cga att        7713
   aag aag aaa caa agg  caa cgc gtc ttt cca  cag cca ggc agt ctg        7758
   tat ctt gca aaa aca  tcc act ctg cct cga  atc tct ctg aaa gca        7803
   gca gta gga ggc caa  gtt ccc tct gcg tgt  tct cat aaa cag ctg        7848
   tat acg tat ggc gtt  tct aaa cat tgc ata  aaa att aac agc aaa        7893
   aat gca gag tct ttt  cag ttt cac act gaa  gat tat ttt ggt aag        7938
   gaa agt tta tgg act  gga aaa gga ata cag  ttg gct gat ggt gga        7983
   tgg ctc ata ccc tcc  aat gat gga aag gct  gga aaa gaa gaa ttt        8028
   tat agg gct ctg tgt  gac act cca ggt gtg  gat cca aag ctt att        8073
   tct aga att tgg gtt  tat aat cac tat aga  tgg atc ata tgg aaa        8118
   ctg gca gct atg gaa  tgt gcc ttt cct aag  gaa ttt gct aat aga        8163
   tgc cta agc cca gaa  agg gtg ctt ctt caa  cta aaa tac aga tat        8208
   gat acg gaa att gat  aga agc aga aga tcg  gct ata aaa aag ata        8253
   atg gaa agg gat gac  aca gct gca aaa aca  ctt gtt ctc tgt gtt        8298
   tct gac ata att tca  ttg agc gca aat ata  tct gaa act tct agc        8343
   aat aaa act agt agt  gca gat acc caa aaa  gtg gcc att att gaa       8388
   ctt aca gat ggg tgg  tat gct gtt aag gcc  cag tta gat cct ccc       8433
   ctc tta gct gtc tta  aag aat ggc aga ctg  aca gtt ggt cag aag       8478
   att att ctt cat gga  gca gaa ctg gtg ggc  tct cct gat gcc tgt       8523
   aca cct ctt gaa gcc  cca gaa tct ctt atg  tta aag att tct gct       8568
   aac agt act cgg cct  gct cgc tgg tat acc  aaa ctt gga ttc ttt       8613
   cct gac cct aga cct  ttt cct ctg ccc tta  tca tcg ctt ttc agt       8658
   gat gga gga aat gtt  ggt tgt gtt gat gta  att att caa aga gca       8703
   tac cct ata cag tgg  atg gag aag aca tca  tct gga tta tac ata       8748
   ttt cgc aat gaa aga  gag gaa gaa aag gaa  gca gca aaa tat gtg       8793
   gag gcc caa caa aag  aga cta gaa gcc tta  ttc act aaa att cag       8838
   gag gaa ttt gaa gaa  cat gaa gaa aac aca  aca aaa cca tat tta       8883
   cca tca cgt gca cta  aca aga cag caa gtt  cgt gct ttg caa gat       8928
   ggt gca gag ctt tat  gaa gca gtg aag aat  gca gca gac cca gct       8973
   tac ctt gag ggt tat  ttc agt gaa gag cag  tta aga gcc ttg aat       9018
   aat cac agg caa atg  ttg aat gat aag aaa  caa gct cag atc cag       9063
   ttg gaa att agg aag  gcc atg gaa tct gct  gaa caa aag gaa caa       9108
   ggt tta tca agg gat  gtc aca acc gtg tgg  aag ttg cgt att gta       9153
   agc tat tca aaa aaa  gaa aaa gat tca gtt  ata ctg agt att tgg       9198
   cgt cca tca tca gat  tta tat tct ctg tta  aca gaa gga aag aga       9243
   tac aga att tat cat  ctt gca act tca aaa  tct aaa agt aaa tct       9288
   gaa aga gct aac ata  cag tta gca gcg aca  aaa aaa act cag tat       9333
   caa caa cta ccg gtt  tca gat gaa att tta  ttt cag att tac cag       9378
   cca cgg gag ccc ctt  cac ttc agc aaa ttt  tta gat cca gac ttt       9423
   cag cca tct tgt tct  gag gtg gac cta ata  gga ttt gtc gtt tct       9468
   gtt gtg aaa aaa aca  gga ctt gcc cct ttc  gtc tat ttg tca gac       9513
   gaa tgt tac aat tta  ctg gca ata aag ttt  tgg ata gac ctt aat       9558
   gag gac att att aag  cct cat atg tta att  gct gca agc aac ctc       9603
   cag tgg cga cca gaa  tcc aaa tca ggc ctt  ctt act tta ttt gct       9648
   gga gat ttt tct gtg  ttt tct gct agt cca  aaa gag ggc cac ttt       9693
   caa gag aca ttc aac  aaa atg aaa aat act  gtt gag aat att gac       9738
   ata ctt tgc aat gaa  gca gaa aac aag ctt  atg cat ata ctg cat       9783
   gca aat gat ccc aag  tgg tcc acc cca act  aaa gac tgt act tca       9828
   ggg ccg tac act gct  caa atc att cct ggt  aca gga aac aag ctt       9873
   ctg atg tct tct cct  aat tgt gag ata tat  tat caa agt cct tta       9918
   tca ctt tgt atg gcc  aaa agg aag tct gtt  tcc aca cct gtc tca       9963
   gcc cag atg act tca  aag tct tgt aaa ggg  gag aaa gag att gat      10008
   gac caa aag aac tgc  aaa aag aga aga gcc  ttg gat ttc ttg agt      10053
   aga ctg cct tta cct  cca cct gtt agt ccc  att tgt aca ttt gtt      10098
   tct ccg gct gca cag  aag gca ttt cag cca  cca agg agt tgt ggc      10143
   acc aaa tac gaa aca  ccc ata aag aaa aaa  gaa ctg aat tct cct      10188
   cag atg act cca ttt  aaa aaa ttc aat gaa  att tct ctt ttg gaa      10233
   agt aat tca ata gct  gac gaa gaa ctt gca  ttg ata aat acc caa      10278
   gct ctt ttg tct ggt  tca aca gga gaa aaa  caa ttt ata tct gtc      10323
   agt gaa tcc act agg  act gct ccc acc agt  tca gaa gat tat ctc      10368
   aga ctg aaa cga cgt  tgt act aca tct ctg  atc aaa gaa cag gag      10413
   agt tcc cag gcc agt  acg gaa gaa tgt gag  aaa aat aag cag gac      10458
   aca att aca act aaa  aaa tat atc taa                               10485
<210> 11
   <211> 3418
   <212> PRT
   <213> Homo sapiens
<400> 11
<210> 12
   <211> 10485
   <212> DNA
   <213> Homo sapiens
<220>
   <221> CDS
   <222> (229)..(10482)
   <223> BRCA2 (OMI5)
<400> 12
   ggtggcgcga gcttctgaaa ctaggcggca gaggcggagc cgctgtggca ctgctgcgcc      60
   tctgctgcgc ctcgggtgtc ttttgcggcg gtgggtcgcc gccgggagaa gcgtgagggg     120
   acagatttgt gaccggcgcg gtttttgtca gcttactccg gccaaaaaag aactgcacct     180
   ctggagcgga cttatttacc aagcattgga ggaatatcgt aggtaaaa atg cct att      237
                                                        Met Pro Ile
                                                        1
   gga tcc aaa gag agg cca aca ttt ttt gaa att ttt aag aca cgc tgc       285
   Gly Ser Lys Glu Arg Pro Thr Phe Phe Glu Ile Phe Lys Thr Arg Cys
       5                   10                  15
   aac aaa gca gat tta gga cca ata agt ctt aat tgg ttt gaa gaa ctt       333
   Asn Lys Ala Asp Leu Gly Pro Ile Ser Leu Asn Trp Phe Glu Glu Leu
   20                  25                  30                  35
   tct tca gaa gct cca ccc tat aat tct gaa cct gca gaa gaa tct gaa       381
   Ser Ser Glu Ala Pro Pro Tyr Asn Ser Glu Pro Ala Glu Glu Ser Glu
                   40                  45                  50
   cat aaa aac aac aat tac gaa cca aac cta ttt aaa act cca caa agg       429
   His Lys Asn Asn Asn Tyr Glu Pro Asn Leu Phe Lys Thr Pro Gln Arg
               55                  60                  65
   aaa cca tct tat aat cag ctg gct tca act cca ata ata ttc aaa gag       477
   Lys Pro Ser Tyr Asn Gln Leu Ala Ser Thr Pro Ile Ile Phe Lys Glu
           70                  75                  80
   caa ggg ctg act ctg ccg ctg tac caa tct cct gta aaa gaa tta gat       525
   Gln Gly Leu Thr Leu Pro Leu Tyr Gln Ser Pro Val Lys Glu Leu Asp
       85                  90                  95
   aaa ttc aaa tta gac tta gga agg aat gtt ccc aat agt aga cat aaa       573
   Lys Phe Lys Leu Asp Leu Gly Arg Asn Val Pro Asn Ser Arg His Lys
   100                 105                 110                 115
   agt ctt cgc aca gtg aaa act aaa atg gat caa gca gat gat gtt tcc       621
   Ser Leu Arg Thr Val Lys Thr Lys Met Asp Gln Ala Asp Asp Val Ser
                   120                 125                 130
   tgt cca ctt cta aat tct tgt ctt agt gaa agt cct gtt gtt cta caa       669
   Cys Pro Leu Leu Asn Ser Cys Leu Ser Glu Ser Pro Val Val Leu Gln
               135                 140                 145
   tgt aca cat gta aca cca caa aga gat aag tca gtg gta tgt ggg agt       717
   Cys Thr His Val Thr Pro Gln Arg Asp Lys Ser Val Val Cys Gly Ser
           150                 155                 160
   ttg ttt cat aca cca aag ttt gtg aag ggt cgt cag aca cca aaa cat       765
   Leu Phe His Thr Pro Lys Phe Val Lys Gly Arg Gln Thr Pro Lys His
       165                 170                 175
   att tct gaa agt cta gga gct gag gtg gat cct gat atg tct tgg tca       813
   Ile Ser Glu Ser Leu Gly Ala Glu Val Asp Pro Asp Met Ser Trp Ser
   180                 185                 190                 195
   agt tct tta gct aca cca ccc acc ctt agt tct act gtg ctc ata gtc       861
   Ser Ser Leu Ala Thr Pro Pro Thr Leu Ser Ser Thr Val Leu Ile Val
                   200                 205                 210
   aga aat gaa gaa gca tct gaa act gta ttt cct cat gat act act gct       909
   Arg Asn Glu Glu Ala Ser Glu Thr Val Phe Pro His Asp Thr Thr Ala
                   215                     220                     225
   aat gtg aaa agc tat ttt tcc aat cat gat gaa agt ctg aag aaa aat      957
   Asn Val Lys Ser Tyr Phe Ser Asn His Asp Glu Ser Leu Lys Lys Asn
           230                     235                     240
   gat aga ttt atc gct tct gtg aca gac agt gaa aac aca aat caa aga     1005
   Asp Arg Phe Ile Ala Ser Val Thr Asp Ser Glu Asn Thr Asn Gln Arg
       245                     250                     255
   gaa gct gca agt cat gga ttt gga aaa aca tca ggg aat tca ttt aaa     1053
   Glu Ala Ala Ser His Gly Phe Gly Lys Thr Ser Gly Asn Ser Phe Lys
   260                     265                     270                 275
   gta aat agc tgc aaa gac cac att gga aag tca atg cca cat gtc cta     1101
   Val Asn Ser Cys Lys Asp His Ile Gly Lys Ser Met Pro His Val Leu
                   280                     285                     290
   gaa gat gaa gta tat gaa aca gtt gta gat acc tct gaa gaa gat agt     1149
   Glu Asp Glu Val Tyr Glu Thr Val Val Asp Thr Ser Glu Glu Asp Ser
               295                     300                     305
   ttt tca tta tgt ttt tct aaa tgt aga aca aaa aat cta caa aaa gta     1197
   Phe Ser Leu Cys Phe Ser Lys Cys Arg Thr Lys Asn Leu Gln Lys Val
           310                     315                     320
   aga act agc aag act agg aaa aaa att ttc cat gaa gca aac gct gat     1245
   Arg Thr Ser Lys Thr Arg Lys Lys Ile Phe His Glu Ala Asn Ala Asp
       325                     330                     335
   gaa tgt gaa aaa tct aaa aac caa gtg aaa gaa aaa tac tca ttt gta     1293
   Glu Cys Glu Lys Ser Lys Asn Gln Val Lys Glu Lys Tyr Ser Phe Val
   340                     345                     350                 355
   tct gaa gtg gaa cca aat gat act gat cca tta gat tca aat gta gca     1341
   Ser Glu Val Glu Pro Asn Asp Thr Asp Pro Leu Asp Ser Asn Val Ala
                   360                     365                     370
   cat cag aag ccc ttt gag agt gga agt gac aaa atc tcc aag gaa gtt     1389
   His Gln Lys Pro Phe Glu Ser Gly Ser Asp Lys Ile Ser Lys Glu Val
               375                     380                     385
   gta ccg tct ttg gcc tgt gaa tgg tct caa cta acc ctt tca ggt cta     1437
   Val Pro Ser Leu Ala Cys Glu Trp Ser Gln Leu Thr Leu Ser Gly Leu
           390                     395                     400
   aat gga gcc cag atg gag aaa ata ccc cta ttg cat att tct tca tgt     1485
   Asn Gly Ala Gln Met Glu Lys Ile Pro Leu Leu His Ile Ser Ser Cys
       405                     410                     415
   gac caa aat att tca gaa aaa gac cta tta gac aca gag aac aaa aga     1533
   Asp Gln Asn Ile Ser Glu Lys Asp Leu Leu Asp Thr Glu Asn Lys Arg
   420                     425                     430                 435
   aag aaa gat ttt ctt act tca gag aat tct ttg cca cgt att tct agc     1581
   Lys Lys Asp Phe Leu Thr Ser Glu Asn Ser Leu Pro Arg Ile Ser Ser
                   440                     445                     450
   cta cca aaa tcg gag aag cca tta aat gag gaa aca gtg gta aat aag     1629
   Leu Pro Lys Ser Glu Lys Pro Leu Asn Glu Glu Thr Val Val Asn Lys
               455                     460                     465
   aga gat gaa gag cag cat ctt gaa tct cat aca gac tgc att ctt gca     1677
   Arg Asp Glu Glu Gln His Leu Glu Ser His Thr Asp Cys Ile Leu Ala
           470                     475                     480
   gta aag cag gca ata tct gga act tct cca gtg gct tct tca ttt cag     1725
   Val Lys Gln Ala Ile Ser Gly Thr Ser Pro Val Ala Ser Ser Phe Gln
       485                     490                     495
   ggt atc aaa aag tct ata ttc aga ata aga gaa tca cct aaa gag act        1773
   Gly Ile Lys Lys Ser Ile Phe Arg Ile Arg Glu Ser Pro Lys Glu Thr
   500                 505                 510                 515
   ttc aat gca agt ttt tca ggt cat atg act gat cca aac ttt aaa aaa        1821
   Phe Asn Ala Ser Phe Ser Gly His Met Thr Asp Pro Asn Phe Lys Lys
                   520                 525                 530
   gaa act gaa gcc tct gaa agt gga ctg gaa ata cat act gtt tgc tca        1869
   Glu Thr Glu Ala Ser Glu Ser Gly Leu Glu Ile His Thr Val Cys Ser
               535                 540                 545
   cag aag gag gac tcc tta tgt cca aat tta att gat aat gga agc tgg        1917
   Gln Lys Glu Asp Ser Leu Cys Pro Asn Leu Ile Asp Asn Gly Ser Trp
           550                 555                 560
   cca gcc acc acc aca cag aat tct gta gct ttg aag aat gca ggt tta        1965
   Pro Ala Thr Thr Thr Gln Asn Ser Val Ala Leu Lys Asn Ala Gly Leu
       565                 570                 575
   ata tcc act ttg aaa aag aaa aca aat aag ttt att tat gct ata cat        2013
   Ile Ser Thr Leu Lys Lys Lys Thr Asn Lys Phe Ile Tyr Ala Ile His
   580                 585                 590                 595
   gat gaa aca tct tat aaa gga aaa aaa ata ccg aaa gac caa aaa tca        2061
   Asp Glu Thr Ser Tyr Lys Gly Lys Lys Ile Pro Lys Asp Gln Lys Ser
                   600                 605                 610
   gaa cta att aac tgt tca gcc cag ttt gaa gca aat gct ttt gaa gca        2109
   Glu Leu Ile Asn Cys Ser Ala Gln Phe Glu Ala Asn Ala Phe Glu Ala
               615                 620                 625
   cca ctt aca ttt gca aat gct gat tca ggt tta ttg cat tct tct gtg        2157
   Pro Leu Thr Phe Ala Asn Ala Asp Ser Gly Leu Leu His Ser Ser Val
           630                 635                 640
   aaa aga agc tgt tca cag aat gat tct gaa gaa cca act ttg tcc tta        2205
   Lys Arg Ser Cys Ser Gln Asn Asp Ser Glu Glu Pro Thr Leu Ser Leu
       645                 650                 655
   act agc tct ttt ggg aca att ctg agg aaa tgt tct aga aat gaa aca        2253
   Thr Ser Ser Phe Gly Thr Ile Leu Arg Lys Cys Ser Arg Asn Glu Thr
   660                 665                 670                 675
   tgt tct aat aat aca gta atc tct cag gat ctt gat tat aaa gaa gca        2301
   Cys Ser Asn Asn Thr Val Ile Ser Gln Asp Leu Asp Tyr Lys Glu Ala
                   680                 685                 690
   aaa tgt aat aag gaa aaa cta cag tta ttt att acc cca gaa gct gat        2349
   Lys Cys Asn Lys Glu Lys Leu Gln Leu Phe Ile Thr Pro Glu Ala Asp
               695                 700                 705
   tct ctg tca tgc ctg cag gaa gga cag tgt gaa aat gat cca aaa agc        2397
   Ser Leu Ser Cys Leu Gln Glu Gly Gln Cys Glu Asn Asp Pro Lys Ser
           710                 715                 720
   aaa aaa gtt tca gat ata aaa gaa gag gtc ttg gct gca gca tgt cac        2445
   Lys Lys Val Ser Asp Ile Lys Glu Glu Val Leu Ala Ala Ala Cys His
       725                 730                 735
   cca gta caa cac tca aaa gtg gaa tac agt gat act gac ttt caa tcc        2493
   Pro Val Gln His Ser Lys Val Glu Tyr Ser Asp Thr Asp Phe Gln Ser
   740                 745                 750                 755
   cag aaa agt ctt tta tat gat cat gaa aat gcc agc act ctt att tta        2541
   Gln Lys Ser Leu Leu Tyr Asp His Glu Asn Ala Ser Thr Leu Ile Leu
                   760                 765                 770
   act cct act tcc aag gat gtt ctg tca aac cta gtc atg att tct aga      2589
   Thr Pro Thr Ser Lys Asp Val Leu Ser Asn Leu Val Met Ile Ser Arg
               775                 780                 785
   ggc aaa gaa tca tac aaa atg tca gac aag ctc aaa ggt aac aat tat      2637
   Gly Lys Glu Ser Tyr Lys Met Ser Asp Lys Leu Lys Gly Asn Asn Tyr
           790                 795                 800
   gaa tct gat gtt gaa tta acc aaa aat att ccc atg gaa aag aat caa      2685
   Glu Ser Asp Val Glu Leu Thr Lys Asn Ile Pro Met Glu Lys Asn Gln
       805                 810                 815
   gat gta tgt gct tta aat gaa aat tat aaa aac gtt gag ctg ttg cca      2733
   Asp Val Cys Ala Leu Asn Glu Asn Tyr Lys Asn Val Glu Leu Leu Pro
   820                 825                 830                 835
   cct gaa aaa tac atg aga gta gca tca cct tca aga aag gta caa ttc      2781
   Pro Glu Lys Tyr Met Arg Val Ala Ser Pro Ser Arg Lys Val Gln Phe
                   840                 845                 850
   aac caa aac aca aat cta aga gta atc caa aaa aat caa gaa gaa act      2829
   Asn Gln Asn Thr Asn Leu Arg Val Ile Gln Lys Asn Gln Glu Glu Thr
               855                 860                 865
   act tca att tca aaa ata act gtc aat cca gac tct gaa gaa ctt ttc      2877
   Thr Ser Ile Ser Lys Ile Thr Val Asn Pro Asp Ser Glu Glu Leu Phe
           870                 875                 880
   tca gac aat gag aat aat ttt gtc ttc caa ata gct aat gaa agg aat      2925
   Ser Asp Asn Glu Asn Asn Phe Val Phe Gln Ile Ala Asn Glu Arg Asn
       885                 890                 895
   aat ctt gct tta gga aat act aag gaa ctt cat gaa aca gac ttg act      2973
   Asn Leu Ala Leu Gly Asn Thr Lys Glu Leu His Glu Thr Asp Leu Thr
   900                 905                 910                 915
   tgt gta aac gaa ccc att ttc aag aac tct acc atg gtt tta tat gga      3021
   Cys Val Asn Glu Pro Ile Phe Lys Asn Ser Thr Met Val Leu Tyr Gly
                   920                 925                 930
   gac aca ggt gat aaa caa gca acc caa gtg tca att aaa aaa gat ttg      3069
   Asp Thr Gly Asp Lys Gln Ala Thr Gln Val Ser Ile Lys Lys Asp Leu
               935                 940                 945
   gtt tat gtt ctt gca gag gag aac aaa aat agt gta aag cag cat ata      3117
   Val Tyr Val Leu Ala Glu Glu Asn Lys Asn Ser Val Lys Gln His Ile
           950                 955                 960
   aaa atg act cta ggt caa gat tta aaa tcg gac atc tcc ttg aat ata      3165
   Lys Met Thr Leu Gly Gln Asp Leu Lys Ser Asp Ile Ser Leu Asn Ile
       965                 970                 975
   gat aaa ata cca gaa aaa aat aat gat tac atg gac aaa tgg gca gga      3213
   Asp Lys Ile Pro Glu Lys Asn Asn Asp Tyr Met Asp Lys Trp Ala Gly
   980                 985                 990                 995
   ctc tta ggt cca att  tca aat cac agt ttt  gga ggt agc ttc aga        3258
   Leu Leu Gly Pro Ile  Ser Asn His Ser Phe  Gly Gly Ser Phe Arg
                   1000                 1005                 1010
   aca gct tca aat aag  gaa atc aag ctc tct  gaa cat aac att aag        3303
   Thr Ala Ser Asn Lys  Glu Ile Lys Leu Ser  Glu His Asn Ile Lys
                   1015                 1020                 1025
   aag agc aaa atg ttc  ttc aaa gat att gaa  gaa caa tat cct act        3348
   Lys Ser Lys Met Phe  Phe Lys Asp Ile Glu  Glu Gln Tyr Pro Thr
                   1030                 1035                 1040
   agt tta gct tgt gtt  gaa att gta aat acc  ttg gca tta gat aat        3393
   Ser Leu Ala Cys Val  Glu Ile Val Asn Thr  Leu Ala Leu Asp Asn
                   1045                 1050                 1055
   caa aag aaa ctg agc  aag cct cag tca att  aat act gta tct gca      3438
   Gln Lys Lys Leu Ser  Lys Pro Gln Ser Ile  Asn Thr Val Ser Ala
                   1060                 1065                 1070
   cat tta cag agt agt  gta gtt gtt tct gat  tgt aaa aat agt cat      3483
   His Leu Gln Ser Ser  Val Val Val Ser Asp  Cys Lys Asn Ser His
                   1075                 1080                 1085
   ata acc cct cag atg  tta ttt tcc aag cag  gat ttt aat tca aac      3528
   Ile Thr Pro Gln Met  Leu Phe Ser Lys Gln  Asp Phe Asn Ser Asn
                   1090                 1095                 1100
   cat aat tta aca cct  agc caa aag gca gaa  att aca gaa ctt tct      3573
   His Asn Leu Thr Pro  Ser Gln Lys Ala Glu  Ile Thr Glu Leu Ser
                   1105                 1110                 1115
   act ata tta gaa gaa  tca gga agt cag ttt  gaa ttt act cag ttt      3618
   Thr Ile Leu Glu Glu  Ser Gly Ser Gln Phe  Glu Phe Thr Gln Phe
                   1120                 1125                 1130
   aga aaa cca agc tac  ata ttg cag aag agt  aca ttt gaa gtg cct      3663
   Arg Lys Pro Ser Tyr  Ile Leu Gln Lys Ser  Thr Phe Glu Val Pro
                   1135                 1140                 1145
   gaa aac cag atg act  atc tta aag acc act  tct gag gaa tgc aga      3708
   Glu Asn Gln Met Thr  Ile Leu Lys Thr Thr  Ser Glu Glu Cys Arg
                   1150                 1155                 1160
   gat gct gat ctt cat  gtc ata atg aat gcc  cca tcg att ggt cag      3753
   Asp Ala Asp Leu His  Val Ile Met Asn Ala  Pro Ser Ile Gly Gln
                   1165                 1170                 1175
   gta gac agc agc aag  caa ttt gaa ggt aca  gtt gaa att aaa cgg      3798
   Val Asp Ser Ser Lys  Gln Phe Glu Gly Thr  Val Glu Ile Lys Arg
                   1180                 1185                 1190
   aag ttt gct ggc ctg  ttg aaa aat gac tgt  aac aaa agt gct tct      3843
   Lys Phe Ala Gly Leu  Leu Lys Asn Asp Cys  Asn Lys Ser Ala Ser
                   1195                 1200                 1205
   ggt tat tta aca gat  gaa aat gaa gtg ggg  ttt agg ggc ttt tat      3888
   Gly Tyr Leu Thr Asp  Glu Asn Glu Val Gly  Phe Arg Gly Phe Tyr
                   1210                 1215                 1220
   tct gct cat ggc aca  aaa ctg aat gtt tct  act gaa gct ctg caa      3933
   Ser Ala His Gly Thr  Lys Leu Asn Val Ser  Thr Glu Ala Leu Gln
                   1225                 1230                 1235
   aaa gct gtg aaa ctg  ttt agt gat att gag  aat att agt gag gaa      3978
   Lys Ala Val Lys Leu  Phe Ser Asp Ile Glu  Asn Ile Ser Glu Glu
                   1240                 1245                 1250
   act tct gca gag gta  cat cca ata agt tta  tct tca agt aaa tgt      4023
   Thr Ser Ala Glu Val  His Pro Ile Ser Leu  Ser Ser Ser Lys Cys
                   1255                 1260                 1265
   cat gat tct gtt gtt  tca atg ttt aag ata  gaa aat cat aat gat      4068
   His Asp Ser Val Val  Ser Met Phe Lys Ile  Glu Asn His Asn Asp
                   1270                 1275                 1280
   aaa act gta agt gaa  aaa aat aat aaa tgc  caa ctg ata tta caa      4113
   Lys Thr Val Ser Glu  Lys Asn Asn Lys Cys  Gln Leu Ile Leu Gln
                   1285                 1290                 1295
   aat aat att gaa atg  act act ggc act ttt  gtt gaa gaa att act      4158
   Asn Asn Ile Glu Met  Thr Thr Gly Thr Phe  Val Glu Glu Ile Thr
                       1300                      1305                      1310
   gaa aat tac aag aga  aat act gaa aat gaa  gat aac aaa tat act          4203
   Glu Asn Tyr Lys Arg  Asn Thr Glu Asn Glu  Asp Asn Lys Tyr Thr
                   1315                 1320                 1325
   gct gcc agt aga aat  tct cat aac tta gaa  ttt gat ggc agt gat          4248
   Ala Ala Ser Arg Asn  Ser His Asn Leu Glu  Phe Asp Gly Ser Asp
                   1330                 1335                 1340
   tca agt aaa aat gat  act gtt tgt att cat  aaa gat gaa acg gac          4293
   Ser Ser Lys Asn Asp  Thr Val Cys Ile His  Lys Asp Glu Thr Asp
                   1345                 1350                 1355
   ttg cta ttt act gat  cag cac aac ata tgt  ctt aaa tta tct ggc          4338
   Leu Leu Phe Thr Asp  Gln His Asn Ile Cys  Leu Lys Leu Ser Gly
                   1360                 1365                 1370
   cag ttt atg aag gag  gga aac act cag att  aaa gaa gat ttg tca          4383
   Gln Phe Met Lys Glu  Gly Asn Thr Gln Ile  Lys Glu Asp Leu Ser
                   1375                 1380                 1385
   gat tta act ttt ttg  gaa gtt gcg aaa gct  caa gaa gca tgt cat          4428
   Asp Leu Thr Phe Leu  Glu Val Ala Lys Ala  Gln Glu Ala Cys His
                   1390                 1395                 1400
   ggt aat act tca aat  aaa gaa cag tta act  gct act aaa acg gag          4473
   Gly Asn Thr Ser Asn  Lys Glu Gln Leu Thr  Ala Thr Lys Thr Glu
                   1405                 1410                 1415
   caa aat ata aaa gat  ttt gag act tct gat  aca ttt ttt cag act          4518
   Gln Asn Ile Lys Asp  Phe Glu Thr Ser Asp  Thr Phe Phe Gln Thr
                   1420                 1425                 1430
   gca agt ggg aaa aat  att agt gtc gcc aaa  gag tca ttt aat aaa          4563
   Ala Ser Gly Lys Asn  Ile Ser Val Ala Lys  Glu Ser Phe Asn Lys
                   1435                 1440                 1445
   att gta aat ttc ttt  gat cag aaa cca gaa  gaa ttg cat aac ttt          4608
   Ile Val Asn Phe Phe  Asp Gln Lys Pro Glu  Glu Leu His Asn Phe
                   1450                 1455                 1460
   tcc tta aat tct gaa  tta cat tct gac ata  aga aag aac aaa atg          4653
   Ser Leu Asn Ser Glu  Leu His Ser Asp Ile  Arg Lys Asn Lys Met
                   1465                 1470                 1475
   gac att cta agt tat  gag gaa aca gac ata  gtt aaa cac aaa ata          4698
   Asp Ile Leu Ser Tyr  Glu Glu Thr Asp Ile  Val Lys His Lys Ile
                   1480                 1485                 1490
   ctg aaa gaa agt gtc  cca gtt ggt act gga  aat caa cta gtg acc          4743
   Leu Lys Glu Ser Val  Pro Val Gly Thr Gly  Asn Gln Leu Val Thr
                   1495                 1500                 1505
   ttc cag gga caa ccc  gaa cgt gat gaa aag  atc aaa gaa cct act          4788
   Phe Gln Gly Gln Pro  Glu Arg Asp Glu Lys  Ile Lys Glu Pro Thr
                   1510                 1515                 1520
   ctg ttg ggt ttt cat  aca gct agc ggg aaa  aaa gtt aaa att gca          4833
   Leu Leu Gly Phe His  Thr Ala Ser Gly Lys  Lys Val Lys Ile Ala
                   1525                 1530                 1535
   aag gaa tct ttg gac  aaa gtg aaa aac ctt  ttt gat gaa aaa gag          4878
   Lys Glu Ser Leu Asp  Lys Val Lys Asn Leu  Phe Asp Glu Lys Glu
                   1540                 1545                 1550
   caa ggt act agt gaa  atc acc agt ttt agc  cat caa tgg gca aag          4923
   Gln Gly Thr Ser Glu  Ile Thr Ser Phe Ser  His Gln Trp Ala Lys
                   1555                 1560                 1565
   acc cta aag tac aga  gag gcc tgt aaa gac  ctt gaa tta gca tgt      4968
   Thr Leu Lys Tyr Arg  Glu Ala Cys Lys Asp  Leu Glu Leu Ala Cys
                   1570                 1575                 1580
   gag acc att gag atc  aca gct gcc cca aag  tgt aaa gaa atg cag      5013
   Glu Thr Ile Glu Ile  Thr Ala Ala Pro Lys  Cys Lys Glu Met Gln
                   1585                 1590                 1595
   aat tct ctc aat aat  gat aaa aac ctt gtt  tct att gag act gtg      5058
   Asn Ser Leu Asn Asn  Asp Lys Asn Leu Val  Ser Ile Glu Thr Val
                   1600                 1605                 1610
   gtg cca cct aag ctc  tta agt gat aat tta  tgt aga caa act gaa      5103
   Val Pro Pro Lys Leu  Leu Ser Asp Asn Leu  Cys Arg Gln Thr Glu
                   1615                 1620                 1625
   aat ctc aaa aca tca  aaa agt atc ttt ttg  aaa gtt aaa gta cat      5148
   Asn Leu Lys Thr Ser  Lys Ser Ile Phe Leu  Lys Val Lys Val His
                   1630                 1635                 1640
   gaa aat gta gaa aaa  gaa aca gca aaa agt  cct gca act tgt tac      5193
   Glu Asn Val Glu Lys  Glu Thr Ala Lys Ser  Pro Ala Thr Cys Tyr
                   1645                 1650                 1655
   aca aat cag tcc cct  tat tca gtc att gaa  aat tca gcc tta gct      5238
   Thr Asn Gln Ser Pro  Tyr Ser Val Ile Glu  Asn Ser Ala Leu Ala
                   1660                 1665                 1670
   ttt tac aca agt tgt  agt aga aaa act tct  gtg agt cag act tca      5283
   Phe Tyr Thr Ser Cys  Ser Arg Lys Thr Ser  Val Ser Gln Thr Ser
                   1675                 1680                 1685
   tta ctt gaa gca aaa  aaa tgg ctt aga gaa  gga ata ttt gat ggt      5328
   Leu Leu Glu Ala Lys  Lys Trp Leu Arg Glu  Gly Ile Phe Asp Gly
                   1690                 1695                 1700
   caa cca gaa aga ata  aat act gca gat tat  gta gga aat tat ttg      5373
   Gln Pro Glu Arg Ile  Asn Thr Ala Asp Tyr  Val Gly Asn Tyr Leu
                   1705                 1710                 1715
   tat gaa aat aat tca  aac agt act ata gct  gaa aat gac aaa aat      5418
   Tyr Glu Asn Asn Ser  Asn Ser Thr Ile Ala  Glu Asn Asp Lys Asn
                   1720                 1725                 1730
   cat ctc tcc gaa aaa  caa gat act tat tta  agt aac agt agc atg      5463
   His Leu Ser Glu Lys  Gln Asp Thr Tyr Leu  Ser Asn Ser Ser Met
                   1735                 1740                 1745
   tct aac agc tat tcc  tac cat tct gat gag  gta tat aat gat tca      5508
   Ser Asn Ser Tyr Ser  Tyr His Ser Asp Glu  Val Tyr Asn Asp Ser
                   1750                 1755                 1760
   gga tat ctc tca aaa  aat aaa ctt gat tct  ggt att gag cca gta      5553
   Gly Tyr Leu Ser Lys  Asn Lys Leu Asp Ser  Gly Ile Glu Pro Val
                   1765                 1770                 1775
   ttg aag aat gtt gaa  gat caa aaa aac act  agt ttt tcc aaa gta      5598
   Leu Lys Asn Val Glu  Asp Gln Lys Asn Thr  Ser Phe Ser Lys Val
                   1780                 1785                 1790
   ata tcc aat gta aaa  gat gca aat gca tac  cca caa act gta aat      5643
   Ile Ser Asn Val Lys  Asp Ala Asn Ala Tyr  Pro Gln Thr Val Asn
                   1795                 1800                 1805
   gaa gat att tgc gtt  gag gaa ctt gtg act  agc tct tca ccc tgc      5688
   Glu Asp Ile Cys Val  Glu Glu Leu Val Thr  Ser Ser Ser Pro Cys
                   1810                 1815                 1820
   aaa aat aaa aat gca  gcc att aaa ttg tcc  ata tct aat agt aat        5733
   Lys Asn Lys Asn Ala  Ala Ile Lys Leu Ser  Ile Ser Asn Ser Asn
                   1825                 1830                 1835
   aat ttt gag gta ggg  cca cct gca ttt agg  ata gcc agt ggt aaa        5778
   Asn Phe Glu Val Gly  Pro Pro Ala Phe Arg  Ile Ala Ser Gly Lys
                   1840                 1845                 1850
   atc gtt tgt gtt tca  cat gaa aca att aaa  aaa gtg aaa gac ata        5823
   Ile Val Cys Val Ser  His Glu Thr Ile Lys  Lys Val Lys Asp Ile
                   1855                 1860                 1865
   ttt aca gac agt ttc  agt aaa gta att aag  gaa aac aac gag aat        5868
   Phe Thr Asp Ser Phe  Ser Lys Val Ile Lys  Glu Asn Asn Glu Asn
                   1870                 1875                 1880
   aaa tca aaa att tgc  caa acg aaa att atg  gca ggt tgt tac gag        5913
   Lys Ser Lys Ile Cys  Gln Thr Lys Ile Met  Ala Gly Cys Tyr Glu
                   1885                 1890                 1895
   gca ttg gat gat tca  gag gat att ctt cat  aac tct cta gat aat        5958
   Ala Leu Asp Asp Ser  Glu Asp Ile Leu His  Asn Ser Leu Asp Asn
                   1900                 1905                 1910
   gat gaa tgt agc acg  cat tca cat aag gtt  ttt gct gac att cag        6003
   Asp Glu Cys Ser Thr  His Ser His Lys Val  Phe Ala Asp Ile Gln
                   1915                 1920                 1925
   agt gaa gaa att tta  caa cat aac caa aat  atg tct gga ttg gag        6048
   Ser Glu Glu Ile Leu  Gln His Asn Gln Asn  Met Ser Gly Leu Glu
                   1930                 1935                 1940
   aaa gtt tct aaa ata  tca cct tgt gat gtt  agt ttg gaa act tca        6093
   Lys Val Ser Lys Ile  Ser Pro Cys Asp Val  Ser Leu Glu Thr Ser
                   1945                 1950                 1955
   gat ata tgt aaa tgt  agt ata ggg aag ctt  cat aag tca gtc tca        6138
   Asp Ile Cys Lys Cys  Ser Ile Gly Lys Leu  His Lys Ser Val Ser
                   1960                 1965                 1970
   tct gca aat act tgt  ggg att ttt agc aca  gca agt gga aaa tct        6183
   Ser Ala Asn Thr Cys  Gly Ile Phe Ser Thr  Ala Ser Gly Lys Ser
                   1975                 1980                 1985
   gtc cag gta tca gat  gct tca tta caa aac  gca aga caa gtg ttt        6228
   Val Gln Val Ser Asp  Ala Ser Leu Gln Asn  Ala Arg Gln Val Phe
                   1990                 1995                 2000
   tct gaa ata gaa gat  agt acc aag caa gtc  ttt tcc aaa gta ttg        6273
   Ser Glu Ile Glu Asp  Ser Thr Lys Gln Val  Phe Ser Lys Val Leu
                   2005                 2010                 2015
   ttt aaa agt aac gaa  cat tca gac cag ctc  aca aga gaa gaa aat        6318
   Phe Lys Ser Asn Glu  His Ser Asp Gln Leu  Thr Arg Glu Glu Asn
                   2020                 2025                 2030
   act gct ata cgt act  cca gaa cat tta ata  tcc caa aaa ggc ttt        6363
   Thr Ala Ile Arg Thr  Pro Glu His Leu Ile  Ser Gln Lys Gly Phe
                   2035                 2040                 2045
   tca tat aat gtg gta  aat tca tct gct ttc  tct gga ttt agt aca        6408
   Ser Tyr Asn Val Val  Asn Ser Ser Ala Phe  Ser Gly Phe Ser Thr
                   2050                 2055                 2060
   gca agt gga aag caa  gtt tcc att tta gaa  agt tcc tta cac aaa        6453
   Ala Ser Gly Lys Gln  Val Ser Ile Leu Glu  Ser Ser Leu His Lys
                   2065                 2070                 2075
   gtt aag gga gtg tta  gag gaa ttt gat tta  atc aga act gag cat        6498
   Val Lys Gly Val Leu Glu Glu Phe Asp Leu Ile Arg Thr Glu His
                   2080                2085                2090
   agt ctt cac tat tca cct acg tct aga caa aat gta tca aaa ata     6543
   Ser Leu His Tyr Ser Pro Thr Ser Arg Gln Asn Val Ser Lys Ile
                   2095                2100                2105
   ctt cct cgt gtt gat aag aga aac cca gag cac tgt gta aac tca     6588
   Leu Pro Arg Val Asp Lys Arg Asn Pro Glu His Cys Val Asn Ser
                   2110                2115                2120
   gaa atg gaa aaa acc tgc agt aaa gaa ttt aaa tta tca aat aac     6633
   Glu Met Glu Lys Thr Cys Ser Lys Glu Phe Lys Leu Ser Asn Asn
                   2125                2130                2135
   tta aat gtt gaa ggt ggt tct tca gaa aat aat cac tct att aaa     6678
   Leu Asn Val Glu Gly Gly Ser Ser Glu Asn Asn His Ser Ile Lys
                   2140                2145                2150
   gtt tct cca tat ctc tct caa ttt caa caa gac aaa caa cag ttg     6723
   Val Ser Pro Tyr Leu Ser Gln Phe Gln Gln Asp Lys Gln Gln Leu
                   2155                2160                2165
   gta tta gga acc aaa gtc tca ctt gtt gag aac att cat gtt ttg     6768
   Val Leu Gly Thr Lys Val Ser Leu Val Glu Asn Ile His Val Leu
                   2170                2175                2180
   gga aaa gaa cag gct tca cct aaa aac gta aaa atg gaa att ggt     6813
   Gly Lys Glu Gln Ala Ser Pro Lys Asn Val Lys Met Glu Ile Gly
                   2185                2190                2195
   aaa act gaa act ttt tct gat gtt cct gtg aaa aca aat ata gaa     6858
   Lys Thr Glu Thr Phe Ser Asp Val Pro Val Lys Thr Asn Ile Glu
                   2200                2205                2210
   gtt tgt tct act tac tcc aaa gat tca gaa aac tac ttt gaa aca     6903
   Val Cys Ser Thr Tyr Ser Lys Asp Ser Glu Asn Tyr Phe Glu Thr
                   2215                2220                2225
   gaa gca gta gaa att gct aaa gct ttt atg gaa gat gat gaa ctg     6948
   Glu Ala Val Glu Ile Ala Lys Ala Phe Met Glu Asp Asp Glu Leu
                   2230                2235                2240
   aca gat tct aaa ctg cca agt cat gcc aca cat tct ctt ttt aca     6993
   Thr Asp Ser Lys Leu Pro Ser His Ala Thr His Ser Leu Phe Thr
                   2245                2250                2255
   tgt ccc gaa aat gag gaa atg gtt ttg tca aat tca aga att gga     7038
   Cys Pro Glu Asn Glu Glu Met Val Leu Ser Asn Ser Arg Ile Gly
                   2260                2265                2270
   aaa aga aga gga gag ccc ctt atc tta gtg gga gaa ccc tca atc     7083
   Lys Arg Arg Gly Glu Pro Leu Ile Leu Val Gly Glu Pro Ser Ile
                   2275                2280                2285
   aaa aga aac tta tta aat gaa ttt gac agg ata ata gaa aat caa     7128
   Lys Arg Asn Leu Leu Asn Glu Phe Asp Arg Ile Ile Glu Asn Gln
                   2290                2295                2300
   gaa aaa tcc tta aag gct tca aaa agc act cca gat ggc aca ata     7173
   Glu Lys Ser Leu Lys Ala Ser Lys Ser Thr Pro Asp Gly Thr Ile
                   2305                2310                2315
   aaa gat cga aga ttg ttt atg cat cat gtt tct tta gag ccg att     7218
   Lys Asp Arg Arg Leu Phe Met His His Val Ser Leu Glu Pro Ile
                   2320                2325                2330
   acc tgt gta ccc ttt cgc aca act aag gaa cgt caa gag ata cag     7263
   Thr Cys Val Pro Phe Arg Thr Thr Lys Glu Arg Gln Glu Ile Gln
                       2335                2340                2345
   aat cca aat ttt acc  gca cct ggt caa gaa  ttt ctg tct aaa tct        7308
   Asn Pro Asn Phe Thr  Ala Pro Gly Gln Glu  Phe Leu Ser Lys Ser
                   2350                 2355                 2360
   cat ttg tat gaa cat  ctg act ttg gaa aaa  tct tca agc aat tta        7353
   His Leu Tyr Glu His  Leu Thr Leu Glu Lys  Ser Ser Ser Asn Leu
                   2365                 2370                 2375
   gca gtt tca gga cat  cca ttt tat caa gtt  tct gct aca aga aat        7398
   Ala Val Ser Gly His  Pro Phe Tyr Gln Val  Ser Ala Thr Arg Asn
                   2380                 2385                 2390
   gaa aaa atg aga cac  ttg att act aca ggc  aga cca acc aaa gtc        7443
   Glu Lys Met Arg His  Leu Ile Thr Thr Gly  Arg Pro Thr Lys Val
                   2395                 2400                 2405
   ttt gtt cca cct ttt  aaa act aaa tca cat  ttt cac aga gtt gaa        7488
   Phe Val Pro Pro Phe  Lys Thr Lys Ser His  Phe His Arg Val Glu
                   2410                 2415                 2420
   cag tgt gtt agg aat  att aac ttg gag gaa  aac aga caa aag caa        7533
   Gln Cys Val Arg Asn  Ile Asn Leu Glu Glu  Asn Arg Gln Lys Gln
                   2425                 2430                 2435
   aac att gat gga cat  ggc tct gat gat agt  aaa aat aag att aat        7578
   Asn Ile Asp Gly His  Gly Ser Asp Asp Ser  Lys Asn Lys Ile Asn
                   2440                 2445                 2450
   gac aat gag att cat  cag ttt aac aaa aac  aac tcc aat caa gca        7623
   Asp Asn Glu Ile His  Gln Phe Asn Lys Asn  Asn Ser Asn Gln Ala
                   2455                 2460                 2465
   gca gct gta act ttc  aca aag tgt gaa gaa  gaa cct tta gat tta        7668
   Ala Ala Val Thr Phe  Thr Lys Cys Glu Glu  Glu Pro Leu Asp Leu
                   2470                 2475                 2480
   att aca agt ctt cag  aat gcc aga gat ata  cag gat atg cga att        7713
   Ile Thr Ser Leu Gln  Asn Ala Arg Asp Ile  Gln Asp Met Arg Ile
                   2485                 2490                 2495
   aag aag aaa caa agg  caa cgc gtc ttt cca  cag cca ggc agt ctg        7758
   Lys Lys Lys Gln Arg  Gln Arg Val Phe Pro  Gln Pro Gly Ser Leu
                   2500                 2505                 2510
   tat ctt gca aaa aca  tcc act ctg cct cga  atc tct ctg aaa gca        7803
   Tyr Leu Ala Lys Thr  Ser Thr Leu Pro Arg  Ile Ser Leu Lys Ala
                   2515                 2520                 2525
   gca gta gga ggc caa  gtt ccc tct gcg tgt  tct cat aaa cag ctg        7848
   Ala Val Gly Gly Gln  Val Pro Ser Ala Cys  Ser His Lys Gln Leu
                   2530                 2535                 2540
   tat acg tat ggc gtt  tct aaa cat tgc ata  aaa att aac agc aaa        7893
   Tyr Thr Tyr Gly Val  Ser Lys His Cys Ile  Lys Ile Asn Ser Lys
                   2545                 2550                 2555
   aat gca gag tct ttt  cag ttt cac act gaa  gat tat ttt ggt aag        7938
   Asn Ala Glu Ser Phe  Gln Phe His Thr Glu  Asp Tyr Phe Gly Lys
                   2560                 2565                 2570
   gaa agt tta tgg act  gga aaa gga ata cag  ttg gct gat ggt gga        7983
   Glu Ser Leu Trp Thr  Gly Lys Gly Ile Gln  Leu Ala Asp Gly Gly
                   2575                 2580                 2585
   tgg ctc ata ccc tcc  aat gat gga aag gct  gga aaa gaa gaa ttt        8028
   Trp Leu Ile Pro Ser  Asn Asp Gly Lys Ala  Gly Lys Glu Glu Phe
                   2590                 2595                 2600
   tat agg gct ctg tgt  gac act cca ggt gtg  gat cca aag ctt att        8073
   Tyr Arg Ala Leu Cys  Asp Thr Pro Gly Val  Asp Pro Lys Leu Ile
                   2605                 2610                 2615
   tct aga att tgg gtt  tat aat cac tat aga  tgg atc ata tgg aaa        8118
   Ser Arg Ile Trp Val  Tyr Asn His Tyr Arg  Trp Ile Ile Trp Lys
                   2620                 2625                 2630
   ctg gca gct atg gaa  tgt gcc ttt cct aag  gaa ttt gct aat aga        8163
   Leu Ala Ala Met Glu  Cys Ala Phe Pro Lys  Glu Phe Ala Asn Arg
                   2635                 2640                 2645
   tgc cta agc cca gaa  agg gtg ctt ctt caa  cta aaa tac aga tat        8208
   Cys Leu Ser Pro Glu  Arg Val Leu Leu Gln  Leu Lys Tyr Arg Tyr
                   2650                 2655                 2660
   gat acg gaa att gat  aga agc aga aga tcg  gct ata aaa aag ata        8253
   Asp Thr Glu Ile Asp  Arg Ser Arg Arg Ser  Ala Ile Lys Lys Ile
                   2665                 2670                 2675
   atg gaa agg gat gac  aca gct gca aaa aca  ctt gtt ctc tgt gtt        8298
   Met Glu Arg Asp Asp  Thr Ala Ala Lys Thr  Leu Val Leu Cys Val
                   2680                 2685                 2690
   tct gac ata att tca  ttg agc gca aat ata  tct gaa act tct agc        8343
   Ser Asp Ile Ile Ser  Leu Ser Ala Asn Ile  Ser Glu Thr Ser Ser
                   2695                 2700                 2705
   aat aaa act agt agt  gca gat acc caa aaa  gtg gcc att att gaa        8388
   Asn Lys Thr Ser Ser  Ala Asp Thr Gln Lys  Val Ala Ile Ile Glu
                   2710                 2715                 2720
   ctt aca gat ggg tgg  tat gct gtt aag gcc  cag tta gat cct ccc        8433
   Leu Thr Asp Gly Trp  Tyr Ala Val Lys Ala  Gln Leu Asp Pro Pro
                   2725                 2730                 2735
   ctc tta gct gtc tta  aag aat ggc aga ctg  aca gtt ggt cag aag        8478
   Leu Leu Ala Val Leu  Lys Asn Gly Arg Leu  Thr Val Gly Gln Lys
                   2740                 2745                 2750
   att att ctt cat gga  gca gaa ctg gtg ggc  tct cct gat gcc tgt        8523
   Ile Ile Leu His Gly  Ala Glu Leu Val Gly  Ser Pro Asp Ala Cys
                   2755                 2760                 2765
   aca cct ctt gaa gcc  cca gaa tct ctt atg  tta aag att tct gct        8568
   Thr Pro Leu Glu Ala  Pro Glu Ser Leu Met  Leu Lys Ile Ser Ala
                   2770                 2775                 2780
   aac agt act cgg cct  gct cgc tgg tat acc  aaa ctt gga ttc ttt        8613
   Asn Ser Thr Arg Pro  Ala Arg Trp Tyr Thr  Lys Leu Gly Phe Phe
                   2785                 2790                 2795
   cct gac cct aga cct  ttt cct ctg ccc tta  tca tcg ctt ttc agt        8658
   Pro Asp Pro Arg Pro  Phe Pro Leu Pro Leu  Ser Ser Leu Phe Ser
                   2800                 2805                 2810
   gat gga gga aat gtt  ggt tgt gtt gat gta  att att caa aga gca        8703
   Asp Gly Gly Asn Val  Gly Cys Val Asp Val  Ile Ile Gln Arg Ala
                   2815                 2820                 2825
   tac cct ata cag tgg  atg gag aag aca tca  tct gga tta tac ata        8748
   Tyr Pro Ile Gln Trp  Met Glu Lys Thr Ser  Ser Gly Leu Tyr Ile
                   2830                 2835                 2840
   ttt cgc aat gaa aga  gag gaa gaa aag gaa  gca gca aaa tat gtg        8793
   Phe Arg Asn Glu Arg  Glu Glu Glu Lys Glu  Ala Ala Lys Tyr Val
                   2845                 2850                 2855
   gag gcc caa caa aag  aga cta gaa gcc tta  ttc act aaa att cag        8838
   Glu Ala Gln Gln Lys  Arg Leu Glu Ala Leu  Phe Thr Lys Ile Gln
                   2860                 2865                 2870
   gag gaa ttt gaa gaa  cat gaa gaa aac aca  aca aaa cca tat tta        8883
   Glu Glu Phe Glu Glu  His Glu Glu Asn Thr  Thr Lys Pro Tyr Leu
                   2875                 2880                 2885
   cca tca cgt gca cta  aca aga cag caa gtt  cgt gct ttg caa gat        8928
   Pro Ser Arg Ala Leu  Thr Arg Gln Gln Val  Arg Ala Leu Gln Asp
                   2890                 2895                 2900
   ggt gca gag ctt tat  gaa gca gtg aag aat  gca gca gac cca gct        8973
   Gly Ala Glu Leu Tyr  Glu Ala Val Lys Asn  Ala Ala Asp Pro Ala
                   2905                 2910                 2915
   tac ctt gag ggt tat  ttc agt gaa gag cag  tta aga gcc ttg aat        9018
   Tyr Leu Glu Gly Tyr  Phe Ser Glu Glu Gln  Leu Arg Ala Leu Asn
                   2920                 2925                 2930
   aat cac agg caa atg  ttg aat gat aag aaa  caa gct cag atc cag        9063
   Asn His Arg Gln Met  Leu Asn Asp Lys Lys  Gln Ala Gln Ile Gln
                   2935                 2940                 2945
   ttg gaa att agg aag  acc atg gaa tct gct  gaa caa aag gaa caa        9108
   Leu Glu Ile Arg Lys  Thr Met Glu Ser Ala  Glu Gln Lys Glu Gln
                   2950                 2955                 2960
   ggt tta tca agg gat  gtc aca acc gtg tgg  aag ttg cgt att gta        9153
   Gly Leu Ser Arg Asp  Val Thr Thr Val Trp  Lys Leu Arg Ile Val
                   2965                 2970                 2975
   agc tat tca aaa aaa  gaa aaa gat tca gtt  ata ctg agt att tgg        9198
   Ser Tyr Ser Lys Lys  Glu Lys Asp Ser Val  Ile Leu Ser Ile Trp
                   2980                 2985                 2990
   cgt cca tca tca gat  tta tat tct ctg tta  aca gaa gga aag aga        9243
   Arg Pro Ser Ser Asp  Leu Tyr Ser Leu Leu  Thr Glu Gly Lys Arg
                   2995                 3000                 3005
   tac aga att tat cat  ctt gca act tca aaa  tct aaa agt aaa tct        9288
   Tyr Arg Ile Tyr His  Leu Ala Thr Ser Lys  Ser Lys Ser Lys Ser
                   3010                 3015                 3020
   gaa aga gct aac ata  cag tta gca gcg aca  aaa aaa act cag tat        9333
   Glu Arg Ala Asn Ile  Gln Leu Ala Ala Thr  Lys Lys Thr Gln Tyr
                   3025                 3030                 3035
   caa caa cta ccg gtt  tca gat gaa att tta  ttt cag att tac cag        9378
   Gln Gln Leu Pro Val  Ser Asp Glu Ile Leu  Phe Gln Ile Tyr Gln
                   3040                 3045                 3050
   cca cgg gag ccc ctt  cac ttc agc aaa ttt  tta gat cca gac ttt        9423
   Pro Arg Glu Pro Leu  His Phe Ser Lys Phe  Leu Asp Pro Asp Phe
                   3055                 3060                 3065
   cag cca tct tgt tct  gag gtg gac cta ata  gga ttt gtc gtt tct        9468
   Gln Pro Ser Cys Ser  Glu Val Asp Leu Ile  Gly Phe Val Val Ser
                   3070                 3075                 3080
   gtt gtg aaa aaa aca  gga ctt gcc cct ttc  gtc tat ttg tca gac        9513
   Val Val Lys Lys Thr  Gly Leu Ala Pro Phe  Val Tyr Leu Ser Asp
                   3085                 3090                 3095
   gaa tgt tac aat tta  ctg gca ata aag ttt  tgg ata gac ctt aat        9558
   Glu Cys Tyr Asn Leu  Leu Ala Ile Lys Phe  Trp Ile Asp Leu Asn
                   3100                 3105                 3110
   gag gac att att aag  cct cat atg tta att  gct gca agc aac ctc        9603
   Glu Asp Ile Ile Lys  Pro His Met Leu Ile  Ala Ala Ser Asn Leu
                   3115                 3120                 3125
   cag tgg cga cca gaa  tcc aaa tca ggc ctt  ctt act tta ttt gct      9648
   Gln Trp Arg Pro Glu  Ser Lys Ser Gly Leu  Leu Thr Leu Phe Ala
                   3130                 3135                 3140
   gga gat ttt tct gtg  ttt tct gct agt cca  aaa gag ggc cac ttt      9693
   Gly Asp Phe Ser Val  Phe Ser Ala Ser Pro  Lys Glu Gly His Phe
                   3145                 3150                 3155
   caa gag aca ttc aac  aaa atg aaa aat act  gtt gag aat att gac      9738
   Gln Glu Thr Phe Asn  Lys Met Lys Asn Thr  Val Glu Asn Ile Asp
                   3160                 3165                 3170
   ata ctt tgc aat gaa  gca gaa aac aag ctt  atg cat ata ctg cat      9783
   Ile Leu Cys Asn Glu  Ala Glu Asn Lys Leu  Met His Ile Leu His
                   3175                 3180                 3185
   gca aat gat ccc aag  tgg tcc acc cca act  aaa gac tgt act tca      9828
   Ala Asn Asp Pro Lys  Trp Ser Thr Pro Thr  Lys Asp Cys Thr Ser
                   3190                 3195                 3200
   ggg ccg tac act gct  caa atc att cct ggt  aca gga aac aag ctt      9873
   Gly Pro Tyr Thr Ala  Gln Ile Ile Pro Gly  Thr Gly Asn Lys Leu
                   3205                 3210                 3215
   ctg atg tct tct cct  aat tgt gag ata tat  tat caa agt cct tta      9918
   Leu Met Ser Ser Pro  Asn Cys Glu Ile Tyr  Tyr Gln Ser Pro Leu
                   3220                 3225                 3230
   tca ctt tgt atg gcc  aaa agg aag tct gtt  tcc aca cct gtc tca      9963
   Ser Leu Cys Met Ala  Lys Arg Lys Ser Val  Ser Thr Pro Val Ser
                   3235                 3240                 3245
   gcc cag atg act tca  aag tct tgt aaa ggg  gag aaa gag att gat     10008
   Ala Gln Met Thr Ser  Lys Ser Cys Lys Gly  Glu Lys Glu Ile Asp
                   3250                 3255                 3260
   gac caa aag aac tgc  aaa aag aga aga gcc  ttg gat ttc ttg agt     10053
   Asp Gln Lys Asn Cys  Lys Lys Arg Arg Ala  Leu Asp Phe Leu Ser
                   3265                 3270                 3275
   aga ctg cct tta cct  cca cct gtt agt ccc  att tgt aca ttt gtt     10098
   Arg Leu Pro Leu Pro  Pro Pro Val Ser Pro  Ile Cys Thr Phe Val
                   3280                 3285                 3290
   tct ccg gct gca cag  aag gca ttt cag cca  cca agg agt tgt ggc     10143
   Ser Pro Ala Ala Gln  Lys Ala Phe Gln Pro  Pro Arg Ser Cys Gly
                   3295                 3300                 3305
   acc aaa tac gaa aca  ccc ata aag aaa aaa  gaa ctg aat tct cct     10188
   Thr Lys Tyr Glu Thr  Pro Ile Lys Lys Lys  Glu Leu Asn Ser Pro
                   3310                 3315                 3320
   cag atg act cca ttt  aaa aaa ttc aat gaa  att tct ctt ttg gaa     10233
   Gln Met Thr Pro Phe  Lys Lys Phe Asn Glu  Ile Ser Leu Leu Glu
                   3325                 3330                 3335
   agt aat tca ata gct  gac gaa gaa ctt gca  ttg ata aat acc caa     10278
   Ser Asn Ser Ile Ala  Asp Glu Glu Leu Ala  Leu Ile Asn Thr Gln
                   3340                 3345                 3350
   gct ctt ttg tct ggt  tca aca gga gaa aaa  caa ttt ata tct gtc     10323
   Ala Leu Leu Ser Gly  Ser Thr Gly Glu Lys  Gln Phe Ile Ser Val
                   3355                 3360                 3365
   agt gaa tcc act agg  act gct ccc acc agt  tca gaa gat tat ctc     10368
   Ser Glu Ser Thr Arg  Thr Ala Pro Thr Ser  Ser Glu Asp Tyr Leu
                       3370                3375                3380
   aga ctg aaa cga cgt  tgt act aca tct ctg  atc aaa gaa cag gag        10413
   Arg Leu Lys Arg Arg  Cys Thr Thr Ser Leu  Ile Lys Glu Gln Glu
                   3385                 3390                 3395
   agt tcc cag gcc agt  acg gaa gaa tgt gag  aaa aat aag cag gac        10458
   Ser Ser Gln Ala Ser  Thr Glu Glu Cys Glu  Lys Asn Lys Gln Asp
                   3400                 3405                 3410
   aca att aca act aaa  aaa tat atc taa                                 10485
   Thr Ile Thr Thr Lys  Lys Tyr Ile
                   3415
<210> 13
   <211> 3418
   <212> PRT
   <213> Homo sapiens
<400> 13
   Met Pro Ile Gly Ser Lys Glu Arg Pro Thr Phe Phe Glu Ile Phe Lys
   1               5                   10                  15
   Thr Arg Cys Asn Lys Ala Asp Leu Gly Pro Ile Ser Leu Asn Trp Phe
               20                  25                  30
   Glu Glu Leu Ser Ser Glu Ala Pro Pro Tyr Asn Ser Glu Pro Ala Glu
           35                  40                  45
   Glu Ser Glu His Lys Asn Asn Asn Tyr Glu Pro Asn Leu Phe Lys Thr
       50                  55                  60
   Pro Gln Arg Lys Pro Ser Tyr Asn Gln Leu Ala Ser Thr Pro Ile Ile
   65                  70                  75                  80
   Phe Lys Glu Gln Gly Leu Thr Leu Pro Leu Tyr Gln Ser Pro Val Lys
                   85                  90                  95
   Glu Leu Asp Lys Phe Lys Leu Asp Leu Gly Arg Asn Val Pro Asn Ser
               100                 105                 110
   Arg His Lys Ser Leu Arg Thr Val Lys Thr Lys Met Asp Gln Ala Asp
           115                 120                 125
   Asp Val Ser Cys Pro Leu Leu Asn Ser Cys Leu Ser Glu Ser Pro Val
       130                 135                 140
   Val Leu Gln Cys Thr His Val Thr Pro Gln Arg Asp Lys Ser Val Val
   145                 150                 155                 160
   Cys Gly Ser Leu Phe His Thr Pro Lys Phe Val Lys Gly Arg Gln Thr
                   165                 170                 175
   Pro Lys His Ile Ser Glu Ser Leu Gly Ala Glu Val Asp Pro Asp Met
               180                 185                 190
   Ser Trp Ser Ser Ser Leu Ala Thr Pro Pro Thr Leu Ser Ser Thr Val
           195                 200                 205
   Leu Ile Val Arg Asn Glu Glu Ala Ser Glu Thr Val Phe Pro His Asp
       210                 215                 220
   Thr Thr Ala Asn Val Lys Ser Tyr Phe Ser Asn His Asp Glu Ser Leu
   225                 230                 235                 240
   Lys Lys Asn Asp Arg Phe Ile Ala Ser Val Thr Asp Ser Glu Asn Thr
                   245                 250                 255
   Asn Gln Arg Glu Ala Ala Ser His Gly Phe Gly Lys Thr Ser Gly Asn
               260                 265                 270
   Ser Phe Lys Val Asn Ser Cys Lys Asp His Ile Gly Lys Ser Met Pro
           275                 280                 285
   His Val Leu Glu Asp Glu Val Tyr Glu Thr Val Val Asp Thr Ser Glu
       290                 295                 300
   Glu Asp Ser Phe Ser Leu Cys Phe Ser Lys Cys Arg Thr Lys Asn Leu
   305                 310                 315                 320
   Gln Lys Val Arg Thr Ser Lys Thr Arg Lys Lys Ile Phe His Glu Ala
                   325                 330                 335
   Asn Ala Asp Glu Cys Glu Lys Ser Lys Asn Gln Val Lys Glu Lys Tyr
               340                 345                 350
   Ser Phe Val Ser Glu Val Glu Pro Asn Asp Thr Asp Pro Leu Asp Ser
           355                 360                 365
   Asn Val Ala His Gln Lys Pro Phe Glu Ser Gly Ser Asp Lys Ile Ser
       370                 375                 380
   Lys Glu Val Val Pro Ser Leu Ala Cys Glu Trp Ser Gln Leu Thr Leu
   385                 390                 395                 400
   Ser Gly Leu Asn Gly Ala Gln Met Glu Lys Ile Pro Leu Leu His Ile
                   405                 410                 415
   Ser Ser Cys Asp Gln Asn Ile Ser Glu Lys Asp Leu Leu Asp Thr Glu
               420                 425                 430
   Asn Lys Arg Lys Lys Asp Phe Leu Thr Ser Glu Asn Ser Leu Pro Arg
           435                 440                 445
   Ile Ser Ser Leu Pro Lys Ser Glu Lys Pro Leu Asn Glu Glu Thr Val
       450                 455                 460
   Val Asn Lys Arg Asp Glu Glu Gln His Leu Glu Ser His Thr Asp Cys
   465                 470                 475                 480
   Ile Leu Ala Val Lys Gln Ala Ile Ser Gly Thr Ser Pro Val Ala Ser
                   485                 490                 495
   Ser Phe Gln Gly Ile Lys Lys Ser Ile Phe Arg Ile Arg Glu Ser Pro
               500                 505                 510
   Lys Glu Thr Phe Asn Ala Ser Phe Ser Gly His Met Thr Asp Pro Asn
           515                 520                 525
   Phe Lys Lys Glu Thr Glu Ala Ser Glu Ser Gly Leu Glu Ile His Thr
       530                 535                 540
   Val Cys Ser Gln Lys Glu Asp Ser Leu Cys Pro Asn Leu Ile Asp Asn
   545                 550                 555                 560
   Gly Ser Trp Pro Ala Thr Thr Thr Gln Asn Ser Val Ala Leu Lys Asn
                   565                 570                 575
   Ala Gly Leu Ile Ser Thr Leu Lys Lys Lys Thr Asn Lys Phe Ile Tyr
               580                 585                 590
   Ala Ile His Asp Glu Thr Ser Tyr Lys Gly Lys Lys Ile Pro Lys Asp
           595                 600                 605
   Gln Lys Ser Glu Leu Ile Asn Cys Ser Ala Gln Phe Glu Ala Asn Ala
       610                 615                 620
   Phe Glu Ala Pro Leu Thr Phe Ala Asn Ala Asp Ser Gly Leu Leu His
   625                 630                 635                 640
   Ser Ser Val Lys Arg Ser Cys Ser Gln Asn Asp Ser Glu Glu Pro Thr
                   645                 650                 655
   Leu Ser Leu Thr Ser Ser Phe Gly Thr Ile Leu Arg Lys Cys Ser Arg
               660                 665                 670
   Asn Glu Thr Cys Ser Asn Asn Thr Val Ile Ser Gln Asp Leu Asp Tyr
           675                 680                 685
   Lys Glu Ala Lys Cys Asn Lys Glu Lys Leu Gln Leu Phe Ile Thr Pro
       690                 695                 700
   Glu Ala Asp Ser Leu Ser Cys Leu Gln Glu Gly Gln Cys Glu Asn Asp
   705                 710                 715                 720
   Pro Lys Ser Lys Lys Val Ser Asp Ile Lys Glu Glu Val Leu Ala Ala
                   725                 730                 735
   Ala Cys His Pro Val Gln His Ser Lys Val Glu Tyr Ser Asp Thr Asp
                   740                 745                 750
   Phe Gln Ser Gln Lys Ser Leu Leu Tyr Asp His Glu Asn Ala Ser Thr
           755                 760                 765
   Leu Ile Leu Thr Pro Thr Ser Lys Asp Val Leu Ser Asn Leu Val Met
       770                 775                 780
   Ile Ser Arg Gly Lys Glu Ser Tyr Lys Met Ser Asp Lys Leu Lys Gly
   785                 790                 795                 800
   Asn Asn Tyr Glu Ser Asp Val Glu Leu Thr Lys Asn Ile Pro Met Glu
                   805                 810                 815
   Lys Asn Gln Asp Val Cys Ala Leu Asn Glu Asn Tyr Lys Asn Val Glu
               820                 825                 830
   Leu Leu Pro Pro Glu Lys Tyr Met Arg Val Ala Ser Pro Ser Arg Lys
           835                 840                 845
   Val Gln Phe Asn Gln Asn Thr Asn Leu Arg Val Ile Gln Lys Asn Gln
       850                 855                 860
   Glu Glu Thr Thr Ser Ile Ser Lys Ile Thr Val Asn Pro Asp Ser Glu
   865                 870                 875                 880
   Glu Leu Phe Ser Asp Asn Glu Asn Asn Phe Val Phe Gln Ile Ala Asn
                   885                 890                 895
   Glu Arg Asn Asn Leu Ala Leu Gly Asn Thr Lys Glu Leu His Glu Thr
               900                 905                 910
   Asp Leu Thr Cys Val Asn Glu Pro Ile Phe Lys Asn Ser Thr Met Val
           915                 920                 925
   Leu Tyr Gly Asp Thr Gly Asp Lys Gln Ala Thr Gln Val Ser Ile Lys
       930                 935                 940
   Lys Asp Leu Val Tyr Val Leu Ala Glu Glu Asn Lys Asn Ser Val Lys
   945                 950                 955                 960
   Gln His Ile Lys Met Thr Leu Gly Gln Asp Leu Lys Ser Asp Ile Ser
                   965                 970                 975
   Leu Asn Ile Asp Lys Ile Pro Glu Lys Asn Asn Asp Tyr Met Asp Lys
               980                 985                 990
   Trp Ala Gly Leu Leu Gly Pro Ile  Ser Asn His Ser Phe  Gly Gly Ser
           995                 1000                1005
   Phe Arg  Thr Ala Ser Asn Lys  Glu Ile Lys Leu Ser  Glu His Asn
       1010                1015                1020
   Ile Lys Lys Ser Lys Met Phe Phe Lys Asp Ile Glu Glu Gln Tyr
   1025 1030 1035
   Pro Thr Ser Leu Ala Cys Val Glu Ile Val Asn Thr Leu Ala Leu
   1040 1045 1050
   Asp Asn Gln Lys Lys Leu Ser Lys Pro Gln Ser Ile Asn Thr Val
   1055 1060 1065
   Ser Ala His Leu Gln Ser Ser Val Val Val Ser Asp Cys Lys Asn
   1070 1075 1080
   Ser His Ile Thr Pro Gln Met Leu Phe Ser Lys Gln Asp Phe Asn
   1085 1090 1095
   Ser Asn His Asn Leu Thr Pro Ser Gln Lys Ala Glu Ile Thr Glu
   1100 1105 1110
   Leu Ser Thr Ile Leu Glu Glu Ser Gly Ser Gln Phe Glu Phe Thr
   1115 1120 1125
   Gln Phe Arg Lys Pro Ser Tyr Ile Leu Gln Lys Ser Thr Phe Glu
   1130 1135 1140
   Val Pro Glu Asn Gln Met Thr Ile Leu Lys Thr Thr Ser Glu Glu
   1145 1150 1155
   Cys Arg Asp Ala Asp Leu His Val Ile Met Asn Ala Pro Ser Ile
   1160 1165 1170
   Gly Gln Val Asp Ser Ser Lys Gln Phe Glu Gly Thr Val Glu Ile
   1175 1180 1185
   Lys Arg Lys Phe Ala Gly Leu Leu Lys Asn Asp Cys Asn Lys Ser
   1190 1195 1200
   Ala Ser Gly Tyr Leu Thr Asp Glu Asn Glu Val Gly Phe Arg Gly
   1205 1210 1215
   Phe Tyr Ser Ala His Gly Thr Lys Leu Asn Val Ser Thr Glu Ala
   1220 1225 1230
   Leu Gln Lys Ala Val Lys Leu Phe Ser Asp Ile Glu Asn Ile Ser
   1235 1240 1245
   Glu Glu Thr Ser Ala Glu Val His Pro Ile Ser Leu Ser Ser Ser
   1250 1255 1260
   Lys Cys His Asp Ser Val Val Ser Met Phe Lys Ile Glu Asn His
   1265 1270 1275
   Asn Asp Lys Thr Val Ser Glu Lys Asn Asn Lys Cys Gln Leu Ile
   1280 1285 1290
   Leu Gln Asn Asn Ile Glu Met Thr Thr Gly Thr Phe Val Glu Glu
   1295 1300 1305
   Ile Thr Glu Asn Tyr Lys Arg Asn Thr Glu Asn Glu Asp Asn Lys
   1310 1315 1320
   Tyr Thr Ala Ala Ser Arg Asn Ser His Asn Leu Glu Phe Asp Gly
   1325 1330 1335
   Ser Asp Ser Ser Lys Asn Asp Thr Val Cys Ile His Lys Asp Glu
   1340 1345 1350
   Thr Asp Leu Leu Phe Thr Asp Gln His Asn Ile Cys Leu Lys Leu
   1355 1360 1365
   Ser Gly Gln Phe Met Lys Glu Gly Asn Thr Gln Ile Lys Glu Asp
   1370 1375 1380
   Leu Ser Asp Leu Thr Phe Leu Glu Val Ala Lys Ala Gln Glu Ala
   1385 1390 1395
   Cys His Gly Asn Thr Ser Asn Lys Glu Gln Leu Thr Ala Thr Lys
   1400 1405 1410
   Thr Glu Gln Asn Ile Lys Asp Phe Glu Thr Ser Asp Thr Phe Phe
   1415 1420 1425
   Gln Thr Ala Ser Gly Lys Asn Ile Ser Val Ala Lys Glu Ser Phe
   1430 1435 1440
   Asn Lys Ile Val Asn Phe Phe Asp Gln Lys Pro Glu Glu Leu His
   1445 1450 1455
   Asn Phe Ser Leu Asn Ser Glu Leu His Ser Asp Ile Arg Lys Asn
   1460 1465 1470
   Lys Met Asp Ile Leu Ser Tyr Glu Glu Thr Asp Ile Val Lys His
   1475 1480 1485
   Lys Ile Leu Lys Glu Ser Val Pro Val Gly Thr Gly Asn Gln Leu
   1490 1495 1500
   Val Thr Phe Gln Gly Gln Pro Glu Arg Asp Glu Lys Ile Lys Glu
   1505 1510 1515
   Pro Thr Leu Leu Gly Phe His Thr Ala Ser Gly Lys Lys Val Lys
   1520 1525 1530
   Ile Ala  Lys Glu Ser Leu Asp  Lys Val Lys Asn Leu  Phe Asp Glu
       1535                 1540                 1545
   Lys Glu  Gln Gly Thr Ser Glu  Ile Thr Ser Phe Ser  His Gln Trp
       1550                 1555                 1560
   Ala Lys  Thr Leu Lys Tyr Arg  Glu Ala Cys Lys Asp  Leu Glu Leu
       1565                 1570                 1575
   Ala Cys  Glu Thr Ile Glu Ile  Thr Ala Ala Pro Lys  Cys Lys Glu
       1580                 1585                 1590
   Met Gln  Asn Ser Leu Asn Asn  Asp Lys Asn Leu Val  Ser Ile Glu
       1595                 1600                 1605
   Thr Val  Val Pro Pro Lys Leu  Leu Ser Asp Asn Leu  Cys Arg Gln
       1610                 1615                 1620
   Thr Glu  Asn Leu Lys Thr Ser  Lys Ser Ile Phe Leu  Lys Val Lys
       1625                 1630                 1635
   Val His  Glu Asn Val Glu Lys  Glu Thr Ala Lys Ser  Pro Ala Thr
       1640                 1645                 1650
   Cys Tyr  Thr Asn Gln Ser Pro  Tyr Ser Val Ile Glu  Asn Ser Ala
       1655                 1660                 1665
   Leu Ala  Phe Tyr Thr Ser Cys  Ser Arg Lys Thr Ser  Val Ser Gln
       1670                 1675                 1680
   Thr Ser  Leu Leu Glu Ala Lys  Lys Trp Leu Arg Glu  Gly Ile Phe
       1685                 1690                 1695
   Asp Gly  Gln Pro Glu Arg Ile  Asn Thr Ala Asp Tyr  Val Gly Asn
       1700                 1705                 1710
   Tyr Leu  Tyr Glu Asn Asn Ser  Asn Ser Thr Ile Ala  Glu Asn Asp
       1715                 1720                 1725
   Lys Asn  His Leu Ser Glu Lys  Gln Asp Thr Tyr Leu  Ser Asn Ser
       1730                 1735                 1740
   Ser Met  Ser Asn Ser Tyr Ser  Tyr His Ser Asp Glu  Val Tyr Asn
       1745                 1750                 1755
   Asp Ser  Gly Tyr Leu Ser Lys  Asn Lys Leu Asp Ser  Gly Ile Glu
       1760                 1765                 1770
   Pro Val  Leu Lys Asn Val Glu  Asp Gln Lys Asn Thr  Ser Phe Ser
       1775                 1780                 1785
   Lys Val  Ile Ser Asn Val Lys  Asp Ala Asn Ala Tyr  Pro Gln Thr
                   1790                    1795                    1800
   Val Asn  Glu Asp Ile Cys Val  Glu Glu Leu Val Thr  Ser Ser Ser
       1805                  1810                 1815
   Pro Cys  Lys Asn Lys Asn Ala  Ala Ile Lys Leu Ser  Ile Ser Asn
       1820                  1825                 1830
   Ser Asn  Asn Phe Glu Val Gly  Pro Pro Ala Phe Arg  Ile Ala Ser
       1835                  1840                 1845
   Gly Lys  Ile Val Cys Val Ser  His Glu Thr Ile Lys  Lys Val Lys
       1850                  1855                 1860
   Asp Ile  Phe Thr Asp Ser Phe  Ser Lys Val Ile Lys  Glu Asn Asn
       1865                  1870                 1875
   Glu Asn  Lys Ser Lys Ile Cys  Gln Thr Lys Ile Met  Ala Gly Cys
       1880                  1885                 1890
   Tyr Glu  Ala Leu Asp Asp Ser  Glu Asp Ile Leu His  Asn Ser Leu
       1895                  1900                 1905
   Asp Asn  Asp Glu Cys Ser Thr  His Ser His Lys Val  Phe Ala Asp
       1910                  1915                 1920
   Ile Gln  Ser Glu Glu Ile Leu  Gln His Asn Gln Asn  Met Ser Gly
       1925                  1930                 1935
   Leu Glu  Lys Val Ser Lys Ile  Ser Pro Cys Asp Val  Ser Leu Glu
       1940                  1945                 1950
   Thr Ser  Asp Ile Cys Lys Cys  Ser Ile Gly Lys Leu  His Lys Ser
       1955                  1960                 1965
   Val Ser  Ser Ala Asn Thr Cys  Gly Ile Phe Ser Thr  Ala Ser Gly
       1970                  1975                 1980
   Lys Ser  Val Gln Val Ser Asp  Ala Ser Leu Gln Asn  Ala Arg Gln
       1985                  1990                 1995
   Val Phe  Ser Glu Ile Glu Asp  Ser Thr Lys Gln Val  Phe Ser Lys
       2000                  2005                 2010
   Val Leu  Phe Lys Ser Asn Glu  His Ser Asp Gln Leu  Thr Arg Glu
       2015                  2020                 2025
   Glu Asn  Thr Ala Ile Arg Thr  Pro Glu His Leu Ile  Ser Gln Lys
       2030                  2035                 2040
   Gly Phe  Ser Tyr Asn Val Val  Asn Ser Ser Ala Phe  Ser Gly Phe
       2045                  2050                 2055
   Ser Thr Ala Ser Gly Lys Gln Val Ser Ile Leu Glu Ser Ser Leu
   2060 2065 2070
   His Lys Val Lys Gly Val Leu Glu Glu Phe Asp Leu Ile Arg Thr
   2075 2080 2085
   Glu His Ser Leu His Tyr Ser Pro Thr Ser Arg Gln Asn Val Ser
   2090 2095 2100
   Lys Ile Leu Pro Arg Val Asp Lys Arg Asn Pro Glu His Cys Val
   2105 2110 2115
   Asn Ser Glu Met Glu Lys Thr Cys Ser Lys Glu Phe Lys Leu Ser
   2120 2125 2130
   Asn Asn Leu Asn Val Glu Gly Gly Ser Ser Glu Asn Asn His Ser
   2135 2140 2145
   Ile Lys Val Ser Pro Tyr Leu Ser Gln Phe Gln Gln Asp Lys Gln
   2150 2155 2160
   Gln Leu Val Leu Gly Thr Lys Val Ser Leu Val Glu Asn Ile His
   2165 2170 2175
   Val Leu Gly Lys Glu Gln Ala Ser Pro Lys Asn Val Lys Met Glu
   2180 2185 2190
   Ile Gly Lys Thr Glu Thr Phe Ser Asp Val Pro Val Lys Thr Asn
   2195 2200 2205
   Ile Glu Val Cys Ser Thr Tyr Ser Lys Asp Ser Glu Asn Tyr Phe
   2210 2215 2220
   Glu Thr Glu Ala Val Glu Ile Ala Lys Ala Phe Met Glu Asp Asp
   2225 2230 2235
   Glu Leu Thr Asp Ser Lys Leu Pro Ser His Ala Thr His Ser Leu
   2240 2245 2250
   Phe Thr Cys Pro Glu Asn Glu Glu Met Val Leu Ser Asn Ser Arg
   2255 2260 2265
   Ile Gly Lys Arg Arg Gly Glu Pro Leu Ile Leu Val Gly Glu Pro
   2270 2275 2280
   Ser Ile Lys Arg Asn Leu Leu Asn Glu Phe Asp Arg Ile Ile Glu
   2285 2290 2295
   Asn Gln Glu Lys Ser Leu Lys Ala Ser Lys Ser Thr Pro Asp Gly
   2300 2305 2310
   Thr Ile Lys Asp Arg Arg Leu Phe Met His His Val Ser Leu Glu
   2315 2320 2325
   Pro Ile Thr Cys Val Pro Phe Arg Thr Thr Lys Glu Arg Gln Glu
   2330 2335 2340
   Ile Gln Asn Pro Asn Phe Thr Ala Pro Gly Gln Glu Phe Leu Ser
   2345 2350 2355
   Lys Ser His Leu Tyr Glu His Leu Thr Leu Glu Lys Ser Ser Ser
   2360 2365 2370
   Asn Leu Ala Val Ser Gly His Pro Phe Tyr Gln Val Ser Ala Thr
   2375 2380 2385
   Arg Asn Glu Lys Met Arg His Leu Ile Thr Thr Gly Arg Pro Thr
   2390 2395 2400
   Lys Val Phe Val Pro Pro Phe Lys Thr Lys Ser His Phe His Arg
   2405 2410 2415
   Val Glu Gln Cys Val Arg Asn Ile Asn Leu Glu Glu Asn Arg Gln
   2420 2425 2430
   Lys Gln Asn Ile Asp Gly His Gly Ser Asp Asp Ser Lys Asn Lys
   2435 2440 2445
   Ile Asn Asp Asn Glu Ile His Gln Phe Asn Lys Asn Asn Ser Asn
   2450 2455 2460
   Gln Ala Ala Ala Val Thr Phe Thr Lys Cys Glu Glu Glu Pro Leu
   2465 2470 2475
   Asp Leu Ile Thr Ser Leu Gln Asn Ala Arg Asp Ile Gln Asp Met
   2480 2485 2490
   Arg Ile Lys Lys Lys Gln Arg Gln Arg Val Phe Pro Gln Pro Gly
   2495 2500 2505
   Ser Leu Tyr Leu Ala Lys Thr Ser Thr Leu Pro Arg Ile Ser Leu
   2510 2515 2520
   Lys Ala Ala Val Gly Gly Gln Val Pro Ser Ala Cys Ser His Lys
   2525 2530 2535
   Gln Leu Tyr Thr Tyr Gly Val Ser Lys His Cys Ile Lys Ile Asn
   2540 2545 2550
   Ser Lys Asn Ala Glu Ser Phe Gln Phe His Thr Glu Asp Tyr Phe
   2555 2560 2565
   Gly Lys  Glu Ser Leu Trp Thr  Gly Lys Gly Ile Gln  Leu Ala Asp
       2570                 2575                 2580
   Gly Gly  Trp Leu Ile Pro Ser  Asn Asp Gly Lys Ala  Gly Lys Glu
       2585                 2590                 2595
   Glu Phe  Tyr Arg Ala Leu Cys  Asp Thr Pro Gly Val  Asp Pro Lys
       2600                 2605                 2610
   Leu Ile  Ser Arg Ile Trp Val  Tyr Asn His Tyr Arg  Trp Ile Ile
       2615                 2620                 2625
   Trp Lys  Leu Ala Ala Met Glu  Cys Ala Phe Pro Lys  Glu Phe Ala
       2630                 2635                 2640
   Asn Arg  Cys Leu Ser Pro Glu  Arg Val Leu Leu Gln  Leu Lys Tyr
       2645                 2650                 2655
   Arg Tyr  Asp Thr Glu Ile Asp  Arg Ser Arg Arg Ser  Ala Ile Lys
       2660                 2665                 2670
   Lys Ile  Met Glu Arg Asp Asp  Thr Ala Ala Lys Thr  Leu Val Leu
       2675                 2680                 2685
   Cys Val  Ser Asp Ile Ile Ser  Leu Ser Ala Asn Ile  Ser Glu Thr
       2690                 2695                 2700
   Ser Ser  Asn Lys Thr Ser Ser  Ala Asp Thr Gln Lys  Val Ala Ile
       2705                 2710                 2715
   Ile Glu  Leu Thr Asp Gly Trp  Tyr Ala Val Lys Ala  Gln Leu Asp
       2720                 2725                 2730
   Pro Pro  Leu Leu Ala Val Leu  Lys Asn Gly Arg Leu  Thr Val Gly
       2735                 2740                 2745
   Gln Lys  Ile Ile Leu His Gly  Ala Glu Leu Val Gly  Ser Pro Asp
       2750                 2755                 2760
   Ala Cys  Thr Pro Leu Glu Ala  Pro Glu Ser Leu Met  Leu Lys Ile
       2765                 2770                 2775
   Ser Ala  Asn Ser Thr Arg Pro  Ala Arg Trp Tyr Thr  Lys Leu Gly
       2780                 2785                 2790
   Phe Phe  Pro Asp Pro Arg Pro  Phe Pro Leu Pro Leu  Ser Ser Leu
       2795                 2800                 2805
   Phe Ser  Asp Gly Gly Asn Val  Gly Cys Val Asp Val  Ile Ile Gln
       2810                 2815                 2820
   Arg Ala  Tyr Pro Ile Gln Trp  Met Glu Lys Thr Ser  Ser Gly Leu
   2825 2830 2835
   Tyr Ile Phe Arg Asn Glu Arg Glu Glu Glu Lys Glu Ala Ala Lys
   2840 2845 2850
   Tyr Val Glu Ala Gln Gln Lys Arg Leu Glu Ala Leu Phe Thr Lys
   2855 2860 2865
   Ile Gln Glu Glu Phe Glu Glu His Glu Glu Asn Thr Thr Lys Pro
   2870 2875 2880
   Tyr Leu Pro Ser Arg Ala Leu Thr Arg Gln Gln Val Arg Ala Leu
   2885 2890 2895
   Gln Asp Gly Ala Glu Leu Tyr Glu Ala Val Lys Asn Ala Ala Asp
   2900 2905 2910
   Pro Ala Tyr Leu Glu Gly Tyr Phe Ser Glu Glu Gln Leu Arg Ala
   2915 2920 2925
   Leu Asn Asn His Arg Gln Met Leu Asn Asp Lys Lys Gln Ala Gln
   2930 2935 2940
   Ile Gln Leu Glu Ile Arg Lys Thr Met Glu Ser Ala Glu Gln Lys
   2945 2950 2955
   Glu Gln Gly Leu Ser Arg Asp Val Thr Thr Val Trp Lys Leu Arg
   2960 2965 2970
   Ile Val Ser Tyr Ser Lys Lys Glu Lys Asp Ser Val Ile Leu Ser
   2975 2980 2985
   Ile Trp Arg Pro Ser Ser Asp Leu Tyr Ser Leu Leu Thr Glu Gly
   2990 2995 3000
   Lys Arg Tyr Arg Ile Tyr His Leu Ala Thr Ser Lys Ser Lys Ser
   3005 3010 3015
   Lys Ser Glu Arg Ala Asn Ile Gln Leu Ala Ala Thr Lys Lys Thr
   3020 3025 3030
   Gln Tyr Gln Gln Leu Pro Val Ser Asp Glu Ile Leu Phe Gln Ile
   3035 3040 3045
   Tyr Gln Pro Arg Glu Pro Leu His Phe Ser Lys Phe Leu Asp Pro
   3050 3055 3060
   Asp Phe Gln Pro Ser Cys Ser Glu Val Asp Leu Ile Gly Phe Val
   3065 3070 3075
   Val Ser Val Val Lys Lys Thr Gly Leu Ala Pro Phe Val Tyr Leu
   3080 3085 3090
   Ser Asp Glu Cys Tyr Asn Leu Leu Ala Ile Lys Phe Trp Ile Asp
   3095 3100 3105
   Leu Asn Glu Asp Ile Ile Lys Pro His Met Leu Ile Ala Ala Ser
   3110 3115 3120
   Asn Leu Gln Trp Arg Pro Glu Ser Lys Ser Gly Leu Leu Thr Leu
   3125 3130 3135
   Phe Ala Gly Asp Phe Ser Val Phe Ser Ala Ser Pro Lys Glu Gly
   3140 3145 3150
   His Phe Gln Glu Thr Phe Asn Lys Met Lys Asn Thr Val Glu Asn
   3155 3160 3165
   Ile Asp Ile Leu Cys Asn Glu Ala Glu Asn Lys Leu Met His Ile
   3170 3175 3180
   Leu His Ala Asn Asp Pro Lys Trp Ser Thr Pro Thr Lys Asp Cys
   3185 3190 3195
   Thr Ser Gly Pro Tyr Thr Ala Gln Ile Ile Pro Gly Thr Gly Asn
   3200 3205 3210
   Lys Leu Leu Met Ser Ser Pro Asn Cys Glu Ile Tyr Tyr Gln Ser
   3215 3220 3225
   Pro Leu Ser Leu Cys Met Ala Lys Arg Lys Ser Val Ser Thr Pro
   3230 3235 3240
   Val Ser Ala Gln Met Thr Ser Lys Ser Cys Lys Gly Glu Lys Glu
   3245 3250 3255
   Ile Asp Asp Gln Lys Asn Cys Lys Lys Arg Arg Ala Leu Asp Phe
   3260 3265 3270
   Leu Ser Arg Leu Pro Leu Pro Pro Pro Val Ser Pro Ile Cys Thr
   3275 3280 3285
   Phe Val Ser Pro Ala Ala Gln Lys Ala Phe Gln Pro Pro Arg Ser
   3290 3295 3300
   Cys Gly Thr Lys Tyr Glu Thr Pro Ile Lys Lys Lys Glu Leu Asn
   3305 3310 3315
   Ser Pro Gln Met Thr Pro Phe Lys Lys Phe Asn Glu Ile Ser Leu
   3320 3325 3330
   Leu Glu Ser Asn Ser Ile Ala Asp Glu Glu Leu Ala Leu Ile Asn
   3335 3340 3345
   Thr Gln Ala Leu Leu Ser Gly Ser Thr Gly Glu Lys Gln Phe Ile
       3350                3355                3360
   Ser Val Ser Glu Ser Thr Arg Thr Ala Pro Thr Ser Ser Glu Asp
       3365                3370                3375
   Tyr Leu Arg Leu Lys Arg Arg Cys Thr Thr Ser Leu Ile Lys Glu
       3380                3385                3390
   Gln Glu Ser Ser Gln Ala Ser Thr Glu Glu Cys Glu Lys Asn Lys
       3395                3400                3405
   Gln Asp Thr Ile Thr Thr Lys Lys Tyr Ile
       3410                3415
<210> 14
   <211> 20
   <212> DNA
   <213> Homo sapiens
<400> 14
   tgagttttac ctcagtcaca 20
<210> 15
   <211> 41
   <212> DNA
   <213> Homo sapiens
<400> 15
   caggaaacag ctatgaccct gtgacgtact gggtttttag c 41
<210> 16
   <211> 24
   <212> DNA
   <213> Homo sapiens
<400> 16
   gatctttaac tgttctgggt caca 24
<210> 17
   <211> 22
   <212> DNA
   <213> Homo sapiens
<400> 17
   cccagcatga cacaattaat ga 22
<210> 18
   <211> 44
   <212> DNA
   <213> Homo sapiens
<400> 18
   tgtaaaacga cggccagtag aatgcaaatt tataatccag agta 44
<210> 19
   <211> 22
   <212> DNA
   <213> Homo sapiens
<400> 19
   atcagattca tctttataga ac 22
<210> 20
   <211> 40
   <212> DNA
   <213> Homo sapiens
<400> 20
   tgtaaaacga cggccagttg tgttggcatt ttaaacatca 40
<210> 21
   <211> 38
   <212> DNA
   <213> Homo sapiens
<400> 21
   caggaaacag ctatgaccca gggcaaaggt ataacgct 38
<210> 22
   <211> 38
   <212> DNA
   <213> Homo sapiens
<400> 22
   tgtaaaacga cggccagtta agtgaaataa agagtgaa 38
<210> 23
   <211> 36
   <212> DNA
   <213> Homo sapiens
<400> 23
   caggaaacag ctatgaccag aagtattaga gatgac 36
<210> 24
   <211> 40
   <212> DNA
   <213> Homo sapiens
<400> 24
   tgtaaaacga cggccagtgc catatcttac caccttgtga 40
<210> 25
   <211> 22
   <212> DNA
   <213> Homo sapiens
<400> 25
   ttgcattcta gtgataatat ac 22
<210> 26
   <211> 19
   <212> DNA
   <213> Homo sapiens
<400> 26
   aattgttagc aatttcaac 19
<210> 27
   <211> 40
   <212> DNA
   <213> Homo sapiens
<400> 27
   tgtaaaacga cggccagttg gacctaggtt gattgcagat 40
<210> 28
   <211> 40
   <212> DNA
   <213> Homo sapiens
<400> 28
   caggaaacag ctatgaccta aactgagatc acgggtgaca 40
<210> 29
   <211> 24
   <212> DNA
   <213> Homo sapiens
<400> 29
   gaataatata aattatatgg ctta 24
<210> 30
   <211> 37
   <212> DNA
   <213> Homo sapiens
<400> 30
   caggaaacag ctatgacccc tagtcttgct agttctt 37
<210> 31
   <211> 42
   <212> DNA
   <213> Homo sapiens
<400> 31
   tgtaaaacga cggccagtar ctgaagtgga accaaatgat ac 42
<210> 32
   <211> 44
   <212> DNA
   <213> Homo sapiens
<400> 32
   caggaaacag ctatgaccac gtggcaaaga attctctgaa gtaa 44
<210> 33
   <211> 40
   <212> DNA
   <213> Homo sapiens
<400> 33
   tgtaaaacga cggccagtca gcatcttgaa tctcatacag 40
<210> 34
   <211> 19
   <212> DNA
   <213> Homo sapiens
<400> 34
   agacagaggt acctgaatc 19
<210> 35
   <211> 40
   <212> DNA
   <213> Homo sapiens
<400> 35
   tgtaaaacga cggccagttg gtactttaat tttgtcactt 40
<210> 36
   <211> 37
   <212> DNA
   <213> Homo sapiens
<400> 36
   caggaaacag ctatgacctg caggcatgac agagaat 37
<210> 37
   <211> 22
   <212> DNA
   <213> Homo sapiens
<400> 37
   aagaagcaaa atgtaataag ga 22
<210> 38
   <211> 22
   <212> DNA
   <213> Homo sapiens
<400> 38
   catttaaagc acatacatct tg 22
<210> 39
   <211> 21
   <212> DNA
   <213> Homo sapiens
<400> 39
   tctagaggca aagaatcata c 21
<210> 40
   <211> 22
   <212> DNA
   <213> Homo sapiens
<400> 40
   caagattatt cctttcatta gc 22
<210> 41
   <211> 22
   <212> DNA
   <213> Homo sapiens
<400> 41
   aaccaaaaca caaatctaag ag 22
<210> 42
   <211> 23
   <212> DNA
   <213> Homo sapiens
<400> 42
   gtcattttta tatgctgctt tac 23
<210> 43
   <211> 21
   <212> DNA
   <213> Homo sapiens
<400> 43
   ggttttatat ggagacacag g 21
<210> 44
   <211> 23
   <212> DNA
   <213> Homo sapiens
<400> 44
   gtatttacaa tttcaacaca agc 23
<210> 45
   <211> 20
   <212> DNA
   <213> Homo sapiens
<400> 45
   atcacagttt tggaggtagc 20
<210> 46
   <211> 21
   <212> DNA
   <213> Homo sapiens
<400> 46
   ctgacttcct gattcttcta a 21
<210> 47
   <211> 21
   <212> DNA
   <213> Homo sapiens
<400> 47
   ctcagatgtt attttccaag c 21
<210> 48
   <211> 21
   <212> DNA
   <213> Homo sapiens
<400> 48
   ctgttaaata accagaagca c 21
<210> 49
   <211> 18
   <212> DNA
   <213> Homo sapiens
<400> 49
   aggtagacag cagcaagc 18
<210> 50
   <211> 22
   <212> DNA
   <213> Homo sapiens
<400> 50
   gtaatatcag ttggcattta tt 22
<210> 51
   <211> 21
   <212> DNA
   <213> Homo sapiens
<400> 51
   tgcagaggta catccaataa g 21
<210> 52
   <211> 21
   <212> DNA
   <213> Homo sapiens
<400> 52
   gatcagtaaa tagcaagtcc g 21
<210> 53
   <211> 23
   <212> DNA
   <213> Homo sapiens
<400> 53
   tactgaaaat gaagataaca aat 23
<210> 54
   <211> 22
   <212> DNA
   <213> Homo sapiens
<400> 54
   attttgttct ttcttatgtc ag 22
<210> 55
   <211> 35
   <212> DNA
   <213> Homo sapiens
<400> 55
   tgtaaaacga cggccagtct actaaaacgg agcaa 35
<210> 56
   <211> 35
   <212> DNA
   <213> Homo sapiens
<400> 56
   caggaaacag ctatgaccgt atgaaaaccc aacag 35
<210> 57
   <211> 22
   <212> DNA
   <213> Homo sapiens
<400> 57
   cacaaaatac tgaaagaaag tg 22
<210> 58
   <211> 19
   <212> DNA
   <213> Homo sapiens
<400> 58
   ggcaccacag tctcaatag 19
<210> 59
   <211> 20
   <212> DNA
   <213> Homo sapiens
<400> 59
   gcaaagaccc taaagtacag 20
<210> 60
   <211> 22
   <212> DNA
   <213> Homo sapiens
<400> 60
   catcaaatat tccttctcta ag 22
<210> 61
   <211> 35
   <212> DNA
   <213> Homo sapiens
<400> 61
   tgtaaaacga cggccagtga aaattcagcc ttagc 35
<210> 62
   <211> 35
   <212> DNA
   <213> Homo sapiens
<400> 62
   caggaaacag ctatgaccat cagaatggta ggaat 35
<210> 63
   <211> 22
   <212> DNA
   <213> Homo sapiens
<400> 63
   gtactatagc tgaaaatgac aa 22
<210> 64
   <211> 20
   <212> DNA
   <213> Homo sapiens
<400> 64
   accactggct atcctaaatg 20
<210> 65
   <211> 20
   <212> DNA
   <213> Homo sapiens
<400> 65
   tgaagatatt tgcgttgagg 20
<210> 66
   <211> 20
   <212> DNA
   <213> Homo sapiens
<400> 66
   gtcagcaaaa accttatgtg 20
<210> 67
   <211> 21
   <212> DNA
   <213> Homo sapiens
<400> 67
   acgaaaatta tggcaggttg t 21
<210> 68
   <211> 21
   <212> DNA
   <213> Homo sapiens
<400> 68
   cttgtcttgc gttttgtaat g 21
<210> 69
   <211> 20
   <212> DNA
   <213> Homo sapiens
<400> 69
   gcttcataag tcagtctcat 20
<210> 70
   <211> 20
   <212> DNA
   <213> Homo sapiens
<400> 70
   tcaaattcct ctaacactcc 20
<210> 71
   <211> 35
   <212> DNA
   <213> Homo sapiens
<400> 71
   tgtaaaacga cggccagtta cagcaagtgg aaagc 35
<210> 72
   <211> 37
   <212> DNA
   <213> Homo sapiens
<400> 72
   caggaaacag ctatgaccaa gtttcagttt taccaat 37
<210> 73
   <211> 22
   <212> DNA
   <213> Homo sapiens
<400> 73
   gttcttcaga aaataatcac tc 22
<210> 74
   <211> 21
   <212> DNA
   <213> Homo sapiens
<400> 74
   tgtaaaaaga gaatgtgtgg c 21
<210> 75
   <211> 39
   <212> DNA
   <213> Homo sapiens
<400> 75
   tgtaaaacga cggccagtac tttttctgat gttcctgtg 39
<210> 76
   <211> 39
   <212> DNA
   <213> Homo sapiens
<400> 76
   caggaaacag ctatgaccta aaaatagtga ttggcaaca 39
<210> 77
   <211> 42
   <212> DNA
   <213> Homo sapiens
<400> 77
   tgtaaaacga cggccagtag tggtgtttta aagtggtcaa aa 42
<210> 78
   <211> 40
   <212> DNA
   <213> Homo sapiens
<400> 78
   caggaaacag ctatgaccgg atccacctga ggtcagaata 40
<210> 79
   <211> 21
   <212> DNA
   <213> Homo sapiens
<400> 79
   taacatttaa gcatccgtta c 21
<210> 80
   <211> 28
   <212> DNA
   <213> Homo sapiens
<400> 80
   aaacgagact tttctcatac tgtattag 28
<210> 81
   <211> 22
   <212> DNA
   <213> Homo sapiens
<400> 81
   accatgtagc aaatgagggt ct 22
<210> 82
   <211> 22
   <212> DNA
   <213> Homo sapiens
<400> 82
   gcttttgtct gttttcctcc aa 22
<210> 83
   <211> 21
   <212> DNA
   <213> Homo sapiens
<400> 83
   ccaggggttg tgctttttaa a 21
<210> 84
   <211> 38
   <212> DNA
   <213> Homo sapiens
<400> 84
   caggaaacag ctatgaccac tctgtcataa aagccatc 38
<210> 85
   <211> 24
   <212> DNA
   <213> Homo sapiens
<400> 85
   tttggtttgt tataattgtt ttta 24
<210> 86
   <211> 20
   <212> DNA
   <213> Homo sapiens
<400> 86
   ccaacttttt agttcgagag 20
<210> 87
   <211> 19
   <212> DNA
   <213> Homo sapiens
<400> 87
   ttcagtatca tcctatgtg 19
<210> 88
   <211> 20
   <212> DNA
   <213> Homo sapiens
<400> 88
   agaaacctta acccatactg 20
<210> 89
   <211> 39
   <212> DNA
   <213> Homo sapiens
<400> 89
   tgtaaaacga cggccagtga attctagagt cacacttcc 39
<210> 90
   <211> 38
   <212> DNA
   <213> Homo sapiens
<400> 90
   caggaaacag ctatgacctt taactgaatc aatgactg 38
<210> 91
   <211> 41
   <212> DNA
   <213> Homo sapiens
<400> 91
   tgtaaaacga cggccagtaa gtgaatattt ttaaggcagt t 41
<210> 92
   <211> 39
   <212> DNA
   <213> Homo sapiens
<400> 92
   caggaaacag ctatgaccaa gagaccgaaa ctccatctc 39
<210> 93
   <211> 38
   <212> DNA
   <213> Homo sapiens
<400> 93
   tgtaaaacga cggccagtca ctgtgcctgg cctgatac 38
<210> 94
   <211> 39
   <212> DNA
   <213> Homo sapiens
<400> 94
   caggaaacag ctatgaccat gttaaattca aagtctcta 39
<210> 95
   <211> 39
   <212> DNA
   <213> Homo sapiens
<400> 95
   tgtaaaacga cggccagtgg gtgttttatg cttggttct 39
<210> 96
   <211> 40
   <212> DNA
   <213> Homo sapiens
<400> 96
   caggaaacag ctatgaccca tttcaacata ttccttcctg 40
<210> 97
   <211> 19
   <212> DNA
   <213> Homo sapiens
<400> 97
   aaccacaccc ttaagatga 19
<210> 98
   <211> 20
   <212> DNA
   <213> Homo sapiens
<400> 98
   gcattagtag tggattttgc 20
<210> 99
   <211> 16
   <212> DNA
   <213> Homo sapiens
<400> 99
   tcacttccat tgcatc 16
<210> 100
   <211> 17
   <212> DNA
   <213> Homo sapiens
<400> 100
   tgccaactgg tagctcc 17
<210> 101
   <211> 20
   <212> DNA
   <213> Homo sapiens
<400> 101
   tacagttagc agcgacaaaa 20
<210> 102
   <211> 38
   <212> DNA
   <213> Homo sapiens
<400> 102
   caggaaacag ctatgaccat ttgccaactg gtagctcc 38
<210> 103
   <211> 20
   <212> DNA
   <213> Homo sapiens
<400> 103
   gctttcgcca aattcagcta 20
<210> 104
   <211> 20
   <212> DNA
   <213> Homo sapiens
<400> 104
   taccaaaatg tgtggtgatg 20
<210> 105
   <211> 20
   <212> DNA
   <213> Homo sapiens
<400> 105
   aatcactgat actggttttg 20
<210> 106
   <211> 20
   <212> DNA
   <213> Homo sapiens
<400> 106
   tatacttaca ggagccacat 20
<210> 107
   <211> 18
   <212> DNA
   <213> Homo sapiens
<400> 107
   ctgtgtgtaa tatttgcg 18
<210> 108
   <211> 40
   <212> DNA
   <213> Homo sapiens
<400> 108
   caggaaacag ctatgacggc aagttcttcg tcagctattg 40
<210> 109
   <211> 40
   <212> DNA
   <213> Homo sapiens
<400> 109
   tgtaaaacga cggccagtga attctcctca gatgactcca 40
<210> 110
   <211> 38
   <212> DNA
   <213> Homo sapiens
<400> 110
   caggaaacag ctatgacctc tttgctcatt gtgcaaca 38

## Description

This is an U.S. utility patent application based on U.S. Provisional Application Serial Nos. 60/055,784 filed on August 15, 1997, 60/064,926 filed on November 7, 1997, and 60/065,367 filed on November 12, 1997.

### FIELD OF THE INVENTION

This invention relates to a gene which has been associated with breast cancer where the gene is found to be mutated. More specifically, this invention relates to five unique coding sequences of BRCA2 gene BRCA2^{(omi1)}, BRCA2^{(omi2)}, BRCA2^{(omi3)}, BRCA2^{(omi4)}, and BRCA2^{(omi5)} identified in human subjects which define five novel haplotypes.

### BACKGROUND OF THE INVENTION

It has been estimated that about 5-10% of breast cancer is inherited (Rowell, S., et al., American Journal of Human Genetics 55:861-865 (1994)). The first gene associated with both breast and ovarian cancer was cloned in 1994 from chromosome 17 by Miki, Y., et al., Science 266:66-71 (1994). A second high-risk breast cancer conferring gene was located on chromosome 13 in 1994 (Wooster, R., et al., Science 265:2088-2090) and subsequently cloned in 1995 (Wooster, R., et al., Nature 378:789-792). Mutations in this "tumor suppressor" gene are thought to account for roughly 35% of inherited breast cancer and 80-90% of families with male breast cancer.

Locating one or more mutations in the BRCA2 region of chromosome 13 provides a promising approach to reducing the high incidence and mortality associated with breast cancer through the early detection of women and men at high risk. These individuals, once identified, can be targeted for more aggressive prevention programs. Screening is carried out by a variety of methods which include karyotyping, probe binding and DNA sequencing.

In DNA sequencing technology, genomic DNA is extracted from whole blood and the coding regions of the BRCA2 gene are amplified. Each of the coding regions may be sequenced completely and the results are compared to the normal DNA sequence of the gene. Alternatively, the coding sequence of the sample gene may be compared to a panel of known mutations or other screening procedure before completely sequencing the gene and comparing it to a normal sequence of the gene.

The BRCA2 gene is divided into 27 separate exons. Exon 1 is noncoding, in that it is not part of the final functional BRCA2 protein product. The BRCA2 coding region spans roughly 10433 base pairs (bp) over 70 kb. Each exon consists of 100-600 bp, except for exons 10, 11 and 27. The full length mRNA is 11-12 kb. To sequence the coding region of the BRCA2 gene, each exon is amplified separately and the resulting PCR products are sequenced in the forward and reverse directions. Because exons 10, 11, and 27 are so large, we have divided them into three, twenty-one, and two overlapping PCR fragments (respectively) of approximately 250-625 bp each (segments "A" through "C" of exon 10, "A" through "U" of exon 11, and "A" through "B" of exon 27).

Many mutations and normal polymorphisms have already been reported in the BRCA2 gene. A world wide web site has been built to facilitate the detection and characterization of alterations in breast cancer susceptibility genes. Such mutations in BRCA2 can be accessed through the Breast Cancer Information Core (BIC) at http://www.nhgri.nih.gov/intramural_research/Lab_transfer/Bic. This data site became publicly available on November 1, 1995. Friend, S. et al. Nature Genetics 11:238, (1995). The information on BRCA2 was added in February, 1996.

The genetics of Breast Cancer Syndrome is autosomal dominant with reduced penetrance. In simple terms, this means that the syndrome runs through families: (1) both sexes can be carriers (mostly women get the disease but men can both pass it on and occasionally get breast cancer); (2) most generations will likely have breast cancer; (3) occasionally women carriers either die young before they have the time to manifest disease (and yet have offspring who get it) or they never develop breast or ovarian cancer and die of old age (the latter people are said to have "reduced penetrance" because they never develop cancer). Pedigree analysis and genetic counseling is absolutely essential to the proper workup of a family prior to any lab work.

Until now, the only sources of genomic sequence information for BRCA2 were GenBank (Accession Number U43746), or through the Breast Information Core (BIC) database on the Internet which requires membership in the BIC consortium. However, based upon the disclosure of this patent application, in neither GenBank nor BIC were the sequences identified and listed entirely accurate. There is a need in the art to correct these mistakes which otherwise may lead to misinterpretation of the sequence data from the patient as abnormal when it was not, or vice versa.

In addition, there is a need in the art to have available a functional allele profile which represents the most likely BRCA2 sequences to be found in the majority of the normal population. This functional allele profile is based upon frequent polymorphisms and the correct backbone sequence. The knowledge of several common normal haplotypes will make it possible for true mutations to be easily identified or differentiated from polymorphisms. Identification of mutations of the BRCA2 gene and protein would allow more widespread diagnostic screening for hereditary breast cancer than is currently possible.

The use of these common normal haplotypes, in addition to the previously published BRCA2 sequence, will reduce the likelihood of misinterpreting a "sequence variation" found in the normal population with a pathologic "mutation" (i.e. causes disease in the individual or puts the individual at a high risk of developing the disease). With large interest in breast cancer predisposition testing, misinterpretation is particularly worrisome. People who already have breast cancer are asking the clinical question: "is my disease caused by a heritable genetic mutation?" The relatives of the those with breast cancer are asking the question: "Am I also a carrier of the mutation my relative has? Thus, is my risk increased, and should I undergo a more aggressive surveillance program?"

E.g. WO 97/18110 discloses the primary encoding gene sequence of the human BRCA2 gene, both at the genomic nucleotide context as well as in view of the encoding cDNA. Likewise, WO 97/22689 discloses the structure of the BRCA2 gene as well as related somatic mutations linked to the diagnosis and predisposition of breast cancer.

When comparing the reported exon/intron boundaries of the human BRCA2 gene with their own findings, the authors of the current patent application became aware of crucial differences within those parts of the BRCA2 gene which the genomic region of exon 15 and 16, resulting in some wrongly identified splice sites. Consequently, there exists a need within the prior art to correct the genomic BRCA2 sequence as currently disclosed.

### SUMMARY OF THE INVENTION

The present invention is based on the discovery of the correct genomic BRCA2 sequence and five novel sequence haplotypes found in normal human subjects of the BRCA2 gene.

It is an object of this invention to provide the correct intronic/exonic sequence of the BRCA2 gene.

It is another object of this invention to provide five unique haplotype sequences of the BRCA2 gene in normal individuals which do not correspond to increased cancer susceptible.

It is another object of this invention to sequence a BRCA2 gene or a portion thereof and compare it to the five haplotype sequences to determine whether a sequence variation noted represents a polymorphism or a potentially harmful mutation.

It is another object of this invention to provide a list of the pairs which occur at each of ten polymorphic points in the BRCA2 gene.

It is another object of this invention to provide the rates of occurrence for the polymorphisms at codons 289, 372, 455, 743, 894, 991, 1132, 1269, 2414, and 2951 in the BRCA2 gene.

It is another object of this invention to provide a method wherein all exons of BRCA2 gene or parts thereof, are amplified with one or more oligonucleotide primers.

It is another object of this invention to provide a method of identifying a individual who carries no mutation(s) of the BRCA2 gene and is therefore at no increased risk or susceptibility to breast or ovarian cancer based on a finding that the individual does not carry an abnormal BRCA2 genes.

It is another object of this invention to provide a method of identifying a mutation in BRCA2 gene leading to predisposition or higher susceptibility to breast or ovarian cancer.

It is another object of this invention to provide five novel BRCA2 protein sequences derived from five BRCA2 haplotype sequences.

It is another object of the invention to encompass prokaryotic or eukaryotic host cells comprising an expression vector having a DNA sequence that encodes for all or a fragment of the five novel BRCA2 protein sequences, a BRCA2 polypeptide thereof, or a functional equivalent thereof.

It is another object of the invention to encompass an anti-BRCA2 protein antibody using all of fragments of the five novel BRCA2 protein sequences, a BRCA2 polypeptide thereof or a functional equivalent thereof as an immunogen.

There is a need in the art for cDNA sequences of the BRCA2 gene and for the protein sequences of BRCA2 gene from normal individuals who are not at risk for increased susceptibility for cancer. In order to determine whether a sample from a patient suspected of containing a BRCA2 mutation actually has the mutation, the patient's BRCA2 DNA and/or amino acid sequence need to be compared to all known normal BRCA2 sequences. Failure to compare the sequence obtained to all naturally occurring normal sequences may result in reporting a sample as containing a potentially harmful mutation when it is a polymorphism without clinical significance.

A person skilled in the art of genetic susceptibility testing will find the present invention useful for:
a) identifying individuals having a normal BRCA2 gene with no coding sequence mutations, who therefore cannot be said to have an increased genetic susceptibility to breast or ovarian cancer from their BRCA2 genes;
b) avoiding misinterpretation of normal polymorphisms found in the BRCA2 gene;
c) determining the presence of a previously unknown mutation in the BRCA2 gene;
d) identifying a mutation in exon 11 of BRCA2 which indicates a
   predisposition or higher susceptibility to ovarian cancer than breast
   cancer (i.e., resides in the putative "ovarian cancer cluster" region);
e) probing a human sample of the BRCA2 gene by allele to determine the presence or absence of either polymorphic alleles or mutations;
f) performing gene therapy with the correct BRCA2 gene sequence.
g) performing protein replacement therapy with the correct BRCA 2 protein sequence or a functional equivalent thereof.

### BRIEF DESCRIPTION OF THE FIGURES

FIGURE 1 shows the GenBank genomic sequence of BRCA2 (Accession Number U43746). The lower case letters denote intronic sequences and the upper case letters denote exonic sequences. Incorrect exonic sequences at exons 5 and 16 are shown with boldface type.
FIGURE 2 shows the corrected genomic sequence of BRCA2. The lower case letters denote intronic sequences and the upper case letters denote exonic sequences. Corrected intronic and exonic sequences at exons 5, 11 and 15 are shown with boldface type.

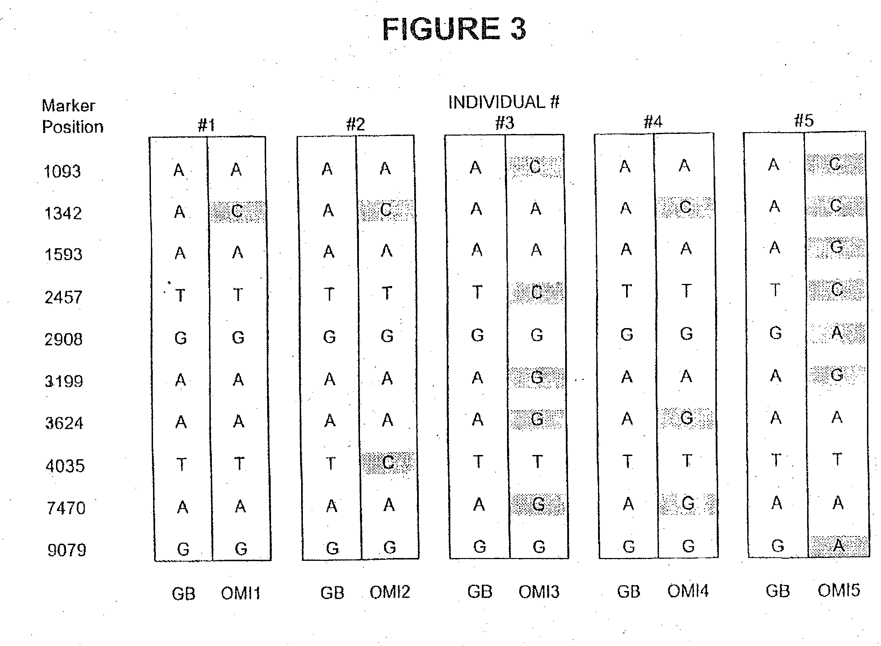

FIGURE 3 shows the alternative alleles at polymorphic sites along a chromosome which can be represented as a unit or "haplotype" within a gene such as BRCA2. The haplotype that is in GenBank (GB) is shown with light shading. Five additional haplotypes are shown in FIGURE 3 (encompassing the alternative alleles found at nucleotide sites 1093, 1342, 1593, 2457, 2908, 3199, 3624, 4035, 7470 and 9079). BRCA2 ^{(omi-1)}, BRCA2 ^{(omi-2)}, BRCA2 ^{(omi-3)}, BRCA2 ^{(omi-4)}, and BRCA2 ^{(omi-5)} are represented with mixed dark and light shading (numbers 2, 4, 6, 8 and 10 from left to right). In total, 5 of 10 haplotypes along the BRCA2 gene are unique.

### DETAILED DESCRIPTION OF THE INVENTION

### DEFINITIONS

The following definitions are provided for the purpose of understanding this invention.

"Breast and Ovarian cancer" is understood by those skilled in the art to include breast, ovarian and pancreatic cancer in women and also breast, prostate and pancreatic cancer in men. BRCA2 is associated with genetic susceptibility to breast, ovarian and pancreatic cancer. Therefore, claims in this document which recite breast and/or ovarian cancer refer to breast, ovarian, prostate, and pancreatic cancers in men and women.

"Coding sequence" refers to those portions of a gene which, taken together, code for a peptide (protein), or which nucleic acid itself has function.

"Protein" or "peptide" refers to a sequence of amino acids which has function.

"BRCA2^{(omi)}" refers to the genomic BRCA2 sequence disclosed in Genbank (Accession Number U43746) wherein,
(1) a 10 bp stretch (5'-TTTATTTTAG-3') is intronic at 3' end of intron 4, rather than at the 5' end of exon 5; and
(2) a 16 bp stretch (5'-GTGTTCTCATAAACAG-3') is exonic at the 3' end of exon 15, rather than at the 5' end of exon.

"BRCA2^{(omi 1-5)}" refers to five unique DNA sequences of the BRCA2 gene and their introns (particularly the slice sites adjacent to the exons). These sequences were found by end to end sequencing of the BRCA2 gene from 5 individuals randomly drawn from the population and who were documented to have no family history of breast or ovarian cancer. The sequenced exons were found not to contain any truncating mutations. In all cases the change of a nucleic acid at a polymorphic site lead to a codon change and a change of amino acid from the previously published standard in GenBank (see TABLE III). In some cases the frequency of occurrence of a nucleic acid change was found to differ from the published frequency or was newly determined. These sequence variations are believed to be alleles whose haplotypes do not indicate an increased risk for cancer.

"Normal DNA sequence" also called " normal gene sequence" refers to a nucleic acid sequence, the nucleic acid of which are known to occur at their respective positions with high frequency in a population of individuals who carry the gene which codes for a normally functioning protein, or which itself has normal function.

"Normal Protein Sequence" refers to the protein sequence, the amino acids of which are known to occur with high frequency in a population of individuals who carry the gene which codes for a normally functioning protein.

"Normal Sequence" refers to the nucleic acid or protein sequence, the nucleic or amino acids of which are known to occur with high frequency in a population of individuals who carry the gene which codes for a normally functioning protein, or which nucleic acid itself has a normal function.

"Haplotype" refers to a series of specific alleles within a gene along a chromosome.

"Functional allele profile" refers a list of those alleles in the normal population which have the funll function.

"Mutation" refers to a base change or a gain or loss of base pair(s) in a DNA sequence, which results in a DNA sequence coding for a non-functional protein or a protein with substantially reduced or altered function.

"Polymorphism" refers to a base change in a DNA sequence which is not associated with known pathology.

"Primer" refers to a sequence comprising about 15 or more nucleotides having a sequence complementary to the BRCA2 gene. Other primers which can be used for primer hybridization will be known or readily ascertainable to those skilled in the art.

"Substantially complementary to" refers to primer sequences which hybridize to the sequences provided under stringent conditions and/or sequences having sufficient homology with BRCA2 sequences, such that the allele specific oligonucleotide primers hybridize to the BRCA2 sequences to which they are complimentary.

"Isolated nucleic acids" refers to nucleic acids substantially free of other nucleic acids, proteins, lipids, carbohydrates or other materials with which they may be associated. Such association is typically either in cellular material or in a synthesis medium.

"Biological sample" or "body sample" refers to a sample containing DNA oatained from a biological source. The sample may be from a living, dead or even archeological source from a variety of tissues and cells. Examples include body fluid (e.g. blood (leukocytes), urine (epithelial cells), saliva, breast milk, menstrual flow, cervical and vaginal secretions, etc.), skin, hair roots/follicle, mucus membrane (e.g. buccal or tongue cell scrapings), cervicovaginal cells (from PAP smear, etc.), lymphatic tissue, internal tissue (normal or tumor).

"Vector" refers to any polynucleotide which is capable of self replication or inducing integration into a self-replicating polynucleotide. Examples include polynucleotides containing an origin or replication or an integration site. Vectors may be intergrated into the host cell's chromosome or form an autonomously replicating unit.

"A tumor growth inhibitor" refers to a molecule such as, all or a fragment of BRCA2 protein, a BRCA2 polypeptide, or a functional equivalent thereof that is effective for preventing the formation of, reducing, or eliminating a transformed or malignant phenotype of breast or ovarian cancer cells.

"A BRCA2 polypeptide" refers to a BRCA2 polypeptide either directly derived from the BRCA2 protein, or homologous to the BRCA2 protein, or a fusion protein consisting of all or fragments of the BRCA2 protein and polypeptides.

"A functional equivalent" refers to a molecule including an unnatural BRCA2 polypeptide, a drug or a natural product which retains substantial biological activity as the native BRCA2 protein. The activity and function of BRCA2 protein may include transactivation, granin, DNA repair, among others.

"A target polynucleotide" refers to the nucleic acid sequence of interest, for example, the BRCA2 encoding polynucleotide. Other primers which can be used for primer hybridization will be known or readily ascertainable to those of skill in the art.

The invention in several of its embodiments includes: an isolated DNA sequence of the BRCA2 coding sequence as set forth in SEQ ID NO:4, 6, 8, 10, and 12, a protein sequence of the BRCA2 protein as set forth in SEQ ID NO:5, 7, 9, 11, 13, a method of identifying individuals having a normal BRCA2 gene with no increased risk for breast and ovarian cancer, a method of detecting an increased genetic susceptibility to breast and ovarian cancer in an individual resulting from the presence of a mutation in the BRCA2 coding sequence, a method of performing gene therapy to prevent or treat a tumor, a method of protein replacement therapy to prevent or treat a tumor, a diagnostic reagent comprising all or fragments of the disclosed BRCA2 cDNA and protein sequences.

### SEQUENCING

Any nucleic acid specimen, in purified or non-purified form, can be utilized as the starting nucleic acid, providing it contains, or is suspected of containing, the specific nucleic acid sequence containing a polymorphic or a mutant allele. Thus, the process may amplify, for example, DNA or RNA, including mRNA and cDNA, wherein DNA or RNA may be single stranded or double stranded. In the event that RNA is to be used as a template, enzymes and/or conditions optimal for reverse transcribing the template to DNA would be utilized. In addition, a DNA-RNA hybrid which contains one strand of each may be utilized. A mixture of nucleic acids may also be employed, or the nucleic acids produced in a previous method such as an amplification reaction using the same or different primers may be so utilized. The specific nucleic acid sequence to be amplified, i.e., the polymorphic and/or the mutant allele, may be a fraction of a larger molecule or can be present initially as a discrete molecule, so that the specific sequence constitutes the entire nucleic acid. A variety of amplification techniques may.be used such as ligating the DNA sample or fragments thereof to a vector capable of replication or incorporation into a replicating system thereby increasing the number of copies of DNA suspected of containing at least a portion of the BRCA2 gene. Amplification techniques include so called "shot gun cloning". It is not necessary that the sequence to be amplified be present initially in a pure form; it may be a minor fraction of a complex mixture, such as contained in whole human DNA.

It should be noted that one need not sequence the entire coding region or even an entire DNA fragment in order to determine whether or not a mutation is present. For example, when a mutation is known in one family member, it is sufficient to determine the sequence at only the mutation site by sequencing or by other mutation detection systems such as ASO when testing other family members.

DNA utilized herein may be extracted from a body sample, such as blood, tissue material and other biological sample by a variety of techniques such as that described by Maniatis, et al. in Molecular Cloning: A Laboratory Manual, Cold Spring Harbor, NY, p 280-281, 1982). If the extracted sample is impure, it may be treated before amplification with an amount of a reagent effective to open the cells, and to expose and/or separate the strand(s) of the nucleic acid(s). This lysing and nucleic acid denaturing step to expose and separate the strands will allow amplification to occur much more readily.

For amplification by cloning, the isolated DNA may be cleaved into fragments by a restriction endonuclease or by shearing by passing the DNA containing mixture through a 25 gauge needle from a syringe to prepare 1-1.5 kb fragments. The fragments are then ligated to a cleaved vector (virus, plasmid, transposon, cosmid etc.) and then the recombinant vector so formed is then replicated in a manner typical for that vector.

For a PCR amplification, the deoxyribonucleotide triphosphates dATP, dCTP, dGTP, and dTTP are added to the synthesis mixture, either separately or together with the primers, in adequate amounts and the resulting solution is heated to about 90°-100°C from about 1 to 10 minutes, preferably from 1 to 4 minutes. After this heating period, the solution is allowed to cool, which is preferable for the primer hybridization. To the cooled mixture is added an appropriate agent for effecting the primer extension reaction (called herein "agent for polymerization"), and the reaction is allowed to occur under conditions known in the art. The agent for polymerization may also be added together with the other reagents if it is heat stable. This synthesis (or amplification) reaction may occur at room temperature up to a temperature above which the agent for polymerization no longer functions. Thus, for example, if DNA polymerase is used as the agent, the temperature is generally no greater than about 40°C. Most conveniently the reaction occurs at room temperature. When using thermostable DNA polymerase such as Taq, higher temperature may be used.

The allele specific oligonucleotide primers are useful in determining whether a subject is at risk of having breast or ovarian cancer, and also useful for characterizing a tumor. Primers direct amplification of a target polynucleotide prior to sequencing. These unique BRCA2 oligonucleotide primers for exons 2-27 shown in TABLE II were designed and produced specifically to optimize amplification of portions of BRCA2 which are to be sequenced.

The primers used to carry out this invention embrace oligonucleotides of sufficient length and appropriate sequence to provide initiation of polymerization. Environmental conditions conducive to synthesis include the presence of nucleoside triphosphates and an agent for polymerization, such as DNA polymerase, and a suitable temperature and pH. The primer is preferably single stranded for maximum efficiency in amplification, but may be double stranded. If double stranded, the primer is first treated to separate its strands before being used to prepare extension products. The primer must be sufficiently long to prime the synthesis of extension products in the presence of the inducing agent for polymerization. The exact length of primer will depend on many factors, including temperature, buffer, and nucleotide composition. The oligonucleotide primer topically contains 18-28 bp plus in some cases an M13 "tail" for convenience.

Primers used to carry out this invention are designed to be substantially complementary to each strand of the genomic locus to be amplified. This means that the primers must be sufficiently complementary to hybridize with their respective strands under conditions which allow the agent for polymerization to perform. In other words, the primers should have sufficient complementarity with the 5' and 3' sequences flanking the mutation to hybridize therewith and permit amplification of the genomic locus.

Oligonucleotide primers of the invention are employed in the amplification process which is an enzymatic chain reaction that produces exponential quantities of polymorphic locus relative to the number of reaction steps involved. Typically, one primer is complementary to the negative (-) strand of the polymorphic locus and the other is complementary to the positive (+) strand. Annealing the primers to denatured nucleic acid followed by extension with an enzyme, such as the large fragment of DNA polymerase I (Klenow) and nucleotides, results in newly synthesized + and - strands containing the target polymorphic locus sequence. Because these newly synthesized sequences are also templates, repeated cycles of denaturing, primer annealing, and extension results in exponential production of the region (*i.e.,* the target polymorphic locus sequence) defined by the primers. The product of the chain reaction is a discreet nucleic acid duplex with termini corresponding to the ends of the specific primers employed.

The oligonucleotide primers of the invention may be prepared using any suitable method, such as conventional phosphotriester and phosphodiester methods or automated embodiments thereof. In one such automated embodiment, diethylphosphoramidites are used as starting materials and may be synthesized as described by Beaucage, et al., Tetrahedron Letters, 22:1859-1862, 1981. One method for synthesizing oligonucleotides on a modified solid support is described in U.S. Patent No. 4,458,066.

The agent for polymerization may be any compound or system which will function to accomplish the synthesis of primer extension products, including enzymes. Suitable enzymes for this purpose include, for example, *E. coli* DNA polymerase I, Klenow fragment of *E. coli* DNA polymerase, polymerase muteins, reverse transcriptase, other enzymes, including heat-stable enzymes (*i*.*e*., those enzymes which perform primer extension after being subjected to temperatures sufficiently elevated to cause denaturation), such as Taq polymerase. Suitable enzymes will facilitate combination of the nucleotides in the proper manner to form the primer extension products which are complementary to each polymorphic locus nucleic acid strand. Generally, the synthesis will be initiated at the 3' end of each primer and proceed in the 5' direction along the template strand, until synthesis terminates, producing molecules of different lengths.

The newly synthesized strand and its complementary nucleic acid strand will form a double-stranded molecule under hybridizing conditions described above and this hybrid is used in subsequent steps of the process. In the next step, the newly synthesized double-stranded molecule is subjected to denaturing conditions using any of the procedures described above to provide single-stranded molecules.

The steps of denaturing, annealing, and extension product synthesis can be repeated as often as needed to amplify the target polymorphic locus nucleic acid sequence to the extent necessary for detection. The amount of the specific nucleic acid sequence produced will accumulate in an exponential fashion. Amplification is described in PCR. A Practical Approach, ILR Press, Eds. M. J. McPherson, P. Quirke, and G. R. Taylor, 1992.

The amplification products may be detected by Southern blots analysis, without using radioactive probes, In such a process, for example, a small sample of DNA containing a very low level of the nucleic acid sequence of the polymorphic locus is amplified, and analyzed via a Southern blotting technique or similarly, using dot blot analysis. The use of nonradioactive probes or labels is facilitated by the high level of the amplified signal. Alternatively, probes used to detect the amplified products can be directly or indirectly detectably labeled, for example, with a radioisotope, a fluorescent compound, a bioluminescent compound, a chemiluminescent compound, a metal chelator or an enzyme. Those of ordinary skill in the art will know of other suitable labels for binding to the probe, or will be able to ascertain such, using routine experimentation.

Sequences amplified by the methods of the invention can be further evaluated, detected, cloned, sequenced, and the like, either in solution or after binding to a solid support, by any method usually applied to the detection of a specific DNA sequence such as PCR, oligomer restriction (Saiki, et.al., Bio/Technology, 3:1008-1012, 1985), allele-specific oligonucleotide (ASO) probe analysis (Conner, et al., Proc. Natl. Acad. Sci. U.S.A., 80:278, 1983), oligonucleotide ligation assays (OLAs) (Landgren, et al., Science, 241:1007, 1988), and the like. Molecular techniques for DNA analysis have been reviewed (Landgren, et al., Science, 242:229-237, 1988).

Preferably, the method of amplifying is by PCR, as described herein and as is commonly used by those of ordinary skill in the art. Alternative methods of amplification have been described and can also be employed as long as the BRCA2 locus amplified by PCR using primers of the invention is similarly amplified by the alternative means. Such alternative amplification systems include but are not limited to self-sustained sequence replication, which begins with a short sequence of RNA of interest and a T7 promoter. Reverse transcriptase copies the RNA into cDNA and degrades the RNA, followed by reverse transcriptase polymerizing a second strand of DNA. Another nucleic acid amplification technique is nucleic acid sequence-based amplification (NASBA) which uses reverse transcription and T7 RNA polymerase and incorporates two primers to target its cycling scheme. NASBA can begin with either DNA or RNA and finish with either, and amplifies to 10⁸ copies within 60 to 90 minutes. Alternatively, nucleic acid can be amplified by ligation activated transcription (LAT). LAT works from a single-stranded template with a single primer that is partially single-stranded and partially double-stranded. Amplification is initiated by ligating a cDNA to the promoter oligonucleotide and within a few hours, and amplification is 10⁸ to 10⁹ fold. Another amplification system useful in the method of the invention is the Qβ Replicase System. The Qβ replicase system can be utilized by attaching an RNA sequence called MDV-1 to RNA complementary to a DNA sequence of interest. Upon mixing with a sample, the hybrid RNA finds its complement among the specimen's mRNAs and binds, activating the replicase to copy the tag-along sequence of interest. Another nucleic acid amplification technique, ligase chain reaction (LCR), works by using two differently labeled halves of a sequence of interest which are covalently bonded by ligase in the presence of the contiguous sequence in a sample, forming a new target. The repair chain reaction (RCR) nucleic acid amplification technique uses two complementary and target-specific oligonucleotide probe pairs, thermostable polymerase and ligase, and DNA nucleotides to geometrically amplify targeted sequences. A 2-base gap separates the oligonucleotide probe pairs, and the RCR fills and joins the gap, mimicking normal DNA repair. Nucleic acid amplification by strand displacement activation (SDA) utilizes a short primer containing a recognition site for *hincII* with short overhang on the 5' end which binds to target DNA, A DNA polymerase fills in the part of the primer opposite the overhang with sulfur-containing adenine analogs. *HincII* is added but only cuts the unmodified DNA strand. A DNA polymerase that lacks 5' exonuclease activity enters at the site of the nick and begins to polymerize, displacing the initial primer strand downstream and building a new one which serves as more primer. SDA produces greater than 10⁷-fold amplification in 2 hours at 37°C. Unlike PCR and LCR, SDA does not require instrumented Temperature cycling.

Another method is a process for amplifying nucleic acid sequences from a DNA or RNA template which may be purified or may exist in a mixture of nucleic acids. The resulting nucleic acid sequences may be exact copies of the template, or may be modified. The process has advantages over PCR in that it increases the fidelity of copying a specific nucleic acid sequence, and it allows one to more efficiently detect a particular point mutation in a single assay. A target nucleic acid is amplified enzymatically while avoiding strand displacement. Three primers are used. A first primer is complementary to the first end of the target. A second primer is complementary to the second end of the target. A third primer which is similar to the first end of the target and which is substantially complementary to at least a portion of the first primer such that when the third primer is hybridized to the first primer, the position of the third primer complementary to the base at the 5' end of the first primer contains a modification which substantially avoids strand displacement. This method is detailed in U.S. Patent 5,593,840 to Bhatnagar et al. 1997, incorporated herein by reference.

Finally, recent application of DNA chips or microarray technology where DNA or oligonucleotides are immobilized on small solid support may also be used to rapidly sequence sample BRCA2 gene and analyze its expression. Typically, high density arrays of DNA fragment are fabricated on glass or nylon substrates by *in situ* light-directed combinatorial synthesis or by conventional synthesis followed by immobilization (Fodor et al. U.S. patent No. 5,445,934). Sample DNA or RNA may be amplified by PCR, labeled with a fluorescent tag, and hybridized to the microarray. Examples of this technology are provided in U.S. Patents 5,510, 270, U.S. 5,547,839, incorporated herein by reference.

All exonic and adjacent intronic sequences of the BRCA2 gene were obtained by end to end sequencing of five normal subjects in the manner described above followed by analysis of the data obtained. The data obtained provided us with the opportunity to establish the correct intronic/exonic structure of the BRCA2 gene. In addition, we evaluated six previously published normal polymorphisms (1342, 2457, 3199, 3624, 4035, and 7470) for correctness and frequency in the population, and to identify four additional polymorphisms not previously characterized (1093, 1593, 2908, and 9079).

### GENE THERAPY

The polynucleotide(s) which result from either sense or antisense transcription of any exon or the entire coding sequence or fragments of BRCA2 gene may be used for gene therapy. A variety of methods are known for gene transfer, any of which might be available for use.

### Direct injection of Recombinant DNA in vivo:

1. Direct injection of "naked" DNA directly with a syringe and needle into a specific tissue, infused through a vascular bed, or transferred through a catheter into endothelial cells.
2. Direct injection of DNA that is contained in artificially generated lipid vesicles or other encapsulating vehicles.
3. Direct injection of DNA conjugated to a target receptor structure, such as a diptheria toxin, an antibody or other suitable receptor.
4. Direct injection by particle bombardment. For example, the DNA may be coated onto gold particles and shot into the cells.

### Human Artificial Chromosomes

The gene delivery approach involves the use of human chromosomes that have been stripped down to contain only the essential components for replication and the genes desired for transfer.

### Receptor-Mediated Gene Transfer

DNA is linked to a targeting molecule that will bind to specific cell-surface receptors, inducing endocytosis and transfer of the DNA into mammalian cells. One such technique uses poly-L-lysine to link asialoglycoprotein to DNA. An adenovirus is also added to the complex to disrupt the lysosomes and thus allow the DNA to avoid degradation and move to the nucleus. Infusion of these particles intravenously has resulted in gene transfer into hepatocytes.

### RECOMBINANT VIRUS VECTORS

Several vectors may be used in gene therapy. Among them are the Moloney Murine Leukemia Virus (MoMLV) Vectors, the adenovirus vectors, the Adeno-Associated Virus (AAV) vectors, the herpes simplex virus (HSV) vectors, the poxvirus vectors, the retrovirus vectors, and human immunodeficiency virus (HIV) vectors.

### GENE REPLACEMENT AND REPAIR

The ideal genetic manipulation for treatment of a genetic disease would be the actual replacement of the defective gene with a normal copy of the gene. Homologous recombination is the term used for switching out a section of DNA and replacing it with a new piece. By this technique, the defective gene may be replaced with a normal gene which expresses a functioning BRCA2 tumor growth inhibitor protein.

A complete description of gene therapy can also be found in "Gene Therapy A Primer For Physicians" 2d Ed. by Kenneth W. Culver, M.D. Publ. Mary Ann Liebert Inc. (1996). Two Gene Therapy Protocols for BRCA1 gene have been approved by the Recombinant DNA Advisory Committee for Jeffrey T. Holt et al. They are listed as 9602-148, and 9603-149 and are available from the NIH. Protocols for BRCA2 gene therapy may be similarly employed. The isolated BRCA2 gene may be synthesized or constructed from amplification products and inserted into a vector such as the LXSN vector.

### A BRCA2 POLYPEPTIDE OR ITS FUNCTIONAL EQUIVALENT

The growth of breast and ovarian cancer may be arrested or prevented by directly increasing the BRCA2 protein level where inadequate functional BRCA2 activity is responsible for breast and ovarian cancer. The cDNA and amino acid sequences of five novel BRCA2 haplotypes are disclosed herein (SEQ ID No:4-13). All or a fragment of BRCA2 protein may be used in therapeutic or prophylactic treatment of breast and ovarian cancer. Such a fragment may have a similar biological function as the native BRCA2 protein or may have a desired biological function as specified bellow. BRCA2 polypeptides or their functional equivalents including homologous and modified polypeptide sequences are also within the scope of the present invention. Changes in the native sequence may be advantageous in producing or using the BRCA2 derived polypeptides or functional equivalents suitable for therapeutic or prophylactic treatment of breast and ovarian cancer. For example, these changes may be desirable for producing resistance against *in vivo* proteolytic cleavage, for facilitating transportation and delivery of therapeutic reagents, for localizing and compartmentalizing tumor suppressing agents, or for expression, isolating and purifying the target species.

There are a variety of methods to produce an active BRCA2 polypeptide or a functional equivalent as a tumor growth inhibitor. For example, one or more amino acids may be substituted, deleted, or inserted using methods well known in the art (Maniatis *et al*., 1982). Considerations of polarity, charge, solubility, hydrophobicity, hydrophilicity and/or the amphiphathic nature of the amino acids play an important role in designing homologous polypeptide changes suitable for the intended treatment. In particular, conservative amino acid substitution using amino acids that are related in side-chain structure and charge may be employed to preserve the chemical and biological property. A homologous polyeptide typically contains at least 70% homology to the native sequence. Unnatural forms of the polypeptide may also be incorporated so long as the modification retains substantial biological activity. These unnatural polypeptides typically include structural mimics and chemical medications, which have similar three-dimensional structures as the active regions of the native BRCA2 protein. For example, these modifications may include terminal D-amino acids, cyclic peptides, unnatural amino acids side chains, pseudopeptide bonds, N-terminal acetylation, glycosylation, and biotinylation, etc. These unnatural forms of polypeptide may have a desired biological function, for example, they may be particularly robust in the presence of cellular or serum proteases and exopeptidase. An effective BRCA2 polypeptide or a functional equivalent may also be recognized by the reduction of the native BRCA2 protein. Regions of the BRCA2 protein may be systematically deleted to identify which regions are essential for tumor growth inhibitor activity. These smaller fragments of BRCA2 protein may then be subjected to structural and functional modification to derive therapeutically or prophylactically effective regiments. Finally, drugs, natural products or small molecules may be screened or synthesized to mimic the function of the BRCA2 protein. Typically, the active species retain the essential three-dimensional shape and chemical reactivity, and therefore retain the desired aspects of the biological activity of the native BRCA2 protein. The activity and function of BRCA2 may include transactivation, granin, DNA repair among others. Functions of BRCA2 protein are also reviewed in Bertwistle and Ashworth, Curr. Opin. Genet. Dev. 8(1): 14-20 (1998) and Zhang et al., Cell 92:433-436 (1998). It will be apparent to one skilled in the art that a BRCA2 polypeptide or a functional equivalent may be selected because such polypeptide or functional equivalent possesses similar biological activity as the native BRCA2 protein.

### EXPRESSION OF THE BRCA2 PROTEIN AND POLYPEPTIDE IN HOST CELLS

All or fragments of the BRCA2 protein and polypeptide may be produced by host cells that are capable of directing the replication and the expression of foreign genes. Suitable host cells include prokaryotes, yeast cells, or higher eukaryotic cells, which contain an expression vector comprising all or a fragment of the BRCA2 cDNA sequence (SEQ. ID No: 4, 6, 8, 10, or 12) operatively linked to one or more regulatory sequences to produce the intended BRCA2 protein or polypeptide. Prokaryotes may include gram negative or gram positive organisms, for example *E*. *coli* or *Bacillus* strains. Suitable eukaryotic host cells may include yeast, virus, and mamalian systems. For example, Sf9 insect cells and human cell lines, such as COS, MCF7, HeLa, 293T, HBL100, SW480, and HCT116 cells.

A broad variety of suitable expression vectors are available in the art. An expression vector typically contains an origin of replication, a promoter, a phenotypic selection gene (antibiotic resistance or autotrophic requirement), and a DNA sequence coding for all or fragments of the BRCA2 protein. The expression vectors may also include other operatively linked regulatory DNA sequences known in the art, for example, stability leader sequences, secretory leader sequences, restriction enzyme cleavage sequences, polyadenylation sequences, and termination sequences, among others. The essential and regulatory elements of the expression vector must be compatible with the intended host cell. Suitable expression vectors containing the desired coding and control regions may be constructed using standard recombinant DNA techniques known in the art, many of which are described in Sambrook, et al., Molecular Cloning: A Laboratory Manual, Second Edition, Cold Spring Harbor Laboratory, Cold Spring Harbor, N.Y. (1989). For example, suitable origins of replication may include Col E1, SV4O viral and M13 origins of replication. Suitable promoters may be constitutive or inducible, for example, tac promoter, lac Z promoter, SV40 promoter, MMTV promoter, and LXSN promoter. Examples of selectable markers include neomycin, ampicillin, and hygromycin resistance and the like. Many suitable prokaryotic, viral and mammalian expression vectors may be obtained commercially, for example, from Invitrogen Corp., San Diego, CA or from Clontech, Palo Alto, CA. It may be desirable that the BRCA2 protein or polypeptide is produced as a fusion protein to enhance the expression in selected host cells, to detect the expression in transfected cells, or to simplify the purification process. Suitable fusion partners for the BRCA2 protein or polypeptide are well known in the art and may include β-galactosidase, glutathione-S-transferase, and poly-histidine tag.

Expression vectors may be introduced into host cells by various methods known in the art. The transformation procedure used depends upon the host to be transformed. Methods for introduction of vectors into host cells may include calcium phosphate precipitation, electrosporation, dextran-mediated transfection, liposome encapsulation, nucleus microinjection, and viral or phage infection, among others.

Once an expression vector has been introduced into a suitable host cell, the host cell may be cultured under conditions permitting expression of large amounts of the BRCA2 protein or polypeptide. The expression product may be identified by many approaches well known in the art, for example, sequencing after PCR-based amplification, hybridization using probes complementary to the desired DNA sequence, the presence or absence of marker gene functions such as enzyme activity or antibiotic resistance, the level of mRNA production encoding the intended sequence, immunological detection of a gene product using monoclonal and polyclonal antibodies, such as Western blotting or ELISA. The BRCA2 protein or polypeptides produced in this manner may then be isolated following cell lysis and purified using various protein purification techniques known in the art, for example, ion exchange chromatography, gel filtration chromatography and immunoaffinity chromatography.

It is generally preferred that whenever possible, longer fragments of BRCA2 protein or polypeptide are used, particularly to include the desired functional domains of BRCA2 protein. Expression of shorter fragments of DNA may be useful in generating BRCA2 derived immunogen for the production of anti-BRCA2 antibodies. It should, of course, be understood that not all expression vectors, DNA regulatory sequences or host cells will function equally well to express the BRCA2 protein or polypeptides of the present invention. However, one of ordinary skill in the art may make a selection among expression vectors, DNA regulatory sequences, host cells, and codon usage in order to optimize expression using known technology in the art without undue experimentation. Studies of BRCA2 protein function and examples of genetic manipulation of BRCA2 protein are summarized in two recent review articles, Bertwistle and Ashworth, Curr. Open. Genet. Dev. 8(1): 14-20 (1998) and Zhang et al., Cell 92:433-436 (1998).

### IN VITRO SYNTHESIS AND CHEMICAL SYNTHESIS

Although it is preferred that fragments of the BRCA2 protein or polypeptides be obtained by overexpression in prokaryotic or eukaryotic host cells, the BRCA2 polypeptides or their functional equivalents may also be obtained by *in vitro* translation or synthetic means by methods known to those of ordinary skill in the art. For example, *in vitro* translation may employ an mRNA encoded by a DNA sequence coding for fragments of the BRCA2 protein or polypeptides. Chemical synthesis methodology such as solid phase synthesis may be used to synthesize a BRCA2 polypeptide structural mimic and chemically modified analogs thereof. The polypeptides or the modifications and mimic thereof produced in this manner may then be isolated and purified using various purification techniques, such as chromatographic procedures including ion exchange chromatography, gel filtration chromatography and immunoaffinity chromatography.

### PROTEIN REPLACEMENT THERAPY

The tumor suppressing function of BRCA2 suggests that various BRCA2 protein targeted therapies may be utilized in treating and preventing tumors in breast and ovarian cancer. The present invention therefore includes therapeutic and prophylactic treatment of breast and ovarian cancer using therapeutic pharmaceutical compositions containing the BRCA2 protein, polypeptides, or their functional equivalents. For example, protein replacement therapy may involve directly administering the BRCA2 protein, a BRCA2 polypeptide, or a functional equivalent in a pharmaceutically effective carrier. Alternatively, protein replacement therapy may utilize tumor antigen specific antibody fused to fragments of the BRCA2 protein, a polypeptide, or a functional equivalent to deliver anti-cancer regiments specifically to the tumor cells.

To prepare the pharmaceutical compositions of the present invention, an active BRCA2 protein, a BRCA2 polypeptide, or its functional equivalent is combined with a pharmaceutical carrier selected and prepared according to conventional pharmaceutical compounding techniques. A suitable amount of the composition may be administered locally to the site of a tumor or systemically to arrest the proliferation of tumor cells. The methods for administration, may include parenteral, oral, or intravenous, among others according to established protocols in the art.

Pharmaceutically acceptable solid or liquid carriers or components which may be added to enhance or stabilize the composition, or to facilitate preparation of the composition include, without limitation, syrup, water, isotonic solution, 5 % glucose in water or buffered sodium or ammonium acetate solution, oils, glycerin, alcohols, flavoring agents, preservatives, coloring agents, starches, sugars, diluents, granulating agents, lubricants, binders, and sustained release materials. The dosage at which the therapeutic compositions are administered may vary within a wide range and depends on various factors, such as the stage of cancer progression, the age and condition of the patient, and may be individually adjusted.

### DIAGNOSTIC REAGENTS

The BRCA2 protein, polypeptides, their functional equivalents, antibodies, and polynucleotides may be used in a wide variety of ways in addition to gene therapy and protein replacement therapy. They may be useful as diagnostic reagents to measure normal or abnormal activity of BRCA2 at the DNA, RNA, and protein level. The present invention therefore encompasses the diagnostic reagents derived from the BRCA2 cDNA and protein sequences as set forth in SEQ. ID. Nos: 4-13. These reagents may be utilized in methods for monitoring disease progression, for determining patients suited for gene and protein replacement therapy, or for detecting the presence or quantifying the amount of a tumor growth inhibitor following such therapy. Such methods may involve conventional histochemical techniques, such as obtaining a tumor tissue from the patient, preparing an extract and testing this extract for tumor growth or metabolism. For example, the test for tumor growth may involve measuring abnormal BRCA2 activity using conventional diagnostic assays, such as Southern, Northern, and Western blotting, PCR, RT-PCR, and immunoprecipitation. In biopsies of tumor tissues, the loss of BRCA2 expression in tumor tissue may be verified by RT-PCR and Northern blotting at the RNA level. A Southern blot analysis, genomic PCR, or fluorescence in situ hybridization (FISH) may also be performed to examine the mutations of BRCA2 at the DNA level. And, a Western blotting, protein truncation assay, or immunoprecipitation may be utilized to analysis the effect at the protein level.

These diagnostic reagents are typically either covalently or non convalently attached to a detectable label. Such a label includes a radioactive label, a colorimetric enzyme label, a fluorescence label, or an epitope label. Frequently, a reporter gene downstream of the regulatory sequences is fused with the BRCA2 protein or polypeptide to facilitate the detection and purification of the target species. Commonly used reporter genes in BRCA2 fusion proteins include β-galactosidase and luciferase gene.

The BRCA2 protein, polypeptides, their functional equivalents, antibodies, and polynucleotides may also be useful in the study of the characteristics of BRCA2 proteins, such as structure and function of BRCA2 in oncogenesis or subcellular localization of BRCA2 protein in normal and cancerous cell. For example, yeast two-hybrid system has been used in the study of cellular function of BRCA2 to identify the regulator and effector of BRCA2 tumor suppressing function (Sharan et al., Nature 386:804-810 (1997) and Katagiri et al., Genes, Chromosomes & Cancer 21:217-222 (1988)). In addition, the BRCA2 protein, polypeptides, their functional equivalents, antibodies, and polynucleotides may also be used in *in vivo* cell based and *is vitro* cell free assays to screen natural products and synthetic compounds which may mimic, regulate or stimulate BRCA2 protein function.

### ANTISENSE INHIBITION

Antisense suppression of endogenous BRCA2 expression may assess the effect of BRCA2 protein on cell growth inhibition using known method in the art (Crooke, Annu. Rev. Pharmacol. Toxicol. 32:329-376 (1992) and Robinson-Benion and Holt, Methods Enzymol. 254:363-375 (1995)). Given the cDNA sequence as set forth in SEQ ID. NO: 4, 6, 8, 10, and 12, one of skill in the art can readily obtain anti-sense strand of DNA and RNA sequences to interfere with the production of wild-type BRCA2 protein or the mutated form of BRCA2 protein. Alternatively, antisense oligonucleotide may be designed to target the control sequences of BRCA2 gene to reduce or prevent the expression of the endogenous BRCA2 gene.

### ANTIBODIES

The BRCA2 protein, polypeptides, or their functional equivalents may be used as immunogens to prepare polyclonal or monoclonal antibodies capable of binding the BRCA2 derived antigens in a known manner (Harlow & Lane, Antibodies: A Laboratory Manual, Cold Spring Harbor Laboratory, Cold Spring Harbor, NY, 1988). These antibodies may be used for the detection of the BRCA2 protein, polypeptides, or a functional equivalent in an immunoassay, such as ELISA, Western blot, radioimmunoassay, enzyme immunoassay, and immunocytochemistry. Typically, an anti-BRCA2 antibody is in solution or is attached to a solid surface such as a plate, a particle, a bead, or a tube. The antibody is allowed to contact a biological sample or a blot suspected of containing the BRCA2 protein or polypeptide to form a primary immunocomplex. After sufficient incubation period, the primary immunocomplex is washed to remove any non-specifically bound species. The amount of specifically bound BRCA2 protein or polypeptide may be determined using the detection of an attached label or a marker, such as a radioactive, a fluorescent, or an enzymatic label. Alternatively, the detection of BRCA2 derived antigen is allowed by forming a secondary immunocomplex using a second antibody which is attached with a such label or marker. The antibodies may also be used in affinity chromatography for isolating or purifying the BRCA2 protein, polypeptides or their functional equivalents.

### EXAMPLE 1

### Determination of the Coding Sequence Haplotypes of the BRCA2 Gene From Normal Individuals

Approximately 150 volunteers were screened in order to identify individuals with no cancer history in their immediate family (i.e. first and second degree relatives). Each person was asked to fill out a hereditary cancer prescreening questionnaire (See TABLE I). Five of these were randomly chosen for end-to-end sequencing of their BRCA2 gene. A first degree relative is a parent, sibling, or offspring. A second degree relative is an aunt, uncle, grandparent, grandchild, niece, nephew, or half-sibling.

Genomic DNA was isolated from white blood cells of five normal subjects selected from analysis of their answers to the questions above. Dideoxy sequence analysis was performed following polymerase chain reaction amplification.

All exons of the BRCA2 gene were subjected to direct dideoxy sequence analysis by asymmetric amplification using the polymerase chain reaction (PCR) to generate a single stranded product amplified from this DNA sample. Shuldiner, et al., Handbook of Techniques in Endocrine Research, p. 457-486, DePablo, F., Scanes, C., eds., Academic Press, Inc., 1993. Fluorescent dye was attached for automated sequencing using the Taq Dye Terminator Kit (Perkin-Elmer^{®} cat# 401628). DNA sequencing was performed in both forward and reverse directions on an Applied Biosystems, Inc. (ABI) automated sequencer (Model 377). The software used for analysis of the resulting data was "Sequence Navigator" purchased through ABI,

### 1. Polymerase Chain Reaction PCR Amplification

Genomic DNA (100 nanograms) extracted from white blood cells of five normal subjects. Each of the five samples was sequenced end to end. Each sample was amplified in a final volume of 25 microliters containing 1 microliter (100 nanograms) genomic DNA, 2.5 microliters 10X PCR buffer (100 mM Tris, pH 8.3, 500 mM KCl, 1.2 mM MgCl₂), 2.5 microliters 10X dNTP mix (2 mM each nucleotide), 2.5 microliters forward primer, 2.5 microliters reverse primer, and 1 microliter Taq polymerase (5 units), and 13 microliters of water.

The primers in TABLE II below were used to carry out amplification of the various sections of the BRCA2 gene samples. The primers were synthesized on an DNA/RNA Synthesizer Model 394^{®}.

Thirty-five cycles were performed, each consisting of denaturing (95°C; 30 seconds), annealing (55°C; 1 minute), and extension (72°C; 90 seconds), except during the first cycle in which the denaturing time was increased to 5 minutes, and during the last cycle in which the extension time was increased to 5 minutes.

PCR products were purified using Qia-quick^{®} PCR purification kits (Qiagen^{®}, cat# 28104; Chatsworth, CA). Yield and purity of the PCR product are determined spectrophotometrically at OD₂₆₀ on a Beckman DU 650 spectrophotometer.

### 2. Dideoxy Sequence Analysis

Fluorescent dye was attached to PCR products for automated sequencing using the Taq Dye Terminator Kit (Perkin-Elmer^{®} cat # 401628). DNA sequencing was performed in both forward and reverse directions on an Applied Biosystems, Inc. (ABI) Foster City, CA., automated sequencer (Model 377). The software used for analysis of the resulting data was "Sequence Navigator^{®}" purchased through ABI.

### 3. RESULTS

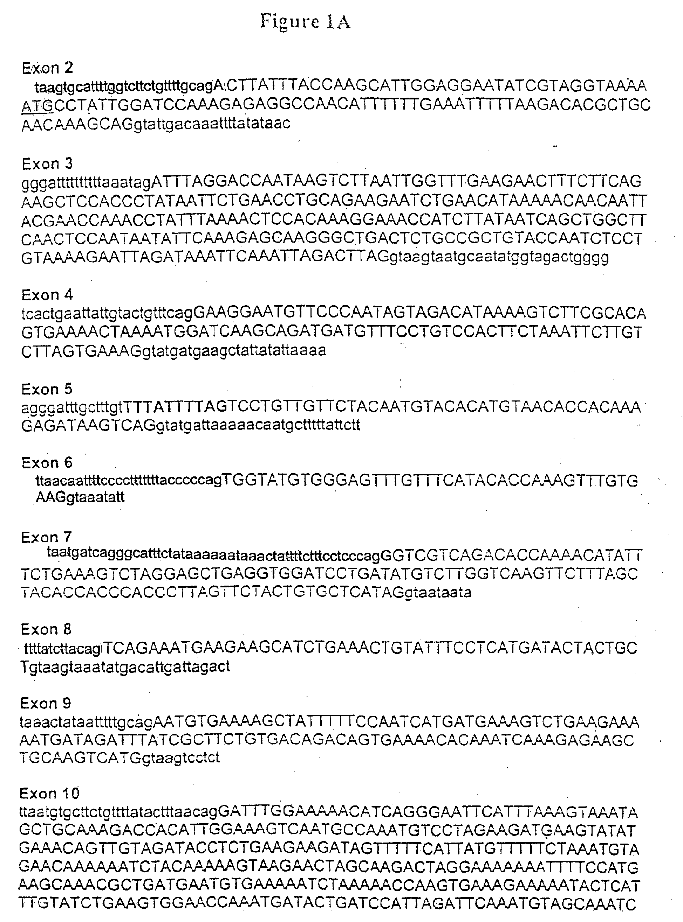

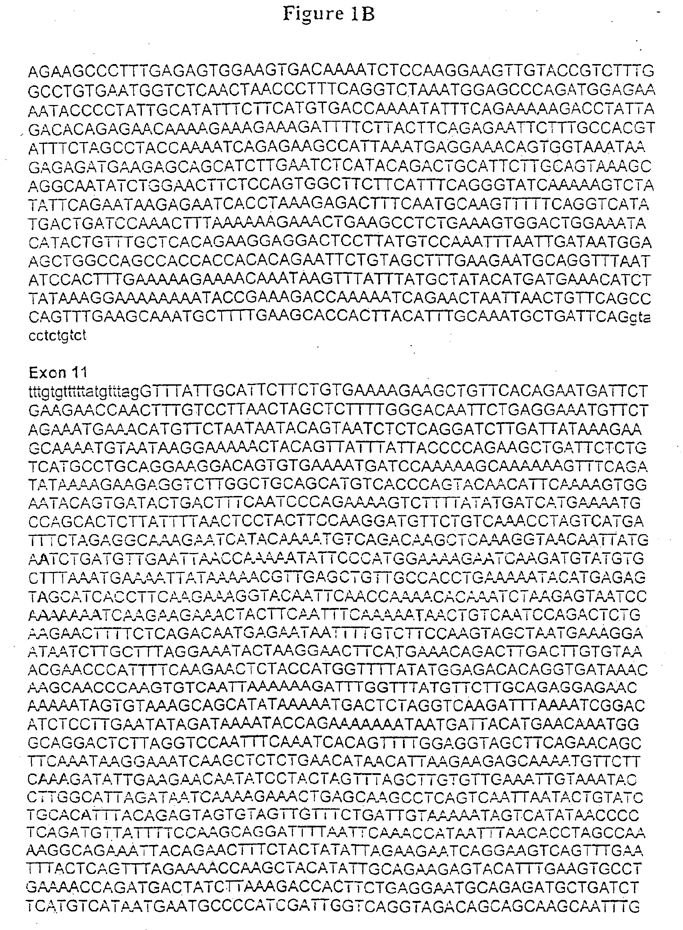

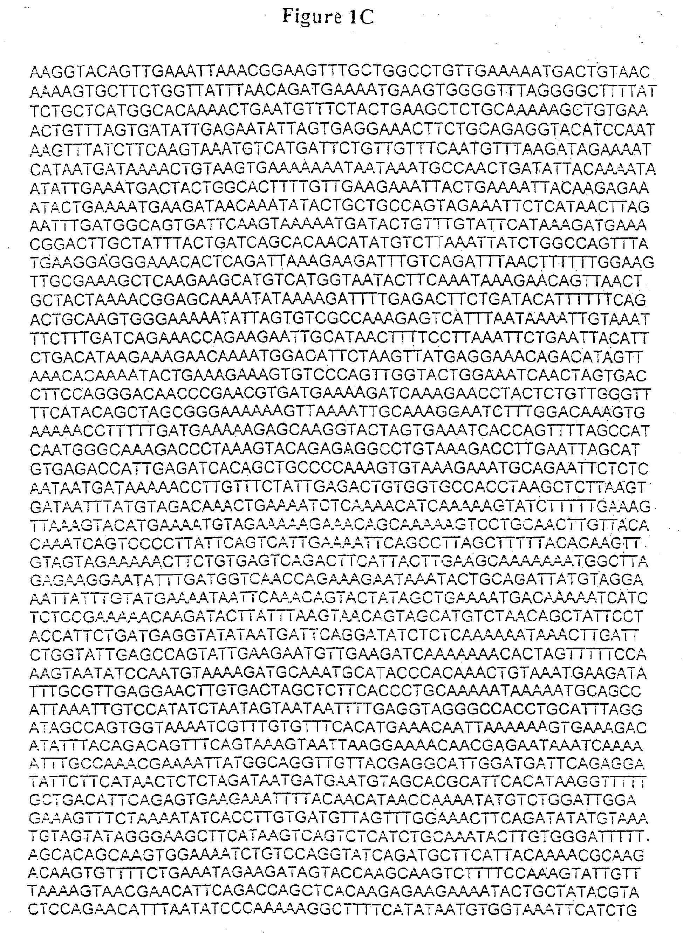

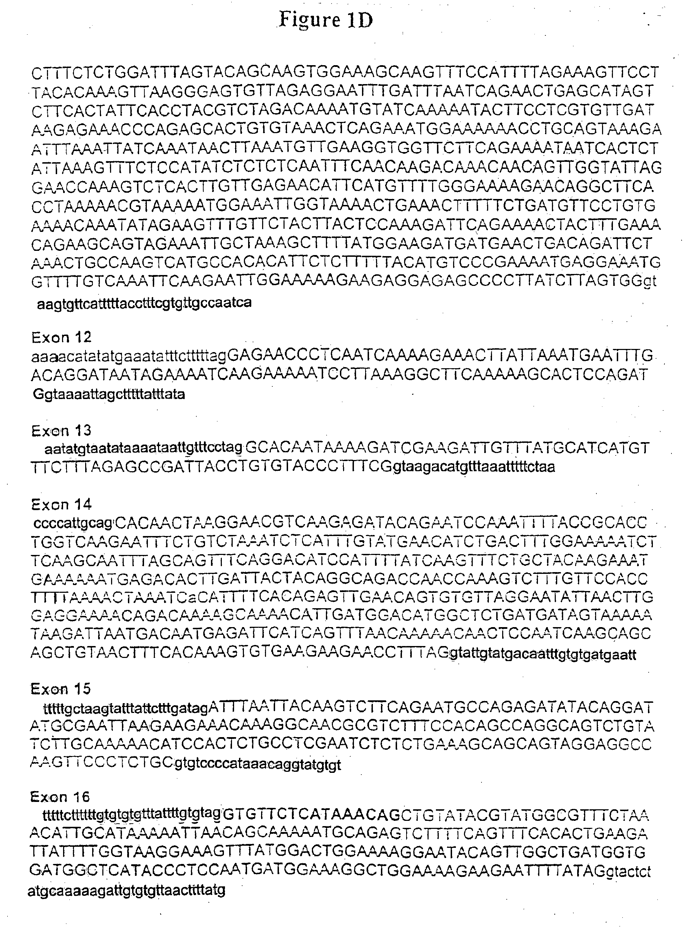

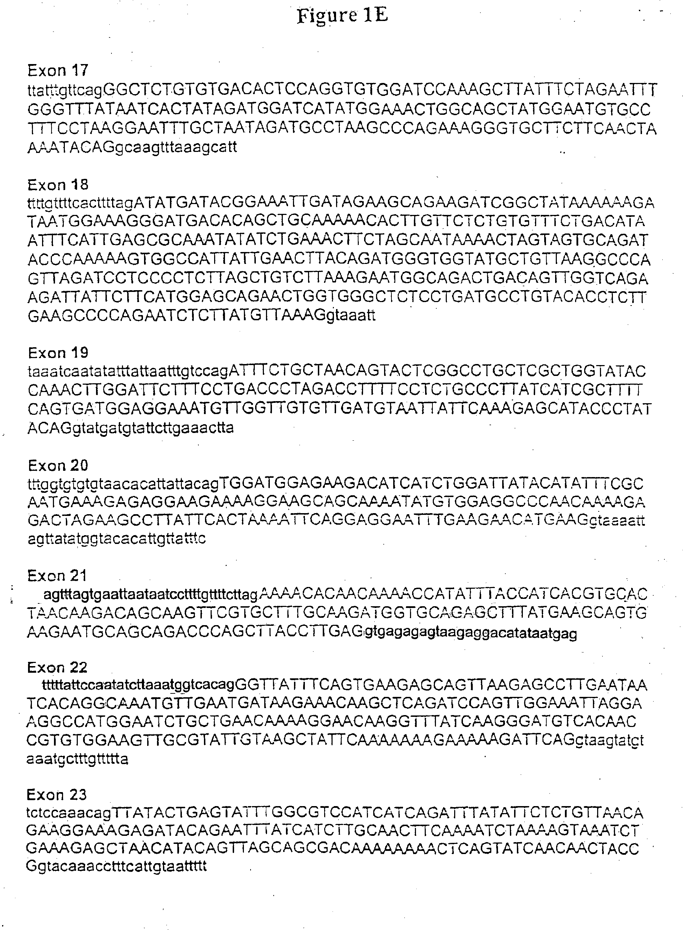

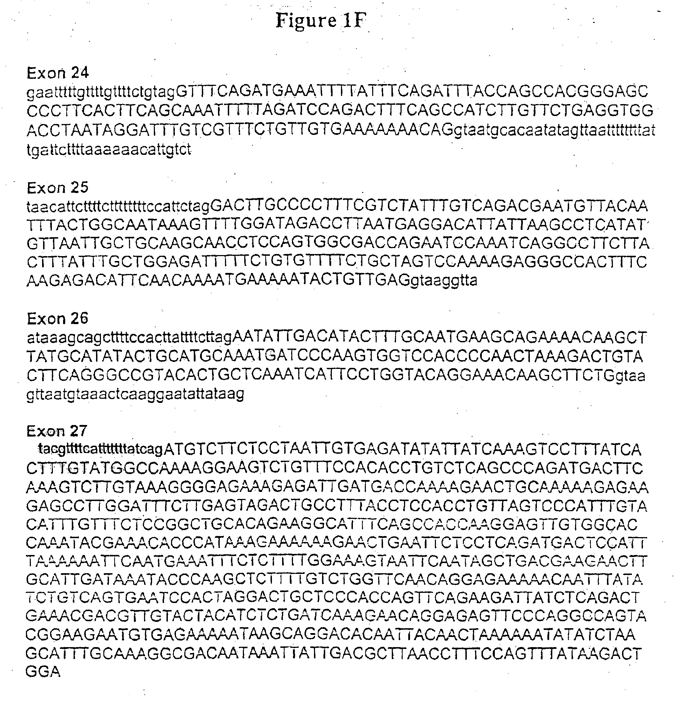

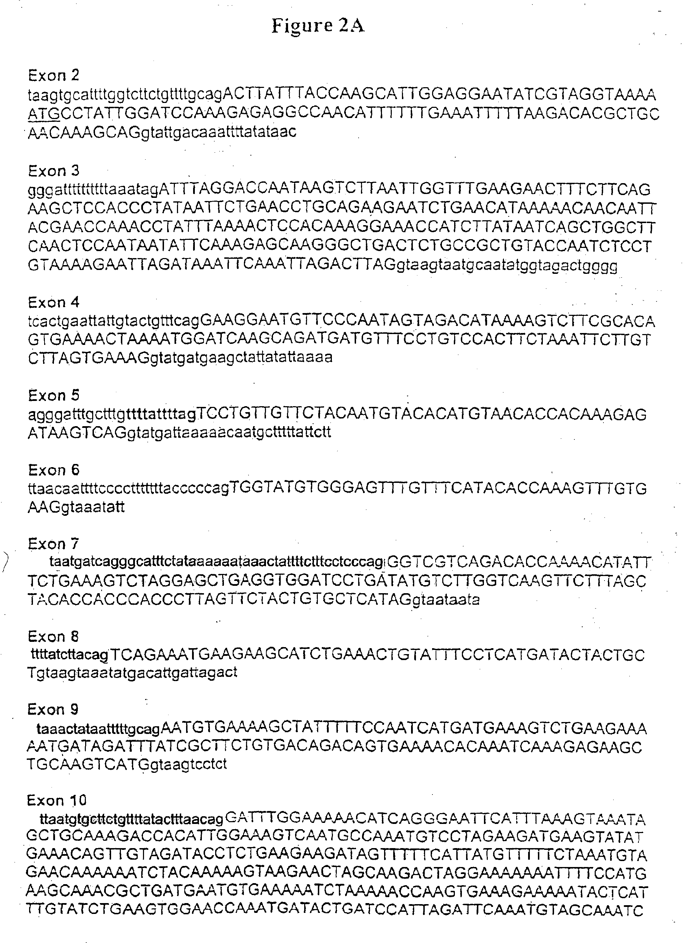

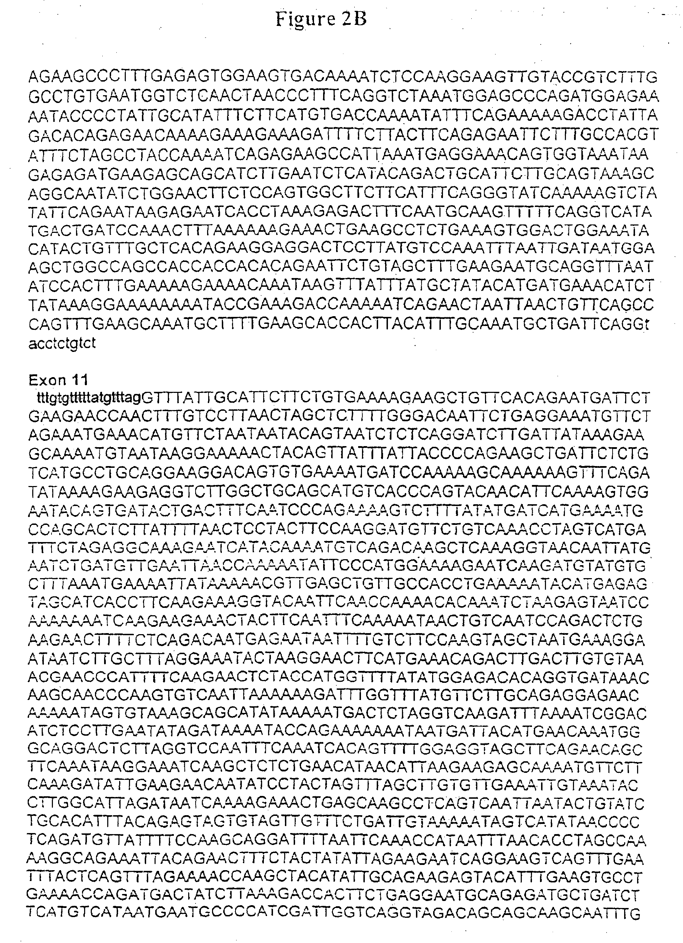

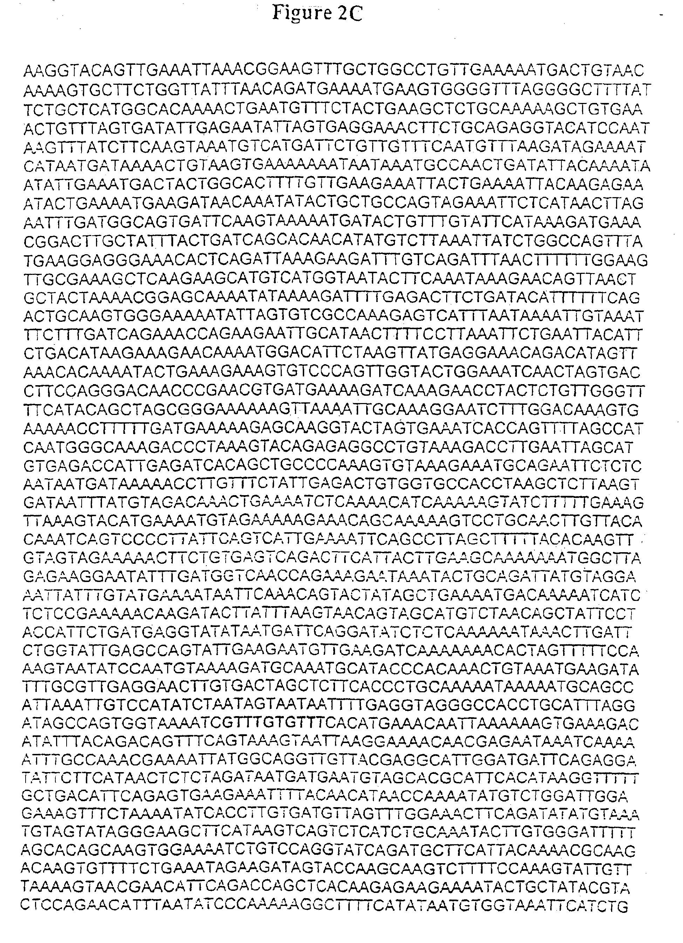

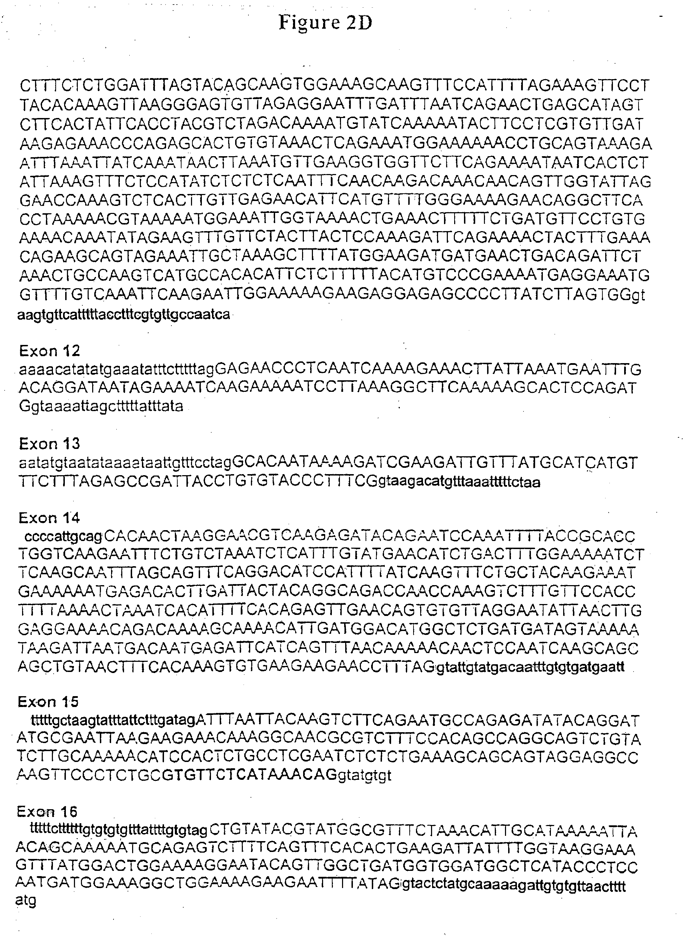

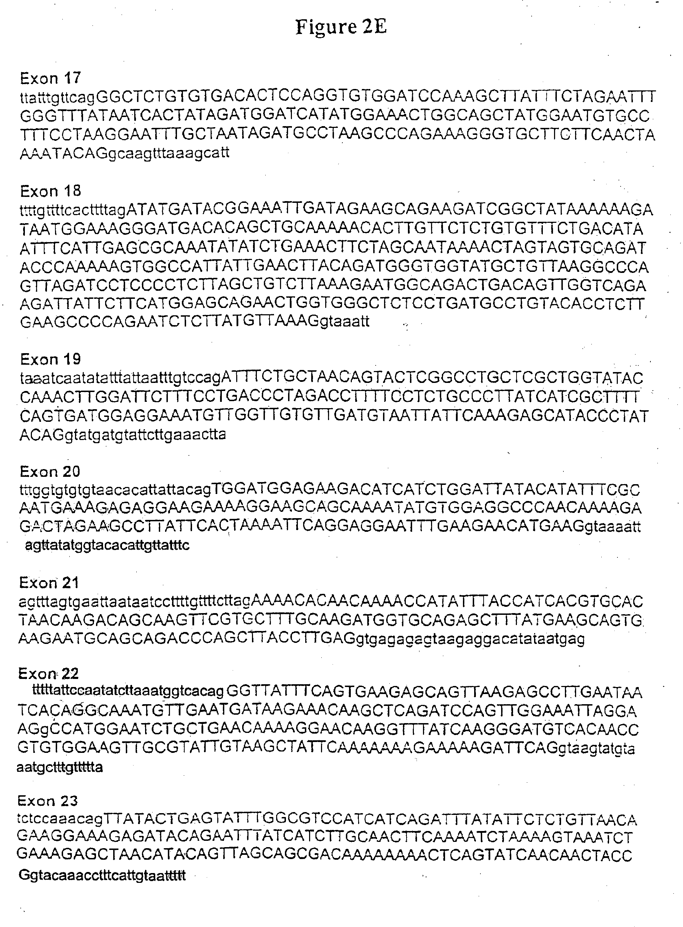

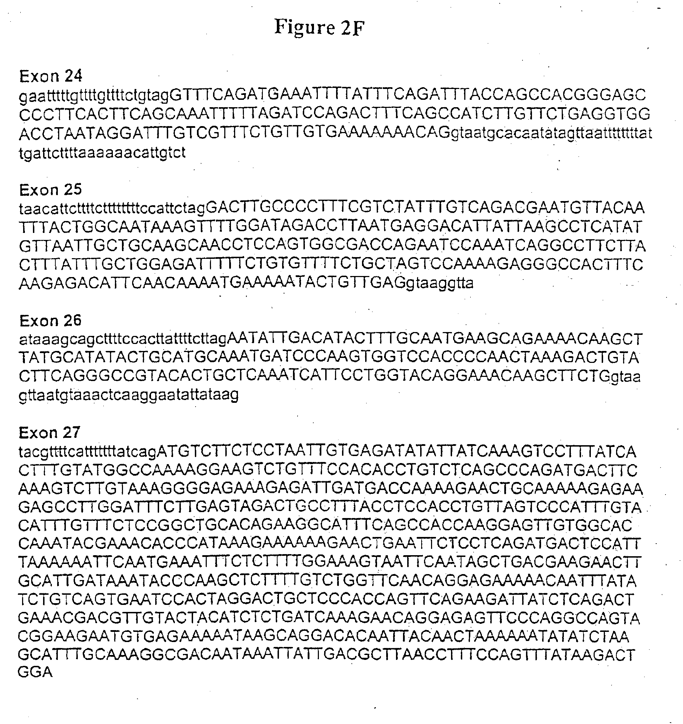

Based upon the sequencing of the five normal individuals, it was determined that the standard sequence found in both GenBank and BIC were inaccurate. In Genbank, a 10 bp stretch (5'-TTTATTTTAG-3') was mistakenly listed as exonic at the 5' end of exon 5 while it should be intronic which would not be included in the cDNA and resultant protein. In addition, a more detrimental error that has the significant potential to lead to an incorrect diagnosis of breast cancer propensity exists in both Genbank and BIC: a sequence of 16 bp (5'-GTGTTCTCATAAACAG-3') should be at the end of exon 15, but instead is listed at the beginning of exon 16 in the database. The disclosure and listing of GenBank is shown in Figure 1. The correct intron/exon sequence of BRCA2 is presented in Figure 2, wherein,
(1) a 10 bp stretch (5'-TTTATTTTAG-3') is intronic at 3' end of intron 4, rather than at the 5' end of exon 5 (corrected exon 5 is listed as SEQ. ID. NO: 1) and
(2) a 16 bp stretch (5'-GTGTTCTCATAAACAG-3') is exonic at the 3' end of exon 15, rather than at the 5' end of exon 16 (corrected exons 15 and 16 are listed as SEQ. ID. No: 2 and 3 respectively)

The BIC BRCA2 sequence also contains sequence errors in which a strech of nine nucleotides at positions 5554-5460 is listed as CGTTTGTGT (amino acids: Arg-Leu-Cys). The correct sequence at these positions is GTTTGTGTT (amino acids: Val-Cys-Val). In addition, the BIC BRCA2 nuclotides at positions 2024 (codon 599), 4553 (codon 1442), 4815 (codon 1529), 5841 (codon 1871), and 5972 (codon 1915) are T, T, A, C, and T respectively, wherein the correct nucleotides at these positions are C, C, G, T, and C respectively. Among them, the nuclotide errors at codon 599, 1442, 1915 result in amino acids changes.

Additional differences in the nucleic acids of the five normal individuals were found in ten polymorphic locations. The changes and their positions are found in TABLE III. The individual haplotypes of each chromosome of BRCA2 are displayed in FIGURE 3. In each case, the initial haplotype reported in Genbank (accession number U43746) was subtracted to determine the new haplotypes OMI 1-5. Thus, the Genbank sequence only represents 50% of the haplotypes found; the five new BRCA2 ^{(omi1-5)} DNA sequences are shown as SEQ. ID. NO: 4, 6, 8, 10, and 12, respectively (See FIGURE 3), and the corresponding polypeptides are listed as SEQ. ID. NO: 5, 7, 9, 11, and 13 respectively. In combination, these seven haplotypes represent a functional allele profile for the BRCA2 gene.

The data show that for each of the samples, all exons of BRCA2 were identical except in the region of ten polymorphisms. Six of these polymorphisms were previously identified (Tartigan et al., Nature Genetics 12: 333-337 (1996); Phelan et al, Nature Genetics 13: 120-122 (1996); Couch ef al., Nature Genetics 13: 123-125 (1996); Teng, et al., Nature Genetics 13: 241-244 (1996); Schubert *et al* 60: 1031-1040 (1997)), but four where unique to this work. Even though the individual polymorphisms may have been identified, none of these complete haplotypes has been previously determined.

**TABLE I**

| **Hereditary Cancer Pre-Screening Questionnaire** | | | |
|---|---|---|---|
| **Part A:** | Answer the following questions about your family | | |
| 1. | To your knowledge, has anyone in your family been diagnosed with a very specific hereditary colon disease called Familial Adenomatous Polyposis (FAP)? | | |
| 2. | To your knowledge, have you or any aunt had breast cancer diagnosed before the age 35? | | |
| 3. | Have you had Inflammatory Bowel Disease, also called Crohn's Disease or Ulcerative Colitis, for more than 7 years? | | |

| **Part B:** | Refer to the list of cancers below for vour responses only to questions in Part B | | |
|---|---|---|---|
| | Bladder Cancer | Lung Cancer | Pancreatic Cancer |
| | Breast Cancer | Gastric Cancer | Prostate Cancer |
| | Colon Cancer | Malignant Melanoma | Renal Cancer |
| | Endometrial Cancer | Ovarian Cancer | Thyroid Cancer |
| | | | |
| 4. | Have your mother or father, your sisters or brothers or your children had any of the listed cancers? | | |
| 5 | Have there been diagnosed in your mother's brothers or sisters, or your mother's parents more than one of the cancers in the above list? | | |
| 6. | Have there been diagnosed in your father's brothers or sisters, or your father's parents more than one of the cancers in the above list? | | |

| **Part C:** | Refer to the list of relatives below for responses only to questions in Part C | | |
|---|---|---|---|
| | You | Your mother | |
| | Your sisters or brothers | Your mother's sisters or brothers | (maternal aunts & uncles) |
| | Your children | Your mother's parents (maternal | grandparents) |
| 7. | Have there been diagnosed in these relatives 2 or more identical types of cancer? Do not count "sample" skin cancer, also called basal cell or squamous cell skin cancer | | |
| 8. | Is there a total of 4 or more of any cancers in the list of relatives above other than "simple" skin cancers? | | |

| **Part D:** | Refer to the list of relatives below for responses only to questions in Part D. | | |
|---|---|---|---|
| | You | Your father | |
| | Your sisters or brothers | Your father's sisters or brothers (paternal aunts and uncles) | |
| | Your children | Your father's parents (paternal grandparents) | |
| 9. | Have there been diagnosed in these relatives 2 or more identical types of cancer? Do not count "sample" skin cancer, also called basal cell or squamous cell skin cancer. | | |
| 10. | Is there a total of 4 or more of any cancers in the list of relatives above other than "simple" skin cancers? | | |

| | | | |
|---|---|---|---|
| © Copyright 1996, OncorMed, Inc. | | | |

**TABLE II**

| **BRCA2 PRIMER SEQUENCES** | | | | | |
|---|---|---|---|---|---|
| | | **SEQUENCE (5' TO 3') NOTE:** | | | |
| **Exon** | **Label** | **M13 TAIL INCLUDED** | **Oligo Length** | **PCR Product Length** | **SEQ. ID. Number** |
| | | M13 FORWARD = TGT AAA ACG ACG GCC AGT | | | |
| | | M13 REVERSE = CAG GAA ACA GCT ATG ACC | | | |
| 2 | BRCA2-2F | 5'-TGA GTT TTA CCT CAG TCA CA-3' | 20 | 263 | 14 |
| 2 | BRCA2-2R/M 13R | 5'-CAG GAA ACA GCT ATG ACC CTG TGA CGT ACT GGG TTT TTA GC-3' | 41 | | 15 |
| 3 | BRCA2-3FII | 5'-GAT CTT TAA CTG TTC TGG GTC ACA-3' | 24 | 364 | 16 |
| 3 | BRCA2-3RII | 5'-CCC AGC ATG ACA CAA TTA ATG A-3' | 22 | | 17 |
| 4 | BRCA2-4F/M 13F | 5'-TGT AAA ACG ACG GCC AGT AGA ATG CAA ATT TAT AAT CCA GAG TA-3' | 44 | 268 | 18 |
| 4 | BRCA2-4R-1A | 5'-ATC AGA TTC ATC TTT ATA GAA C-3' | 22 | | 19 |
| 5&6 | BRCA2-5+6F/M13F | 5'-TGT AAA ACG ACG GCC AGT TGT GTT GGC ATT TTA AAC ATG A-3' | 40 | 453 | 20 |
| 5&6 | BRCA2-5+6R/M13R | 5'-CAG GAA ACA GCT ATG ACC CAG GGC AAA GGT ATA ACG CT-3' | 38 | | 21 |
| 7 | BRCA2-7F/M13F | 5-TGT AAA ACG AGG GCC AGT TAA GTG AAA TAA AGA GTG AA-3' | 38 | 248 | 22 |
| 7 | BRCA2-7R/M13R | 5'-CAG GAA ACA GCT ATG ACC AGA AGT ATT AGA GAT GAC-3' | 36 | | 23 |
| 8 | BRCA2-8F/M13F | 5'-TGT AAA ACG ACG GCC AGT GCC ATA TCT TAC CAC CTT GTG A-3' | 40 | 319 | 24 |
| | BRCA2-8FIA | 5'-TTG CAT TCT AGT GAT AAT ATA C-3' | 22 | 143 | 25 |
| 8 | BRCA2-8RIA | 5'-AAT TGT TAG CAA TTT CAA C-3' | 19 | | 26 |
| 9 | BRCA2-9F/M13F | 5'-TGT AAA ACG ACG GCC AGT TGG ACC TAG GTT GAT TGC AGA T-3' | 40 | 338 | 27 |
| 9 | BRCA2-9R/M13R | 5'-CAG GAA ACA GCT ATG ACC TAA ACT GAG ATC ACG GGT GAC A-3' | 40 | | 28 |
| 10A | BRCA2-10AF | 5'-GAA TAA TAT AAA TTA TAT GGC TTA-3' | 24 | 255 | 29 |
| 10A | BRCA2-10AR/M13R | 5'-CAG GAA ACA GCT ATG ACC CCT AGT CTT GCT AGT TCT T-3' | 37 | | 30 |
| 10B | BRCA2-10BF/M13F | 5'-TGT AAA ACG ACG GCC AGT ARC TGA AGT GGA ACC AAA TGA TAC-3' | 42 | 621 | 31 |
| 10B | BRCA2-10BR/M13R | 5'- CAG GAA ACA GCT ATG ACC ACG TGG CAA AGA ATT CTC TGA AGT AA-3' | 44 | | 32 |
| 10C | BRCA2-10CF/M13F | 5'-TGT AAA ACG ACG GCC AGT CAG CAT CTT GAA TCT CAT ACA G-3' | 40 | 508 | 33 |
| 10C | BRCA2-10CRII | 5'-AGA CAG AGG TAC CTG AAT C-3' | 19 | | 34 |
| 11 | BRCA2-11AF-M13 | 5'- TGT AAA ACG ACG GCC AGT TGG TAC TTT AAT TTT GTC ACT T-3' | 40 | 304 | 35 |
| 11 | BRCA2-11AR-M13 | 5'-CAG GAA ACA GCT ATG ACC TGC AGG CAT GAC AGA GAA T-3' | 37 | | 36 |
| 11 | BRCA22-11BF | 5'-AAG AAG CAA AAT GTA ATA AGG A-3' | 22 | 411 | 37 |
| 11 | BRCA2-11BR | 5'-CAT TTA AAG CAC ATA CAT CTT G-3' | 22 | | 38 |
| 11 | BRCA2-11CF | 5'-TCT AGA GGC AAA GAA TCA TAC-3' | 21 | 349 | 39 |
| 11 | BRCA2-11CR | 5'-CAA GAT TAT TCC TTT CAT TAG C-3' | 22 | | 40 |
| 11 | BRCA2-11DF | 5'-AAC CAA AAC ACA AAT CTA AGA G-3' | 22 | 344 | 41 |
| 11 | BRCA2-11DR | 5'-GTC ATT TTT ATA TGC TGC TTT AC-3' | 23 | | 42 |
| 11 | BRCA2-11EF | 5'-GGT TTT ATA TGG AGA CAC AGG-3' | 21 | 369 | 43 |
| 11 | BRCA2-11ER | 5'-GTA TTT ACA ATT TCA ACA CAA GC-3' | 23 | | 44 |
| 11 | BRCA2-11FF | 5'-ATC ACA GTT TTG GAG GTA GC-3' | 20 | 368 | 45 |
| 11 | BRCA2-11FR | 5'-CTG ACT TCC TGA TTC TTC TAA-3' | 21 | | 46 |
| 11 | BRCA2-11GF | 5'-CTC AGA TGT TAT TTT CCA AGC-3' | 21 | 366 | 47 |
| 11 | BRCA2-11GR | 5'-CTG TTA AAT AAC CAG AAG CAC-3' | 21 | | 48 |
| 11 | BRCA2-11HF | 5'-AGGTAGACAGCAGCAAGC-3' | 18 | 360 | 49 |
| 11 | BRCA2-11HR | 5'-GTA ATA TCA GTT GGC ATT TAT T-3' | 22 | | 50 |
| 11 | BRCA2-11IF | 5'-TGC AGA GGT ACA TCC AAT AAG-3' | 21 | 326 | 51 |
| 11 | BRCA22-11IR | 5'-GAT CAG TAA ATA GCA AGT CCG-3' | 21 | | 52 |
| 11 | BRCA2-11 JF | 5'-TAC TGA AAA TGA AGA TAA CAA AT-3' | 23 | 477 | 53 |
| 11 | BRCA2-11JR | 5'-ATT TTG TTC TTT CTT ATG TCA G-3' | 22 | | 54 |
| 11 | BRCA2-11KF-M13 | 5'-TGT AAA ACG ACG GCC AGT CTA CTA AAA CGG AGC AA-3' | 35 | 382 | 55 |
| 11 | BRCA2-11KR-M13 | 5'-CAG GAA ACA GCT ATG ACC GTA TGA AAA CCC AAC AG-3' | 35 | | 56 |
| 11 | BRCA2-11LF | 5'-CAC AAA ATA CTG AAA GAA AGT G-3' | 22 | 374 | 57 |
| 11 | BRCA2-11LR | 5'-GGC ACC ACA GTC TCA ATA G-3' | 19 | | 58 |
| 11 | BRCA2-11MF | 5'-GCA AAG ACC CTA AAG TAC AG-3' | 20 | 409 | 59 |
| 11 | BRCA2-11MR | 5'-CAT CAA ATA TTC CTT CTC TAA G-3' | 22 | | 60 |
| 11 | BRCA2-11NF-M13 | 5'-TGT AAA ACG ACG GCC AGT GAA AAT TCA GCC TTA GC-3' | 35 | 306 | 61 |
| 11 | BRCA2-11NR-M13 | 5'- CAG GAA ACA GCT ATG ACC ATC AGA ATG GTA GGA AT-3' | 35 | | 62 |
| 11 | BRCA2-11OF | 5'-GTA CTA TAG CTG AAA ATG ACA A-3' | 22 | 383 | 63 |
| 11 | BRCA2-11OR | 5'-ACC ACT GGC TAT CCT AAA TG-3' | 20 | | 64 |
| 11 | BRCA2-11PF | 5'-TGA AGA TAT TTG CGT TGA GG-3' | 20 | 355 | 65 |
| 11 | BRCA2-11PR | 5'-GTC AGC AAA AAC CTT ATG TG-3' | 20 | | 66 |
| 11 | BRCA2-11QF | 5'-ACG AAA ATT ATG GCA GGT TGT-3' | 21 | 337 | 67 |
| 11 | BRCA2-11QR | 5'-CTT GTC TTG CGT TTT GTA ATG-3' | 21 | | 68 |
| 11 | BRCA2-11RF | 5'-GCT TCA TAA GTC AGT CTC AT-3' | 20 | 360 | 69 |
| 11 | BRCA2-11RR | 5'-TCA AAT TCC TCT AAC ACT CC-3' | 20 | | 70 |
| 11 | BRCA2-11SF-M13 | 5'-TGT AAA ACG ACG GCC AGT TAC AGC AAG TGG AAA GC-3' | 35 | 458 | 71 |
| 11 | BRCA2-11SR-M13 | 5'-CAG GAA ACA GCT ATG ACC AAG TTT CAG TTT TAC CAAT-3' | 37 | | 72 |
| 11 | BRCA2-11TF | 5'-GTT CTT CAG AAA ATA ATC ACT C-3' | 22 | 344 | 73 |
| 11 | BRCA22-11TR | 5'-TGT AAA AAG AGA ATG TGT GGC-3' | 21 | | 74 |
| 11 | BRCA2-11UF-fVll3 | 5'-TGT AAA ACG ACG GCC AGT ACT TTT TCT GAT GTT CCT GTG-3' | 39 | 328 | 75 |
| 11 | BRCA2-11UR-M13 | 5'-CAG GAA ACA GCT ATG ACC TAA AAA TAG TGA TTG GCA ACA-3' | 39 | | 76 |
| 12 | BRCA2-12F/M13F | 5'-TGT AAA ACG ACG GCC AGT AGT GGT GTT TTA AAG TGG TCA AAA-3' | 42 | 391 | 77 |
| 12 | BRCA2-12R/M13R | 5'-CAG GAA ACA GCT ATG ACC GGA TCC ACC TGA GGT CAG AAT A-3' | 40 | | 78 |
| 13 | BRCA2/13-2F | 5'-TAA CAT TTA AGC ATC CGT TAC-3' | 21 | 310 | 79 |
| 13 | BRCA2/13-2R | 5'-AAA CGA GAC TTT TCT CAT ACT GTA TTA G-3' | 28 | | 80 |
| 14 | BRCA2-14F | 5'-ACC ATG TAG CAA ATG AGG GTC T-3' | 22 | 391 | 81 |
| 14 | BRCA2-14AR | 5'-GCT TTT GTC TGT TTT CCT CCA A-3' | 22 | | 82 |
| 15 | BRCA2-15-2F | 5'-CCA GGG GTT GTG CTT TTT AAA-3' | 21 | 284 | 83 |
| 15 | BRCA2-1 SFUTItV913-R | 5'-CAG GAA ACA GCT ATG ACC ACT CTG TCA TAA AAG CCA TC-3' | 38 | | 84 |
| 16 | BRCA2-16AF | 5'-TTT GGT TTG TTA TAA TTG TTT TTA-3' | 24 | 394 | 85 |
| 16 | BRCA2-16AR | 5'-CCA ACT TTT TAG TTC GAG AG-3' | 20 | | 86 |
| 17 | BRCA2-17F | 5'-TTC AGT ATC ATC CTA TGT G-3' | 19 | 282 | 87 |
| 17 | BRCA2-17AR | 5'-AGA AAC CTT AAC CCA TAC TG-3' | 20 | | 88 |
| 18 | BRCA2-18FUT/M13-AF | 5'-TGT AAA ACG ACG GCC AGT GAA TTC TAG AGT CAC ACT TCC-3' | 39 | 275 | 89 |
| 18 | BRCA2-18R/M13R | 5'-CAG GAA ACA GCT ATG ACC TTT AAC TGA ATC AAT GAC TG-3' | 38 | | 90 |
| 19 | BRCA2-19F/M13F | 5'-TGT AAA ACG ACG GCC AGT AAG TGA ATA TTT TTA AGG CAG TT-3' | 41 | 355 | 91 |
| 19 | BRCA2-19FUT/M13-R | 5'-CAG GAA ACA GCT ATG ACC AAG AGA CCG AAA CTC CAT CTC-3' | 39 | | 92 |
| 20 | BRCA2-20F/M13F | 5'-TGT AAA ACG ACG GCC AGT CAC TGT GCC TGG CCT GAT AC-3' | 38 | 296 | 93 |
| 20 | BRCA2-20R/M13R | 5'-CAG GAA ACA GCT ATG ACC ATG TTA AAT TCA AAG TCT CTA-3' | 39 | | 94 |
| 21 | BRCA2-21F/M13F | 5'-TGT AAA ACG ACG GCC AGT GGG TGT TTT ATG CTT GGT TCT-3' | 39 | 304 | 95 |
| 21 | BRCA2-21R/M13R | 5'-CAG GAA ACA GCT ATG ACC CAT TTC AAC ATA TTC CTT CCT G-3' | 40 | | 96 |
| 22 | BRCA2-22F-1 A | 5'-AAC CAC ACC CTT AAG ATG A-3' | 19 | 453 | 97 |
| 22 | BRCA2-22R-1A | 5'-GCA TTA GTA GTG GAT TTT GC-3' | 20 | | 98 |
| 23 | BRCA2-23FII | 5'-TCA CTT CCA TTG CAT C-3' | 16 | 290 | 99 |
| 23 | BRCA2-23RII | 5-TGC CAA CTG GTA GCT CC-3' | 17 | | 100 |
| 24 | BRCA2-24 2F | 5'-TAC AGT TAG CAG CGA CAA AA-3' | 20 | 373 | 101 |
| 24 | BRCA2-24R/M13R | 5'-CAG GAA ACA GCT ATG ACC ATT TGC CAA CTG GTA GCT CC-3' | 38 | | 102 |
| 25 | BRCA2-25F-7/23 | 5'-GCT TTC GCC AAA TTC AGC TA-3' | 20 | 427 | 103 |
| 25 | BRCA2-25R-7/23 | 5'-TAC CAA AAT GTG TGG TGA TG-3' | 20 | | 104 |
| 26 | BRCA2/26-2F | 5'-AAT CAC TGA TAC TGG TTT TG-3' | 20 | 530 | 105 |
| 26 | BRCA2/26-2R | 5'-TAT ACT TAC AGG AGC CAC AT-3' | 20 | | 106 |
| 27A | BRCA2-27AF-1A | 5'-CTG TGT GTA ATA TTT GCG-3' | 18 | 495 | 107 |
| 27A | BRCA2-27AR/M13R | 5'-CAG GAA ACA GCT ATG ACG GCA AGT TCT TCG TCA GCT ATT G-3' | 40 | | 108 |
| 27B | BRCA2-27BF/M13F | 5'-TGT AAA ACG ACG GCC AGT GAA TTC TCC TCA GAT GAC TCC A-3' | 40 | 417 | 109 |
| 27B | BRCA2-27BR/M13R | 5'-CAG GAA ACA GCT ATG ACC TCT TTG CTC ATT GTG CAA CA-3' | 38 | | 110 |

**TABLE III**

| **NORMAL PANEL TYPING** | | | | | | | | |
|---|---|---|---|---|---|---|---|---|
| **Position nt/codon** | **Nucleotide Change** | **Amino Acid Change** | **1** | **2** | **3** | **4** | **5** | **Frequency** |
| 1093/289 | AAT → CAT | Asn → His | A/A | A/C | A/A | A/A | A/C | A =.8 |
| | | | | | | | | C = .2 |
| 1342/372 | AAT → CAT | Asn → His | A/C | A/A | A/C | A/C | A/C | A = 0.6 |
| | | | | | | | | C = 0.4 |
| 1593/455 | TCA → TCG | Ser → Ser | A/A | A/A | A/A | A/A | A/G | A = 0.9 |
| | | | | | | | | G = 0.1 |
| 2457/743 | CAT → CAC | His → His | T/T | C/T | TIT | T/T | C/T | T = 0.8 |
| | | | | | | | | C = 0.2 |
| 2908/894 | GTA → ATA | Val → Ile | G/G | G/G | G/G | G/G | A/G | G = 0.9 |
| | | | | | | | | A = 0.1 |
| 3199/991 | AAC → GAC | Asn → Asp | A/A | A/G | A/A | A/A | A/G | A = 0.8 |
| | | | | | | | | G = 0.2 |
| 3624/1132 | AAA → AAG | Lys → lys | A/A | A/G | A/A | A/G | A/A | A = 0.8 |
| | | | | | | | | G = 0.2 |
| 4035/1269 | GTT → GTC | Val → Val | C/T | T/T | T/T | T/T | T/T | T = 0.9 |
| | | | | | | | | C = 0.1 |
| 7470/2414 | TCA → TCG | Ser → Ser | A/A | A/G | A/A | A/G | A/A | A = 0.8 |
| | | | | | | | | G = 0.2 |
| 9079/2951 | GCC → ACC | Ala → Thr | G/G | G/G | G/G | G/G | A/G | G = 0.9 |
| | | | | | | | | A = 0.1 |

### EXAMPLE 2

### Determination Of A Normal Individual Using BRCA2^{(OM 1-5)} and The Ten Polymorphisms For Reference

A person skilled in the art of genetic susceptibility testing will find the present invention useful for:
a) identifying individuals having a normal BRCA2 gene;
b) avoiding misinterpretation of normal polymorphisms found in the normal population.

Sequencing was carried out as in EXAMPLE 1 using a blood sample from the patient in question. However, the BRCA2 ^{(omi1-5)} sequences were used for reference and any polymorphic sites seen in the patient were compared to the nucleic acid sequences listed above for normal codons at each polymorphic site. A normal sample is one which is comparable to the BRCA2^{(omi 1-5)} sequences and contains only minor variations which occur at minor polymorphic sites. The allelic variations which occur at each of the polymorphic sites are paired here for reference.
- AAT (Asn) and CAT (His) at position 1093 (codon 289)
- CAT (His) and AAT (Asn) at position 1342 (codon 372)
- TCA (Ser) and TCG (Ser) at position 1593 (codon 455)
- CAT (His) and CAC (His) at position 2457 (codon 743)
- GTA (Val) and ATA (Ile) at position 2908 (codon 894)
- AAC (Asn) and GAC (Asp) at position 3199 (codon 991)
- AAA (Lys) and AAG (Lys) at position 3624 (codon 1132)
- GTT (Val) and GTC (Val) at position 4035 (codon 1269)
- TCA (Ser) and TCG (Ser) at position 7470 (codon 2414)
- GCC (Ala) and ACC (Thr) at position 9079 (codon 2951)

The availability of these polymorphic pairs provides added assurance that one skilled in the art can correctly interpret the polymorphic variations without mistaking a normal variation for a mutation.

All exons of the BRCA2 gene are subjected to direct dideoxy sequence analysis by asymmetric amplification using the polymerase chain reaction (PCR) to generate a single stranded product amplified from this DNA sample. Shuldiner, et al., Handbook of Techniques in Endocrine Research, p. 457-486, DePablo, F., Scanes, C., eds., Academic Press, Inc., 1993. Fluorescent dye is attached for automated sequencing using the Taq Dye Terminator Kit (Perkin-Eimer^{®} cat# 401628). DNA sequencing is performed in both forward and reverse directions on an Applied Biosystems, Inc. (ABI) automated sequencer (Model 377). The software used for analysis of the resulting data is "Sequence Navigator" purchased through ABI.

### 1. Polymerase Chain Reaction (PCR) Amplification

The PCR primers used to amplify a patient's sample BRCA2 gene are listed in TABLE II. The primers were synthesized on a DNA/RNA Synthesizer Model 394^{®}. Thirty-five cycles are of amplification are performed, each consisting of denaturing (95°C; 30 seconds), annealing (55°C; 1 minute), and extension (72°C; 90 seconds), except during the first cycle in which the denaturing time is increased to 5 minutes and during the last cycle in which the extension time is increased to 5 minutes.

PCR products are purified using Qia-quick^{®} PCR purification kits (Qiagen^{®}, cat# 28104; Chatsworth, CA). Yield and purity of the PCR product are determined spectrophotometrically at OD₂₆₀ on a Beckman DU 650 spectrophotometer.

### 2. Dideoxy Sequence Analysis

Fluorescent dye is attached to PCR products for automated sequencing using the Taq Dye Terminator Kit (Perkin-Elmer^{®}) cat# 401628). DNA sequencing is performed in both forward and reverse directions on an Applied Biosystems, Inc. (ABI) Foster City, CA., automated sequencer (Model 377). The software used for analysis of the resulting data is "Sequence Navigator^{®}" purchased through ABI. The BRCA2^{(omi 1-5)} sequences were entered sequentially into the Sequence Navigator software as the standards for comparison. The Sequence Navigator software compares the patient sample sequence to each BRCA2 ^{(omi 1-5)} standard, base by base. The Sequence Navigator highlights all differences between the standards (omi 1-5) and the patient's sample sequence.

A first technologist checks the computerized results by comparing visually the BRCA2^{(omi 1-5)} standards against the patient's sample, and again highlights any differences between the standard and the sample. The first primary technologist then interprets the sequence variations at each position along the sequence. Chromatograms from each sequence variation are generated by the Sequence Navigator and printed on a color printer. The peaks are interpreted by the first primary technologist and a second primary technologist. A secondary technologist then reviews the chromatograms. The results are finally interpreted by a geneticist. In each instance, a variation is compared to known normal polymorphism for position and base change.

### 3. Results

The patient's BRCA2 sequence was found to be heterozygous at seven nucleotide positions: 1093 (A/C), 1342 (A/C), 1593 (A/G), 2457 (C/T), 2908 (A/G), 3199 (A/G) and 9079 (A/G). In addition, this changes five amino acids in the polypeptide product: Asn to His at codon 289, Asn to His at codon 372, Val to lie at codon 894, Asn to Asp at codon 991, and Ala to Thr at codon 2951. The question arises whether any or all of these changes have significance to the patient. Comparison of the patient's results to the BRCA ^{(omi 1-5)} haplotypes demonstrates that it matches one of the BRCA2 omi standards (#5), and thus the patient sample is interpreted as carrying a normal gene sequence without causing any elevation in their risk status for breast cancer.

### EXAMPLE 3

### DETERMINING THE PRESENCE OF A MUTATION IN EXON 11 OF THE BRCA2 GENE USING BRCA2^{(omi1-5)}

A person skilled in the art of genetic susceptibility testing will find the present invention useful for determining the presence of a known or previously unknown mutation in the BRCA2 gene. A list of mutations of BRCA2 is publicly available in the Breast Cancer Information Core at http://www.nchgr.nih.gov/dir/lab_transfer/bic. This data site became publicly available on November 1, 1995. Friend, S. *et al. Nature Genetic* 11:238, (1995).

In this example, a mutation in exon 11 is characterized by amplifying the region of the mutation with a primer set which amplifies the region of the mutation. Sequencing was carried out as in Example 1 using a blood sample from the patient in question. Specifically, exon 11 of the BRCA2 gene is subjected to direct dideoxy sequence analysis by asymmetric amplification using the polymerase chain reaction (PCR) to generate a single stranded product amplified from this DNA sample. Shuldiner, et al., Handbook of Techniques in Endocrine Research, p. 457-486, DePablo, F., Scanes, C., eds., Academic Press, Inc., 1993. Fluorescent dye is attached for automated sequencing using the Taq Dye Terminator Kit (Perkin-Elmer^{®} cat# 401628). DNA sequencing is performed in both forward and reverse directions on an Applied Biosystems, Inc. (ABI) automated sequencer (Model 377). The software used for analysis of the resulting data is "Sequence Navigator" purchased through ABI.

### 1. Polymerase Chain Reaction (PCR) Amplification

Genomic DNA (100 nanograms) extracted from white blood cells of the subject is amplified in a final volume of 25 microliters containing 1 microliter (100 nanograms) genomic DNA, 2.5 microliters 10X PCR buffer (100 mM Tris, pH 8.3, 500 mM KCl, 1.2 mM MgCl₂), 2.5 microliters 10X dNTP mix (2 mM each nucleotide), 2.5 microliters forward primer (BRCA2-11Q-F, 10 micromolar solution), 2.5 microliters reverse primer (BRCA2-11Q-R, 10 micromolar solution), and 1 microliter Taq polymerase (5 units), and 13 microliters of water.

The PCR primers used to amplify segment Q of exon 11 (where the mutation 6174delT is found) are as follows:
BRCA2-11Q-F: 5'- ACG' AAA' ATT' ATG' GCA' GGT' TGT-3'
BRCA2-11Q-R: 5'- CTT GTC' TTG' CGT' TTT' GTA' ATG-3'

The primers are synthesized on an DNA/RNA Synthesizer Model 394^{®}. Thirty-five cycles are performed, each consisting of denaturing (95°C; 30 seconds), annealing (55°C; 1 minute), and extension (72°C; 90 seconds), except during the first cycle in which the denaturing time is increased to 5 minutes, and during the last cycle in which the extension time is increased to 5 minutes.

PCR products are purified using Qia-quick^{®} PCR purification kits (Qiagen^{®}, cat# 28104; Chatsworth, CA). Yield and purity of the PCR product are determined spectrophotometrically at OD₂₆₀ on a Beckman DU 650 spectrophotometer.

### 2. Dideoxy Sequence Analysis

Fluorescent dye is attached to PCR products for automated sequencing using the Taq Dye Terminator Kit (Perkin-Elmer^{®} cat# 401628). DNA sequencing is performed in both forward and reverse directions on an Applied Biosystems, Inc. (ABI) Foster City, CA., automated sequencer (Model 377). The software used for analysis of the resulting data is "Sequence Navigator^{®}" purchased through ABI. The BRCA2^{(omi 1-5)} sequence is entered into the Sequence Navigator software as the Standard for comparison. The Sequence Navigator software compares the sample sequence to the BRCA2^{(omi)} standard, base by base. The Sequence Navigator highlights all differences between the BRCA2^{(omi)} normal DNA sequence and the patient's sample sequence.

A first technologist checks the computerized results by comparing visually the BRCA2^{(omi 1-5)} standard against the patient's sample, and again highlights any differences between the standard and the sample. The first primary technologist then interprets the sequence variations at each position along the sequence. Chromatograms from each sequence variation are generated by the Sequence Navigator and printed on a color printer. The peaks are interpreted by the first primary technologist and a second primary technologist. A secondary technologist then reviews the chromatograms. The results are finally interpreted by a geneticist. In each instance, a sequence variation is compared to known normal polymorphisms for position and base change. The ten frequent polymorphisms which occur in BRCA2 are:
- AAT (Asn) and CAT (His) at position 1093 (codon 289)
- CAT (His) and AAT (Asn) at position 1342 (codon 372)
- TCA (Ser) and TCG (Ser) at position 1593 (codon 455)
- CAT (His) and CAC (His) at position 2457 (codon 743)
- GTA (Val) and ATA (Ile) at position 2908 (codon 894)
- AAC (Asn) and GAC (Asp) at position 3199 (codon 991)
- AAA (Lys) and AAG (Lys) at position 3624 (codon 1132)
- GTT (Val) and GTC (Val) at position 4035 (codon 1269)
- TCA (Ser) and TCG (Ser) at position 7470 (codon 2414)
- GCC (Ala) and ACC (Thr) at position 9079 (codon 2951)

### 3. Results

Using the above PCR amplification and standard fluorescent sequencing technology, the 6174delT mutation may be found. Mutations are noted by the length of non-matching sequence variation. Such a lengthy mismatch pattern occurs with deletions and insertions. This mutation is named in accordance with the suggested nomenclature for naming mutations, Beaudet, A et al., Human Mutation 2:245-248, (1993). The 6174delT mutation at codon 1982 of the BRCA2 gene lies in segment "Q" of exon 11. The DNA sequence results demonstrate the presence of a one base pair deletion of a T at nucleotide 6174 of the BRCA2^{(omi 1-5)} sequences. This mutation interrupts the normal reading frame of the BRCA2 transcript, resulting in the appearance of an in-frame terminator (TAG) at codon position 2003. This mutation is, therefore, predicted to result in a truncated, and most likely, non-functional protein.

### EXAMPLE 4

### GENERATION OF MONOCLONAL AND POLYCLONAL ANTIBODIES USING GST-BRCA2 FUSION PROTEIN AS AN IMMUNOGEN

DNA primers are used to amplify a fragment of BRCA2 using PCR technology. The product is then digested with suitable restriction enzymes and fused in frame with the gene encoding glutathione S-transferase (GST) in *Escherichia coli* using GST expression vector pGEX (Pharmacia Biotech Inc.) The expression of the fusion protein is induced by the addition of isopropyl-β-thiogalactopyranoside. The bacteria are then lysed and the overexpressed fusion protein is purified with glutathione-sepharose beads. The fusion protein is then verified by SDS/PAGE gel and N-terminus protein sequencing. The purified protein is used to immunize rabbits according to standard procedures described in Harlow & Lane (1988). Polycolonal antibody is collected from the serum several weeks after and purified using known methods in the art. Monoclonal antibodies against all or fragments of BRCA2 protein, polypeptides, or functional equivalents are obtained using hybridoma technology, see also Harlow & Lane (1988). The BRCA2 protein or polypeptide is coupled to the carrier keyhole limpet hemocyanin in the presence of glutaraldehyde. The conjugated immunogen is mixed with an adjuvant and injected into rabbits. Spleens from antibody-containing rabbits are removed. The B-cells isolated from spleen are fused to myeloma cells using polyethylene glycol (PEG) to promote fusion. The hybrids between the myeloma and B-cells are selected and screened for the production of antibodies to immunogen BRCA2 protein or polypeptide. Positive cells are recloned to generate monoclonal antibodies.

### EXAMPLE 5

### DETECTION OF BRCA2 EXPRESSION IN HUMAN TISSUES AND CELL LINES

The expression of BRCA2 in human tissues is determined using Northern blot analysis. Human tissues include those from pancreas, testis, prostate, ovary, breast, small intestine, and colon are obtained from Clontech Laboratories, Inc., Palo Alto, CA. The poly(A)+ mRNA Northern blots from different human tissues is hybridized to BRCA2 cDNA probes according to manufacture protocol. The expression level is further conformed by RT-PCR using oligo-d(T) as a primer and other suitable primers.

For Northern Blot analysis of cancer cell lines, the human ovarian cancer cell line SKOV-3 and the human breast cancer cell line MCF-7 are obtained from the American Type Culture Collection. Total RNA is prepared by lysing cell in the presence of guanidinium isocyanate. Poly(A)⁺ mRNA is isolated using the PolyATract mRNA isolation system from Promega, Madison, WI. The isolated RNA is then electrophoresed under denaturing conditions and transferred to Nylon membrane. The probe used for Northern blot is a fragment of BRCA2 sequence obtained by PCR amplification. The probes are labeled with [α-³²P] dCTP using a random-primed labeling kit (Amersham Life Science, Arlington Heights, IL).

### EXAMPLE 6

### EXPRESSION OF THE BRCA2 PROTEIN

The whole-cell extracts of BRCA2 transfected cells are subjected to immunoprecipitation and immunoblotting to determine the BRCA2 protein level. The BRCA2 protein or polypeptide is immunoprecipitated using anti-BRCA2 antibodies prepared according to Example 4. Samples are then fractionated using SDS/PAGE gel and transferred to nitrocellulose. Western blot of the BRCA2 protein or polypeptide is performed with the indicated antibodies. Antibody reaction is revealed using enhanced chemiluminescence reagents (Dupont New England Nuclear, Boston, MA).

### EXAMPLE 7

### USE OF THE BRCA2^{(omi1-5)} GENE THERAPY

The growth of ovarian or breast cancer may be arrested by increasing the Expression of the BRCA2 gene where inadequate expression of that gene is responsible for hereditary ovarian or breast cancer. Gene therapy may be performed on a patient to reduce the size of a tumor. The LXSN vector may be transformed with a BRCA2(^{omi1-5}) coding sequence as presented SEQ ID NO:4, 6, 8, 10, or 12 or a fragment thereof.

### Vector

The LXSN vector is transformed with a fragment of the wildtype BRCA2(^{omi1-5)} coding sequence as set forth in SEQ ID NO:4, 6, 8, 10, or 12. The LXSN-BRCA2^{(omi1-5)} retroviral expression vector is constructed by cloning a *Sal* I linkered BRCA2^{(omi1-5)} cDNA or fragments thereof into the *Xho* I site of the vector LXSN. Constructs are confirmed by DNA sequencing. See Holt et al., *Mature Genetics* 12- 298-302 (1996). Retroviral vectors are manufactured from viral producer cells using serum free and phenol-red free conditions and tested for sterility, absence of specific pathogens, and absence of replication-competent retrovirus by standard assays. Retrovirus is stored frozen in aliquots which have been tested.

Patients receive a complete physical exam, blood, and urine tests to determine overall health. They may also have a chest X-ray, electrocardiogram, and appropriate radiologic procedures to assess tumor stage.

Patients with metastatic ovarian cancer are treated with retroviral gene therapy by infusion of recombinant LXSN-BRCA2^{(omi1-5)} retroviral vectors into peritoneal sites containing tumor, between 10⁹ and 10¹⁰ viral particles per dose. Blood samples are drawn each day and tested for the presence of retroviral vector by sensitive polymerase chain reaction (PCR)-based assays. The fluid which is removed is analyzed to determine:
1. The percentage of cancer cells which are taking up the recombinant LXSN-BRCA2^{(omi1-5)} retroviral vector combination. Successful transfer of BRCA1 gene into cancer cells has been shown by both RT-PCR analysis and *in situ* hybridization. RT-PCR is performed with by the method of Thompson et al., Nature Genetics 9: 444-450 (1995), using primers derived from a BRCA2^{(omi1-5)} coding sequence as in SEQ ID NO:4, 6, 8, 10; or 12 or fragments thereof. Cell lysates are prepared and immunoblotting is performed by the method of Jensen et al., Nature Genetics 12: 303-308 (1996) and Jensen et al., Biochemistry 31: 10887-10892 (1992).
2. Presence of programmed cell death using APOTAG^{®} *in situ* apoptosis detection kit (ONCOR, INC., Gaithersburg, Maryland) and DNA analysis.
3. Measurement of BRCA2 gene expression by slide immunofluorescence or Western blot.

Patients with measurable disease are also evaluated for a clinical response to LXSN-BRCA2^{(omi1-5)} especially those that do not undergo a palliative intervention immediately after retroviral vector therapy. Fluid cytology, abdominal girth, CT scans of the abdomen, and local symptoms are followed.

For other sites of disease, conventional response criteria are used as follows:
1. Complete Response (CR), complete disappearance of all measurable lesions and of all signs and symptoms of disease for at least 4 weeks.
2. Partial Response (PR), decrease of at least 50% of the sum of the products of the 2 largest perpendicular diameters of all measurable lesions as determined by 2 observations not less than 4 weeks apart. To be considered a PR, no new lesions should have appeared during this period and none should have increased in size.
3. Stable Disease, less than 25% change in tumor volume from previous evaluations.
4. Progressive Disease, greater than 25% increase in tumor measurements from prior evaluations. The number of doses depends upon the response to treatment.

### EXAMPLE 8

### PROTEIN REPLACEMENT THERAPY

Therapeutically elevated level of functional BRCA2 protein may alleviate the absence or reduced endogenous BRCA2 tumor suppressing activity. Breast or ovarian cancer is treated by the administration of a therapeutically effective amount of the BRCA2 protein, a polypeptide, or its functional equivalent in a pharmaceutically acceptable carrier. Clinically effective delivery method is applied either locally at the site of the tumor or systemically to reach other metastasized locations with known protocols in the art. These protocols may employ the methods of direct injection into a tumor or diffusion using time release capsule. A therapeutically effective dosage is determined by one of skill in the art.

Breast or ovarian cancer may be prevented by the administration of a prophylactically effective amount of the BRCA2 protein, polypeptide, or its functional equivalent in a pharmaceutically acceptable carrier. Individuals with known risk for breast or ovarian cancer are subjected to protein replacement therapy to prevent tumorigenesis or to decrease the risk of cancer. Elevated risk for breast and ovarian cancer includes factors such as carriers of one or more known BRCA1 and BRCA2 mutations, late child bearing, early onset of menstrual period, late occurrence of menopause, and certain high risk dietary habits. Clinically effective delivery method is used with known protocols in the art, such as administration into peritoneal cavity, or using an implantable time release capsule. A prophylactically effective dosage is determined by one of skill in the art.

### TABLE OF REFERENCES

1. Sanger, F., et al., J. Mol. Biol. 42:1617, (1980).
2. Beaucage, et al., Tetrahedron Letters 22:1859-1862, (1981).
3. Maniatis, et al. in Molecular Cloning: A Laboratory Manual, Cold Spring Harbor, NY, p 280-281, (1982).
4. Conner, et al., Proc. Natl. Acad. Sci. U.S.A. 80:278, (1983)
5. Saiki, et.al., Bio/Technology 3:1008-1012, (1985)
6. Landgren, et al., Science 241:1007,(1988)
7. Landgren, et al., Science 242 :229-237, (1988).
8. PCR. A Practical Approach, ILR Press, Eds. M. J. McPherson, P. Quirke, and G. R. Taylor, (1992).
9. Easton et al., American Journal of Human Genetics 52:678-701, (1993).
10. Patent No. 4,458,066.
11. Rowell, S., et al., American Journal of Human Genetics 55:861-865, (1994)
12. Miki, Y. et a/., Science 266:66-71, (1994).
13. Wooster, R. et al., Science 265:2088-2090, (1994).
14. Wooster, R. et al., Nature 378:789-792, (1995),
15. Beaudet, A et al., Human Mutation 2:245-248, (1993).
16.Friend, S. et al. Nature Genetics 11:238, (1995).
17.Teng et al, Nature Genetics 13: 241-244 (1996).
18.Couch et al, Nature Genetics 13: 123-125 (1996).
19.Tartigan et al, Nature Genetics 12: 333-337 (1996).
20.Phelan et al, Nature Genetics 13: 120-122 (1996).
21. Schubert et al, American Journal of Human Genetics 60: 1031-1040 (1996).
22.Sambrook, et al., Molecular Cloning: A Laboratory Manual, Second Edition, Cold Spring Harbor Laboratory, Cold Spring Harbor, N.Y. (1989).
23. Bertwistle and Ashworth, Curr. Opin. Genet. Dev. 8(1): 14-20 (1998).
24.Zhang et al., Cell 92:433-436 (1998).
25. Sharan et al., Nature 386:804-810 (1991).
26. Katagiri et al., Genes, Chromosomes & Cancer 21:217-222 (1988).
27.Crooke, Annu. Rev. Pharmacol. Toxicol. 32:329-376 (1992)
28. Robinson-Benion and Holt, Methods Enzymol. 254:363-375 (1995).
29. Harlow & Lane, Antibodies: A Laboratory Manual, Cold Spring Harbor Laboratory, Cold Spring Harbor, NY, 1988.
30.Shuldiner, et al., Handbook of Techniques in Endocrine Research, p. 457-486, DePablo, F., Scanes, C., eds., Academic Press, Inc., 1993.
31. Holt et al., Nature Genetics 12: 298-302 (1996).
32.Thompson et al., Nature Genetics 9:444-450 (1995).
33. Jensen et al., Nature Genetics 12: 303-308 (1996)
34. Jensen et al., Biochemistry 31: 10887-10892 (1992).

Although the invention has been described with reference to the presently preferred embodiments, it should be understood that various modifications can be made without departing from the spirit of the invention. Accordingly, the invention is limited only by the following claims.

## Claims

1. A nucleic acid comprising at least 18 consecutive nucleotides of exon 15 of the BRCA2 gene as shown in Figure 2, wherein said nucleic acid comprises the 3' exon/intron sequence of exon 15 of the BRCA2 gene as shown in Figure 2 and wherein the 3'-end of the exon portion of said nucleic acid has the nucleotide sequence 5'-GTGTTCTCATAAACAG-3' or 5'-CTGCGTGTTCTCATAAACAG-3'.

2. A nucleic acid comprising at least 18 consecutive nucleotides of exon 16 of the BRCA2 gene as shown in Figure 2, wherein said isolated nucleic acid comprises the 5' intron/exon sequence of exon 16 of the BRCA2 gene as shown in Figure 2 and wherein the 5'-end of the exon portion of said nucleic acid has the nucleotide sequence 5'-CTGTATACGTATGGCGTTTC-3'.

3. A nucleic acid comprising the following nucleotide sequence:

4. A nucleic acid comprising the following nucleotide sequence:

5. An in vitro method of analyzing exon 15 of a *BRCA2* gene in a human patient comprising:
(a) sequencing a nucleic acid comprising exon 15 of a *BRCA2* gene from a sample from said patient; and
(b) comparing the nucleotide sequence determined in (a) to a nucleotide sequence comprising the following nucleotide sequence, including the exon/intron boundaries as shown:

6. The method of claim 5 comprising synthesizing a nucleic acid of claim 1 or 3.

7. An in vitro method of analyzing exon 16 of a *BRCA2* gene in a human patient comprising:
(a) sequencing a nucleic acid comprising exon 16 of a *BRCA2* gene from a sample from said patient; and
(b) comparing the nucleotide sequence determined in (a) to a nucleotide sequence comprising the following nucleotide sequence, including the exon/intron boundaries as shown:

8. The method of claim 7 comprising synthesizing a nucleic acid of claim 2 or 4.

## Patentansprüche

1. Nucleinsäure, die mindestens 18 aufeinanderfolgende Nucleotide von Exon 15 des BRCA2-Gens wie in Figur 2 gezeigt umfasst, wobei die Nucleinsäure die 3'-Exon/Intron-Sequenz von Exon 15 des BRCA2-Gens wie in Figur 2 gezeigt umfasst, und wobei das 3'-Ende des Exon-Teils der Nucleinsäure die Nucleotidsequenz 5'-GTGTTCTCATAAACAG-3' oder 5'-CTGCGTGTTCTCATAAACAG-3' aufweist.

2. Nucleinsäure, die mindestens 18 aufeinanderfolgende Nucleotide von Exon 16 des BRCA2-Gens wie in Figur 2 gezeigt umfasst, wobei die isolierte Nucleinsäure die 5'-Exon/Intron-Sequenz von Exon 16 des BRCA2-Gens wie in Figur 2 gezeigt umfasst, und wobei das 5'-Ende des Exon-Teils der Nucleinsäure die Nucleotidsequenz 5'-CTGTATACGTATGGCGTTTC-3' aufweist.

3. Nucleinsäure, die die folgende Nucleotidsequenz umfasst:

4. Nucleinsäure, die die folgende Nucleotidsequenz umfasst:

5. *In* vitro-Verfahren zur Analyse von Exon 15 eines BRCA2-Gens bei einem menschlichen Individuum, umfassend:
(a) das Sequenzieren einer Nucleinsäure, die Exon 15 eines *BRCA2-*Gens von einer Probe des Individuums umfasst; und
(b) das Vergleichen der in (a) bestimmten Nucleotidsequenz mit einer Nucleotidsequenz, die die folgende Nucleotidsequenz einschließlich der Exon/Intron-Grenzen wie gezeigt umfasst:

6. Verfahren nach Anspruch 5, umfassend das Synthetisieren einer Nucleinsäure nach Anspruch 1 oder 3.

7. *In vitro*-Verfahren zur Analyse von Exon 16 eines *BRCA2*-Gens bei einem menschlichen Individuum, umfassend:
(a) das Sequenzieren einer Nucleinsäure, die Exon 16 eines *BRCA2-*Gens von einer Probe des Individuums umfasst; und
(b) das Vergleichen der in (a) bestimmten Nucleotidsequenz mit einer Nucleotidsequenz, die die folgende Nucleotidsequenz einschließlich der Exon/Intron-Grenzen wie gezeigt umfasst:

8. Verfahren nach Anspruch 7, umfassend das Synthetisieren einer Nucleinsäure nach Anspruch 2 oder 4.

## Revendications

1. Acide nucléique comprenant au moins 18 nucléotides consécutifs de l'exon 15 du gène BRCA2 comme illustré sur la Figure 2, dans lequel ledit acide nucléique comprend la séquence 3' exon/intron de l'exon 15 du gène BRCA2 comme illustré sur la Figure 2 et dans lequel l'extrémité 3' de la partie exon dudit acide nucléique a la séquence de nucléotides 5'-GTGTTCTCATAAACAG-3' ou 5'-CTGCGTGTTCTCATAAACAG-3'.

2. Acide nucléique comprenant au moins 18 nucléotides consécutifs de l'exon 16 du gène BRCA2 comme illustré sur la Figure 2, dans lequel ledit acide nucléique isolé comprend la séquence 5' intron/exon de l'exon 16 du gène BRCA2 comme illustré sur la Figure 2 et dans lequel l'extrémité 5' de la partie exon dudit acide nucléique a la séquence de nucléotides 5'-CTGTATACGTATGGCGTTTC-3'.

3. Acide nucléique comprenant la séquence de nucléotides suivante :

4. Acide nucléique comprenant la séquence de nucléotides suivante :

5. Méthode in vitro d'analyse de l'exon 15 d'un gène *BRCA2* chez un patient humain comprenant :
(a) le séquençage d'un acide nucléique comprenant l'exon 15 d'un gène *BRCA2* issu d'un échantillon provenant dudit patient ; et
(b) la comparaison de la séquence de nucléotides déterminée en (a) à une séquence de nucléotides comprenant la séquence de nucléotides suivante, incluant les limites exon/intron comme illustré :

6. Méthode selon la revendication 5 comprenant la synthèse d'un acide nucléique selon la revendication 1 ou 3.

7. Méthode in vitro d'analyse de l'exon 16 d'un gène *BRCA2* chez un patient humain comprenant :
(a) le séquençage d'un acide nucléique comprenant l'exon 16 d'un gène *BRCA2* issu d'un échantillon provenant dudit patient ; et
(b) la comparaison de la séquence de nucléotides déterminée en (a) à une séquence de nucléotides comprenant la séquence de nucléotides suivante, incluant les limites exon/intron comme illustré :

8. Méthode selon la revendication 7 comprenant la synthèse d'un acide nucléique selon la revendication 2 ou 4.
